(19) Europäisches Patentamt
European Patent Office
Office européen des brevets

(11) EP 2 216 429 A1

(12) **EUROPEAN PATENT APPLICATION**
published in accordance with Art. 153(4) EPC

(43) Date of publication:
**11.08.2010 Bulletin 2010/32**

(21) Application number: **08850939.3**

(22) Date of filing: **12.11.2008**

(51) Int Cl.:
***C40B 30/02*** *(2006.01)* ***G06F 19/00*** *(2006.01)*

(86) International application number:
**PCT/JP2008/070973**

(87) International publication number:
**WO 2009/064015 (22.05.2009 Gazette 2009/21)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **12.11.2007 JP 2007293751**

(71) Applicant: **In-Silico Sciences, Inc.**
**Ota-ku**
**Tokyo 145-0065 (JP)**

(72) Inventors:
- **UMEYAMA, Hideaki**
**Urayasu-shi**
**Chiba 279-0011 (JP)**
- **TAKAYA, Daisuke**
**Urayasu-shi**
**Chiba 279-0012 (JP)**
- **SHITAKA, Mayuko**
**Higashimurayama-shi**
**Tokyo 189-0001 (JP)**
- **KANOU, Kazuhiko**
**Tokyo 120-0003 (JP)**
- **TERASHI, Genki**
**Tokyo 152-0034 (JP)**
- **KOMATSU, Katsuichiro**
**Tokyo 145-0065 (JP)**

(74) Representative: **HOFFMANN EITLE**
**Patent- und Rechtsanwälte**
**Arabellastrasse 4**
**81925 München (DE)**

# (54) IN SILICO SCREENING SYSTEM AND IN SILICO SCREENING METHOD

(57) The present invention provides an apparatus including a compound database which has been produced by extracting fingerprints of compound related to plural atoms in a compound for each candidate compound, **characterized by** extracting the fingerprints of compound of binding compounds that are already known to bind to family proteins having 3-dimensional structure that is identical or similar to that of target protein, together with the 3-dimensional coordinates that have been converted into the coordinate system of the target protein, to produce a fingerprint set of binding compound, and for a candidate compound stored in the compound database, computing the 3-dimensional structure of the candidate compound with respect to the target protein, so that the interaction score based on the root-mean-square deviation of a unit of fingerprint of compound calculated on the basis of the 3-dimensional coordinates of the fingerprint set of binding compound is optimized.

FIG.4

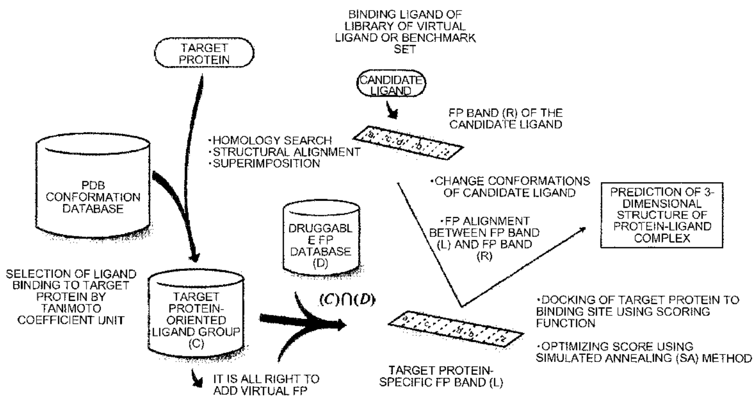


EP 2 216 429 A1

**Description**

TECHNICAL FIELD

The present invention relates to an apparatus for in silico screening, and a method of in silico screening.

BACKGROUND ART

There have been hitherto compounds commercially available from reagent supplier companies or the like, such as the compounds falling under the category of pharmaceutical products, or reagent compounds. Furthermore, like those macromolecules confirmed by various tests based mainly on mass spectrometry as a macromolecule that interacts with compounds, or those macromolecules publicly recognized through the documents included in the journals represented by, for example, Nature or Science, there are target macromolecules which interact with compounds such as invented pharmaceutical products, and thereby bring about healing of the state of illness or the state of disease of animals and plants, alleviation of symptoms, maintenance of the status quo and the like, such as drug target proteins, drug target nucleic acids, drug target saccharides, or drug target lipids.

In the occasion of performing low molecular compound docking to target macromolecules and in silico screening, the conventional procedure has been such that each compound in the compound database in which an enormous number of compounds such as the pharmaceutical candidate compounds as described above are stored, are subjected to docking interaction with, for example, a target macromolecular protein which is mainly composed of a protein, to thereby determine the conformations by which compounds amounting to several hundred thousand individuals that exist in reality, directly interact with target proteins, and thus the interaction energy or score values corresponding thereto are obtained. The procedure has also been such that the relevant score values are lined up using stability as an index, in order of high to low stability, and the order of the interaction between a compound and a target protein to drug is determined.

In conventional methods such as Dock by Kuntz, et al. [reference literature (Ewing, et al. J. Comput. Aided Mol. Des. 2001; 15(5): 411-428)], AutoDock by Goodsell et al. [reference literature (Goodsell, et al. J. Mol. Recognit. 1996; 9: 1-5)], GOLD by Gareth, et al. [reference literature (Jones et al. J. Mol. Biol. 1997; 267: 724-748)], FlexX by Rarey, et al., Fragment Potential by Nicolas, et al., calculation of the score value has been carried out by the respective methods, using the grid information of the environment for ligand binding of a target protein as the target macromolecule, or the multipoint information of compounds, which emphasizes the vector between a compound and a target macromolecule.

That is, even though there is some sort of ingenuity such as the biological environment of the target protein, the processes has been carried out such that the interaction energy and the like are calculated from the formulas for classical physical interatomic potential between the atoms of a compound and the atoms constituting a target macromolecular protein, based on the information such as the grid information or the multipoint information, and the order related to the conformation of the compound or the intensity of binding in the interaction of the compound is determined as score values. Furthermore, to determine the order of interaction, devises for determining the order regarding the conformations of various compounds that involved interaction, using techniques such as be clustering, have been implemented.

However, since the conventional methods of in silico screening focus on predicting protein-ligand complexes with high accuracy, there has been a problem that such prediction is not consistent with the results of directly selecting a large number of compounds that get a hit.

Furthermore, since the conventional methods of in silico screening make nonempirical predictions using the potential functions of classical physics, there has been a problem that screening cannot be carried out with high prediction efficiency while taking the information of biochemical experiments or the like into consideration.

The present invention has been made under such circumstances, and it is an object of the invention to provide an apparatus for in silico screening and a method of in silico screening, which can predict the binding between a protein and a compound with high accuracy while being able to select many compounds that gets a hit, and can increase the prediction efficiency.

DISCLOSURE OF INVENTION

In view of the fact that an enormous amount of information on 3-dimensional coordinates representing the interactions between a compound and a target macromolecule, which are obtained by experiments such as X-ray analysis, NMR, electron beam analysis and high-resolution electron microscope photographs, are registered on a publicly accessible database, or of the recent enhancement of the performance of computers and the progress in bioinformatics, the inventors of the present invention conceived an idea that instead of performing the general methods of in silico screening of classical physics in a traditional manner, it is possible to perform semiempirical in silico screening of compounds based on human intelligence, using the information of bioinformatics such as the collectively overlapping state of various compounds that are bound to target macromolecular proteins.

The present invention has been completed as a result of the present invention devotedly investigating by the inventors based on the idea described above. An apparatus for in silico screening for performing screening of candidate compounds that bind to a target protein includes a storage unit, and a control unit, wherein the storage unit includes a compound database produced by extracting a chemical descriptor that includes the atom type and the interatomic bonding rules as the fingerprint of a compound related to a plurality of atoms in the compound, for each of the candidate compounds, and the control unit includes a fingerprint of compound producing unit that extracts the fingerprint of compound from a binding compound known to bind to a family protein, which has a 3-dimensional structure that is identical or similar to that of the target protein, along with 3-dimensional coordinates that have been converted into the coordinate system of the target protein, to thereby produce a fingerprint set of binding compound, and an optimizing unit that computes, for the candidate compounds stored in the compound database, the 3-dimensional structures of the candidate compounds with respect to the target protein, so that the interaction scores that are based on the root-mean-square deviations of each unit of fingerprint of compound that have been calculated using the 3-dimensional coordinates of the fingerprint set of binding compound as a basic, are optimized.

That is, according to the present invention, the binding between a protein and a compound can be predicted with high accuracy, while a large number of compounds that get a hit can be selected. Also, semiempirical screening can be performed while taking the information of biochemical experiments and the like into consideration, and the prediction efficiency can be increased.

As discussed above, the present invention is different from conventional techniques, from the viewpoint of making the bioinformatics technology which uses a fingerprint set of 3-dimensional compound, to exhibit a performance equivalent to the docking of a low molecular compound and a macromolecular protein, which uses the techniques of classical physical energy. Particularly, when it is considered that technologies such as X-ray analysis, NMR, electron beam analysis and high-resolution electron microscopic analysis are making remarkable progress, it is predicted that the number of molecules of the compounds binding to target macromolecular proteins would enormously increase, and therefore, the present invention exhibits high effects.

The apparatus for in silico screening according to one aspect of the present invention, is connected to a protein database apparatus that stores the respective 3-dimensional structure and amino acid sequence of the proteins bound to a compound, wherein the control unit further includes a homology searching unit that searches for the family protein and the binding compound from the protein database system, based on the homology of the target protein with the amino acid sequence, and the fingerprint of compound producing unit extracts the fingerprint of compound from the binding compound to bind to the family protein searched by the homology searching unit, along with 3-dimensional coordinates that have been converted into the coordinate system of the target protein to thereby produce the fingerprint set of binding compound.

Here, as a specific exemplary embodiment of the present invention, when a family macromolecule set is extracted under the conditions used for extracting a collective conformation by which various low molecular compounds bind to a family macromolecule set that is similar in the 3-dimensional structure to a target protein, among target macromolecules, the detection is performed through homology search based on PSI-Blast or the like, for the sequence of the target protein as the query sequence. According to the present invention, when a protein-ligand complex that has been searched as coming under the detected proteins, contains a low molecular ligand, the protein-ligand complex is superimposed to the target protein using CE (an operation of superimposing the conformations of proteins not in awareness of the kind of atoms) or the like. According to the present invention, when the Z-score which represents similarity in the structure has a predetermined value (for example, equal to or more than 3.7), the ligand bound to the searched homologous protein is converted from the coordinate system of the homologous protein into the coordinate system of the target protein, along with the ligand coordinates, and only the ligand can be extracted.

Here, the CE performs the operation of superimposing the conformations of proteins not in awareness of the kind of atoms, but a program having the same functions can also be used in substitution. According to the present invention, when only those sequences having high homology are obtained by the homology search based on the PSI-Blast or the like, for the sequence of the target protein as the query sequence, a program that performs an operation of superimposing the conformations of proteins in awareness of the kind of atoms, may also be used. According to the present invention, the homology search is not limited to the PSI-Blast, but any homology search program may be applied as long as it is a software program capable of performing homology search using a sequence as a query, and performing an evaluation of the similarity of sequence in a quantitative manner.

The present invention is the apparatus for in silico screening according to another aspect of the present invention, wherein the fingerprint of compound producing unit converts the 3-dimensional coordinates of the binding compound that binds to the family protein, into the coordinate system of the target protein, through the superimposition of conformations of the family protein and the target protein, and extracts the fingerprints of compound along with the converted 3-dimensional coordinates of compound, to thereby produce the fingerprint set of binding compound.

The present invention is the apparatus for in silico screening according to still another aspect of the present invention, wherein the fingerprint of compound producing unit further includes a fingerprint of new compound adding unit that performs superimposition of conformations by referring to another compound that is different from the binding compound, extracts a fingerprint of compound that straddles between the atoms of the binding compound and the atoms of the other compound, and adds the fingerprint of compound to the fingerprint set of binding compound.

In a specific example according to the invention, a specific example of the fingerprint set of binding compound may be constituted of "CElib" (FP (fingerprint) set extracted from collected ligands in the binding site), which is a database of various low molecular compounds bound to a family macromolecular protein set that is similar in the 3-dimensional structure to a target protein among target macromolecules. This CElib includes the coordinates and Sybyl atom-type in the coordinate system of target proteins, and the information on bonding rules such as in a single bond, a double bond, or bonding in aromatic rings. Here, according to the present invention, an arbitrary FP (fingerprint: refers to the "fingerprint of compound"; hereinafter, the same applies) may be added to the CElib according to the necessity of the purpose of searching for low molecular compounds in regard to target proteins.

That is, according to the present invention, instead of extracting the FP from the various low molecular compounds collectively bound to a family macromolecule set that is similar in 3-dimensional structure to an existing target protein, the kind of atom is interchanged within various low molecular compounds, while maintaining the similarity between the FP and generally existing ordinary compound molecules. According to the present invention, the interaction energy with a target protein is calculated using a program that can evaluate the stability, such as "Circle, " and thereby a "modified FP," which is slightly different in the structure and performs the interaction more stably, is obtained. According to the present invention, the modified FP which is stable against a target protein in terms of local energy is used, and is adopted as a counterpart FP in the superimposition of FP, such as in the instance of the FPs obtained from the collectively bound various low molecular compounds that are obtained as a result of the operation of superimposition of conformations between proteins, which have been considered and used as new FPs as described for the previously discussed inventions.

According to the present invention, in regard to the docking between a protein and a ligand, a ligand conformation which uses bioinformatics called a fingerprint set of compound including 3-dimensional coordinates, is obtained instead of the conventionally used physicochemical interaction functions. According to the present invention, instead of extracting the FP from the various low molecular compounds collectively bound to a family macromolecular protein set that is similar in 3-dimensional structure to an existing target protein, a fingerprint set of plural compound-bound 3-dimensional compound resembling the FP of generally existing conventional molecules is created by referring to compounds having different molecular structures, among various low molecular compounds. According to the present invention, the created fingerprint set of compound adopts the counterpart FP for the superimposition of FP, such as in the instance of the FP obtained from the collectively bound various low molecular compounds that are obtained as a result of the operation of superimposition of conformations between proteins, which have been considered and used as new FPs as described for the previously discussed inventions.

That is, according to the present invention, the various low molecular compounds collectively bound to a family macromolecule set are completely separated, so that the FPs of the various low molecular compounds that have been parted discretely are used as the basic of docking, instead of the calculation of docking by physical formulas in conventional cases. The present invention has been created as a result of careful consideration of the fact that the entity of the conformations of the various low molecular compounds collectively bound to a family macromolecular protein set that is similar in 3-dimensional structure to an existing protein, is close to the most stable conformation that has interacted with the family protein of the target protein, and thus has superior effects unlike the conventional techniques. Thus, the present invention is useful.

The present invention is the apparatus for in silico screening according to still another aspect of the present invention, wherein the fingerprint of compound producing unit further includes a fingerprint of new compound adding unit that, in regard to the compound that is similar to the binding compound on the basis of the Tanimoto coefficient, interchanges the kind of atom between the atoms of the binding compound and them of the compound, calculates the interaction energy with respect to the target protein to thereby produce a fingerprint of compound that is more stable in terms of local energy than the fingerprint of compound from the binding compound, and adding the produced fingerprint of compound to the fingerprint set of binding compound.

In a specific example according to the invention, the present invention uses an interaction calculating program such as the Circle program, in regard to the various low molecular compounds bound to the target family macromolecular protein set from the CElib, so that the interaction with a ligand would be stabilized with respect to the complexes of each family macromolecular protein and ligand. According to the present invention, the kind of atom or the kind of bonding in a fingerprint (fp) unit, that is, a chemical descriptor unit, is ameliorated or modified, and this is used as a new fingerprint (fp) unit, that is, a new chemical descriptor unit, to adopt the unit as the counterpart FP in the superimposition of FP, such as in the instance of the new unit being used as a new FP as described for the previously discussed inventions.

Furthermore, the FP of the CElib, which is a database of various low molecular compounds bound to a family macromolecular protein set that is similar in 3-dimensional structure to a target protein among target macromolecules, contributes largely to the determination of docking score. Thus, according to the present invention, when the docking structure of an ideal low molecular ligand that binds to a target macromolecular protein, has been completely analyzed experimentally in the present invention, if various substituted groups are added so as to improve the interaction energy by using a low molecular ligand that is ideal to the binding, as a lead compound, or if an arbitrary low molecular ligand is found, whose Tanimoto coefficient, which is a quantifying function of the fingerprint of compound, is very similar to that of an ideal low molecular ligand, that is, close to 1, the FP region is limited to a region (for example, 4 or 5 Angstroms) surrounding with an ideal low molecular ligand that has been completely analyzed experimentally. Thereby, according to the present invention, the docking structure and the score of these compounds having a similar chemical structure, that is, having a very similar Tanimoto coefficient, can be easily calculated. This corresponds to the lead optimization of a binding compound or a design de novo of a compound, and has high effects, unlike the conventional techniques, for the combination with the role of the FP in the inventions described above, and is useful.

Conventionally, when a functional group such as a benzene ring, which is a partial moiety of various low molecular compounds, is bound to a target protein to thereby obtain a physically stable partial structure, and these results are used to calculate the interaction between a target protein and various low molecular compounds containing a large extent of intramolecular free rotation, reducing the occurrence of the docking conformation has been generally implemented. According to the present invention, modified FPs are created by calculating the interaction energy with the target protein using a program that can evaluate the stability, such as the "Circle," which is a technique of bioinformatics. In this regard, publications such as literature articles are not found, and when the superimposition of FP is adopted as the basic for the calculation of docking, there is no conventional technique which reports the usage of a modified FP as the basic for the calculation of the docking, as in the case of the present invention. The present invention has excellent effects, unlike the conventional techniques, and thus is useful.

The present invention is the apparatus for in silico screening according to still another aspect of the present invention, wherein the binding compound is a compound that is predicted by a known docking algorithm to have a stable conformation with respect to the target protein.

In a specific example according to the invention, the present invention adopts a first-principle approach (Ab-initio Approach), which uses a physical potential function such as hydrogen bond, hydrophobic interaction, or electrostatic interaction, that are conventionally implemented in general. For example, the present invention adds an FP (fingerprint) extracted from the 3-dimensional coordinates of a low molecular compound that is predicted such that a stable conformation has a high score, by the docking calculation using an existing docking software such as DOCK, AutoDock or GOLD, such as that a fraction that can predict the correct structure with an rmsd of 2.0 or less has been verified by a blind test concealing the correct structure.

According to the present invention, the conformation obtained by scoring of the interaction between a target protein and various low molecular compounds, may be used as the initial conformation for existing docking software programs such as DOCK, AutoDock and GOLD. As a result, the initial conformation obtained for the present invention can be conveniently obtained, and also, since the accuracy of reproduction of experiments is high, useful results are obtained from a combination with other software programs.

The present invention is the apparatus for in silico screening according to still another aspect of the present invention, wherein the optimizing unit further includes an interaction score calculating unit that calculates the interaction score, based on a function that takes into consideration of not only the root-mean-square deviation for a unit of fingerprint of compound but also the collision state of the candidate compound with the target protein, the existential rate of the candidate compound in the region of interaction of the target protein, and the fraction of direct interaction of the candidate compound with the target protein.

The present invention is the apparatus for in silico screening according to still another aspect of the present invention, wherein the optimizing unit optimizes the interaction score by determining the interaction score based on the Metropolis method, and modifying, increasing or decreasing the basal fingerprint of compound from the candidate compound according to the results of determination.

In a specific example according to the present invention, if the score of this round is larger than the score of the previous round, the Metropolis decision of the present invention accepts the structure of the candidate ligand. If the score is smaller, the adopting probability, Paccept, is calculated, and whether the structure of a candidate ligand should be rejected or accepted may be determined based on the Paccept.

The present invention is the apparatus for in silico screening according to still another aspect of the present invention, wherein the optimizing unit further includes a structure transforming unit that repeatedly changes the conformation of the candidate compound in the process of optimizing the interaction score, and repeatedly translates or rotates the candidate compound as a rigid body, for each of the conformations of the candidate compound based on a simulated annealing method, and the optimizing unit calculates the interaction score of the candidate compound for each of the conformations translated or rotated by the conformation transforming unit.

In another specific example according to the present invention, for FPs including the several information on the 3-dimensional coordinates of various low molecular compounds bound to a family macromolecule set having a similar 3-dimensional structure of the target protein, mathematical calculation is repeatedly performed by Monte Carlo type simulated annealing, so that the score would be the highest, in order to look for a conformation optimal for the interaction by docking low molecular compounds from a library of virtual compounds to the target protein.

More specifically, first, the present invention changes the conformation by randomly varying the rotatable dihedral angle of a candidate ligand, and uses the coordinates of the candidate ligand with the changed conformation. The present invention randomly selects ten FPs from an FP band derived from the binding compound set bound to a family protein of the target protein. The present invention then randomly selects a candidate ligand from the selected FP band, and an FP atomic coordinate set from the LIBRARY LIGANDS. Then, the present invention uses this state as the fingerprint (FP) alignment, and performs least square fitting for a correspondence relationship thereof. The present invention calculates the interaction score using the root-mean-square deviation (rmsd) of the superimposition at that time, and the atomic coordinates of a candidate ligand after the superimposition. Then, from the second round, the present invention stores the state of the previous round, and performs translation and rotation while maintaining the conformation of the ligand atoms, that is, rigid body translation and rotation. The present invention performs an increase or decrease of one FP, and modification and addition of the correspondence relationship of the atomic coordinate set. The present invention performs this step, for example, 10,000 times. Here, the temperature of the simulated annealing may be decreased, starting from 30 K and down to 0.07 K. As such, the present invention calculates the maximum value of the score of one conformation, compares the value for 1000 conformations generated initially, and predicts and outputs the structure with the maximum score as the protein-ligand complex structure. At this time, the process of ranking the 1000 conformations in terms of the score may be devised in regard to the time for calculation or the search for the maximum value, by using a genetic algorithm or the like.

The present invention is the apparatus for in silico screening according to still another aspect of the present invention, wherein the optimizing unit calculates the interaction score based on the following mathematical formula (1):

$$\begin{aligned} FPAScore &= F(aligned_fp, fp_rmsd, molecule) \\ &= BaseScore(aligned_fp, fp_rmsd) \\ &\times fp_volume(molecule) \\ &\times fp_contact_surface(molecule) \end{aligned} \quad (1)$$

wherein the FPAScore represents the interaction score, the F(aligned_fp,fp_rmsd, molecule) is a function using, as variables, the degree of alignment and the root-mean-square deviation of the unit of fingerprint of compound between the binding compound and the candidate compound, and the 3-dimensional structure of the candidate compound with respect to the target protein, the BaseScore(aligned_fp,fp_rmsd) is an index representing the degree of consistency and crowded degree of the unit of fingerprint of compound, the fp_volume(molecule) is an index representing the fraction occupied by the candidate compound in a space formed by the 3-dimensional coordinates of the fingerprint set of binding compound, and the collision state with the target protein, and the fp_contact_surface(molecule) is an index representing the contacting degree of the candidate compound with the target protein, and the degree of attribution to the 3-dimensional coordinates of the fingerprint set of binding compound.

As described above, these mathematical calculations described above according to the invention calculate the interaction between a target protein and a low molecular compound of library of virtual compounds using a conventional physical interacting function. However, the mathematical calculation is different from conventional techniques in view of semiempirically performing the calculation using the information of bioinformatics, and the success ratio of the structure prediction exhibits excellent effects such that the mathematical calculation according to the present invention is never inferior to the world-renowned docking software programs, no matter however excellent the programs are. Furthermore, since accumulation of information leads the results of the calculation of interaction by the semiempirical bioinformatics technique to be favorable, the mathematical calculations are useful, unlike the conventional techniques.

The present invention is the apparatus for in silico screening according to still another aspect of the present invention, wherein the BaseScore (aligned_fp, fp_rmsd) in the mathematical formula (1) is calculated based on the following mathematical formula (2):

$$BaseScore(aligned_fp, fp_rmsd) = \frac{RawScore(aligned_fp)}{1 + \ln(fp_rmsd^{k1} + 1)} \quad (2)$$

wherein the RawScore(aligned_fp) is an index based on the number of atoms in the fingerprints of compound that are aligned between the binding compound and the candidate compound, and the fp_rmsd is the root-mean-square deviation, the fp_volume(molecule) is calculated based on the following mathematical formula (6):

$$fp_volume(molecule) = \ln\frac{1.0 + nafp^{k2}}{1.0 + nap^{k3}} \quad (6)$$

wherein the nafp is the number of lattice points occupied by the 3-dimensional coordinates of the candidate compound in a region of proper grid based on the 3-dimensional coordinates of the fingerprint set of binding compound, the nap is the number of lattice points to which the 3-dimensional coordinates of the candidate compound fall into the region of proper grid of the atoms in the 3-dimensional structure of the target protein, and the k2 and k3 are arbitrary constants, and the fp_contact_surface(molecule) is calculated based on the following mathematical formula (7):

$$fp_contact_surface(molecule) = \frac{\sum_{i=1}^{n} density_of_atom(atom(i))}{total_density_of_atom(molecule)} \quad (7)$$

wherein n is the number of atoms of the candidate compound, atom(i) is the 3-dimensional coordinates of the *i*th atom in the candidate compound, the density_of_atom(atom(i)) is a function that reciprocates the sum of the number of atoms in the target protein contacting with the atoms of the fingerprint of compound at a predetermined distance and the number of atoms in the binding compound falling into the same lattice points of the fingerprint of compound when the 3-dimensional coordinates of the atom belong to the fingerprint of compound of the fingerprint set of binding compound, and the total_ density__of_atom(molecule) is the number obtained by reordering the distribution of the density_of_atom in a descending order, and summing the numeric values in order as many times as the number of atoms in the candidate compound.

In a specific example according to the present invention, the present invention looks for already known active compounds with respect to an intrinsic target protein such as EGFR or VEGFR so as to clarify the values of k2 and k3 in the matter described above, and optimizes k2 and k3. The present invention relates to a method for performing in silico screening so that the values become, for example, k2=2.0 and k3=1.0, in the in silico screening of an inhibitor of EGFR. Since adequately listing up compounds that fit into the intrinsic target protein such as EGFR or VEGFR, by the in silico screening according to the present invention, is directly related to the development of new drugs for anticancer agent, the method of the present invention has excellent effects, unlike the conventional techniques, and is thus useful.

Typically, a docking software program such as GOLD is devised to select a good set in a genetic algorithm using the atoms participating in the biologically important hydrogen bond as a point or a vector. Such a point or vector is distinguished from the FP of the 3-dimensional descriptor, which is an element as the conditions for extracting the collective conformation in which various low molecular compounds are bound to a family macromolecule set that is similar to the 3-dimensional structure of the target protein as described in the aspect of the invention previously described. According to this embodiment of the invention, when an atom point or vector set constituting the biologically important hydrogen bond or the like is to be took in from the interacting conformation according to the aspect of the invention described above, if the fp_rmsd value is obtained by the following formula, the atoms participating in the biologically important hydrogen bond, hydrophobic bond or van der Waals interaction can be incorporated into the invention described above without any problem.

That is, according to the present invention, the formula of the fp_rmsd+distance rmsd indicative atom set composed of important points vectors may be extended to the form of fp_rmsd**k1+distance_rmsd**k4 (**k1<<**k4 indicates small contribution to FP: **k1>>**k4 emphasizes the contribution to FP), or may be extended to the form of distance_rmsd**k4. Here, when there are biologically important hydrogen bond, hydrophobic bond or van der Waals interaction for the ligand atoms at the ligand binding site of a target protein in the interaction between the target protein and a low molecular compound that is docking, the distance_rmsd is defined as the least square error of the ideal coordinates at the ligand binding site of the target protein, and the final point coordinates of a vector generated from the biologically important atoms, or nearby atoms, of the target protein.

According to the present invention, in regard to various low molecular compounds, when most of the compounds are peptides having amino acid residues linked up, since the correspondence relationship of FP becomes complicated because of the large number of peptide groups, the correspondence relationship is underestimated in the process of score calculation. Thus, the part corresponding to the formula for the FP of the peptide moieties in the mathematical formula for the RawScore according to the present invention may be replaced with an underestimated number such as zero.

That is, according to the present invention, in regard to the method for calculating the interaction of a target protein and a docking low molecular compound on the basis of the FP, any of devices such as the grid information of the environment for ligand binding of a target protein that is a target macromolecule, the multipoint information of a compound which emphasizes a vector between the compound and the target macromolecule, and a vector directed from a compound representing the biological environment of the target protein to the target protein, is implemented. In addition to that, another aspect of the present invention is an extended invention of the invention described above, such as including and merging a method for calculating the interaction energy or the like from an interatomic potential formula of classical physics between various atoms of a compound and various atoms constituting a target macromolecular protein. This aspect of the invention relates to determining the order related to the conformation or the strength of binding in interaction of compounds, to be expressed as score values. Thus, the present invention has excellent effects, unlike the conventional techniques, and thus is useful.

An method of in silico screening executed by an apparatus for in silico screening for performing screening of candidate compounds that bind to a target protein includes a storage unit and a control unit, wherein the storage unit includes a compound database produced by extracting a chemical descriptor that includes the atom type and the interatomic bonding rules as the fingerprint of a compound related to a plurality of atoms in the compound, for each of the candidate compounds, and the method includes a fingerprint of compound producing step of extracting the fingerprint of compound from a binding compound known to bind to a family protein, which has a 3-dimensional structure that is identical or similar to that of the target protein, along with 3-dimensional coordinates that have been converted into the coordinate system of the target protein, to thereby produce a fingerprint set of binding compound, and an optimizing step of computing, for the candidate compounds stored in the compound database, the 3-dimensional structures of the candidate compounds with respect to the target protein, so that the interaction scores that are based on the root-mean-square deviations of each unit of fingerprint of compound that have been calculated using the 3-dimensional coordinates of the fingerprint set of binding compound as a basic, are optimized, wherein the steps are executed by the control unit.

Therefore, according to the present invention, binding between a protein and a compound can be predicted with high accuracy, and a large number of compounds that get a hit can be selected. Furthermore, semiempirical screening can be performed while taking into consideration of the information of biochemical experiments or the like, and an effect of increasing the prediction efficiency is obtained.

BRIEF DESCRIPTION OF DRAWINGS

Fig. 1 is a block diagram showing one example of a configuration of an apparatus for in silico screening according to the present invention; Fig. 2 is a flowchart showing one example of a processing of the apparatus for in silico screening 100; Fig. 3 is a situation diagram representing the docking method according to the present example, based on a conventional docking software program and bioinformatics making effective use of a number of X-ray structures or NMR structures of protein-ligand complexes; Fig. 4 is a block diagram showing a principle of docking between a protein and a ligand according to the present example (ChooseLD); Fig. 5 is a diagram depicting an example of the method of producing an FP (fingerprint); Fig. 6 is a chart presenting the list of character strings of the atoms used in the present example; Fig. 7 is a schematic diagram depicting the method for calculating similarity between compounds based on the Tanimoto coefficient; Fig. 8 is a schematic diagram presenting the FP in the case of docking a ligand to the binding site of a target protein as an example; Fig. 9 is a diagram depicting an example of the process of obtaining atomic coordinates from the traced path and registering the atomic coordinates on an FP band; Fig. 10 is a diagram depicting an example of the method step of arranging to be decreasing fingerprint band in the present example; Fig. 11 is a schematic diagram presenting an example of the process of defining a correspondence relationship between coordinate vectors; Fig. 12 is a diagram depicting a specific example of nafp and nap by using the ligand having the number of atoms of 31; Fig. 13 is a diagram depicting an example of the location of a ligand derived from the FP library in the neighborhood of the binding site of the target protein; Fig. 14 is a conceptual diagram depicting an example of the process of simulated annealing; Fig. 15 is a diagram schematically depicting the FP alignment and the least square fitting for calculating the FPAScore; Fig. 16 is a diagram presenting the distribution of calculation time in the in silico screening of EGFR; Fig. 17 is a diagram depicting an example of the outline of benchmark; Fig. 18 is a diagram presenting the yearly distribution of the number of registrations on the PDB; Fig. 19 is a table summarizing the rmsd between the prediction and the experimental results; Fig. 20 is a chart presenting a list of ratio of predictive success (relationship between k1 and Tc range) in the 85 sets; Fig. 21 is chart presenting the fractions capable of prediction within the $10^{th}$ rank with an rmsd of 2.0 or less; Fig. 22 is chart presenting the fractions capable of prediction within the $10^{th}$ rank with an rmsd of 2.5 (Close) or less; Fig. 23 is a chart representing the case of performing with a value other than 2.0Å for the rmsd with a correct structure that is regarded as successful; Fig. 24 is a chart presenting the results of benchmarking of the Dock, AutoDock and GOLD as compared to the results of the ChooseLD; Fig. 25 is a diagram presenting the frequency distribution of collisions with the respective target proteins when the rmsd of the predictive structure based on the FPAScore and the experimental structure is 2.0Å or less in the benchmarking of 85 sets; Fig. 26 is a diagram presenting the frequency distribution of predictively successful structure in the benchmarking of 85 sets; Fig. 27 is a diagram presenting the counting of the number of successes in performing docking with each target total 10 times; Fig. 28 is a diagram presenting the results of the rmsd distribution of the predictive structures of DOCK, AutoDock and GOLD and the results of the ChooseLD method in the benchmarking of 133 sets; Fig. 29 is a diagram presenting the results of the rmsd distribution of the predictive structures of DOCK, AutoDock and GOLD and the results of the ChooseLD method in the benchmarking of 133 sets; Fig. 30 is a diagram indicating the number of successes in performing docking with each target total 10 times; Fig. 31 is a diagram indicating the number of successes in performing docking with respect to each target total 10 times; Fig. 32 is a diagram presenting the probability of obtaining a structure having an rmsd with an experimental structure of 2.0Å or less from the distribution of FPAScore ranking in the FP library that has been limited to the Tc range; Fig. 33 is a diagram presenting the probability of obtaining a structure having an rmsd with an experimental structure of 2.0Å or less from the distribution of FPAScore ranking in the FP library that has been limited to the Tc range; Fig. 34 is a diagram presenting the frequency distribution of collisions of the predictively successful structure; Fig. 35 is a diagram presenting the performance in the case of further lowering the upper limit value of the Tc range of the ligands used in the FP library to be 0.16, 0.24, 0.36, and to be 0.08 as the lower limit value, and the ratio of predictive success in the Tc range described above, namely, 0.56, 0.76, 0.96 as the upper limit value, and 0.08 as the lower limit value; Fig. 36 is a diagram showing the predicted protein-ligand complex structure for 1DR1; Fig. 37 is a diagram showing the predicted protein-ligand complex structure for 4EST; Fig. 38 is a diagram presenting the targets that GOLD has failed, but ChooseLD has succeeded in prediction for 1CDG; Fig. 39 is a diagram presenting the targets that GOLD has failed, but ChooseLD has succeeded in prediction for 1DR1; Fig. 40 is a diagram presenting the targets that GOLD has failed, but ChooseLD has succeeded in prediction for 1LDM; Fig. 41 is a diagram presenting the targets that GOLD has failed, but ChooseLD has succeeded in prediction for 4EST; Fig. 42 is a chart presenting the ratio of predictive success for 90 targets in the 133 sets; Fig. 43 is a chart presenting the degree of similarity of the PDBIDs of a successfully predicted target protein between the docking software programs, calculated in terms of Tc (Tanimoto coefficient); Fig. 44 is a cross table showing success and failure of prediction by the respective docking software programs with respect to one target protein among the 90 targets; Fig. 45 is a diagram presenting the targets that DOCK failed but ChooseLD succeeded in prediction for 1HYT; Fig. 46 is a diagram presenting the targets that DOCK failed but ChooseLD succeeded in prediction for 1PHG; Fig. 47 is a diagram presenting the targets that DOCK failed but ChooseLD succeeded in prediction for 1TMN; Fig. 48 is a diagram presenting the fraction for which the structure with an rmsd of 2.0 can be collected not only for the $1^{st}$ rank but also within the $10^{th}$ rank; Fig. 49 is a diagram presenting the fraction for which the structure with an rmsd of 2.5 (Close) can be collected not only for the $1^{st}$ rank but also within the $10^{th}$ rank; Fig. 50 is a chart presenting the instance of changing the rmsd that is defined as successful; Fig. 51 is a table showing the result of processing according to the present example; Fig. 52 is a diagram presenting an intracellular signal transduction pathway starting from EGFR; Fig. 53 is a diagram presenting the alignment of the amino acid sequence of EGFR; Fig. 54 is a diagram presenting constructed model of EGFR; Fig. 55 is a diagram showing the 2-dimensional structure of the obtained eleven inhibitors; Fig. 56 is a diagram presenting a line chart of harvest rate when the k2 value defined for the FPAScore was changed in the range of 0.5 to 5.0; Fig. 57 is a diagram presenting a line chart of harvest rate when the k3 value defined for the FPAScore was changed in the range of 0.5 to 2.0; Fig. 58 is a diagram presenting the results of in silico screening for the respective Tc ranges, when the Tc upper limit value was set at 1.00, and the range of the Tc lower limit value was changed from 0.08 to 0.32 at an increment of 0.08; Fig. 59 is a diagram presenting the PDBIDs for which the protein-ligand complex structures are registered on the PDB are already known, and the ranking of their ligands; Fig. 60 is a diagram of corresponding the ligand IDs and the compound names in Fig. 59; Fig. 61 is a diagram presenting the protein-ligand complexes of high ranking to the $10^{th}$ rank, as a result of refined selection by in silico screening of Kinase; Fig. 62 is a diagram from another angle of Fig. 61; Fig. 63 is a diagram presenting the neighborhood of the TGF-α binding domain; Fig. 64 is a diagram presenting the results of in silico screening for the TGF-α binding domain of EGFR using the MDL Comprehensive Medicinal Chemistry (MDL CMC) Library; Fig. 65 is a diagram presenting the results of the same in silico screening using the MDL ACD Library; Fig. 66 is a diagram showing the 2-dimensional structure of KRN633 (IC50 = 1.16nm/L); Fig. 67 is a diagram showing the 2-dimensional structure of KRN951 (IC50 = 0.16nm/L); Fig. 68 is a diagram presenting a set of top ten ligands used in the docking for the ligands that belong to the FP library used in the docking with the neighborhood of the VEGFR2 binding site of KRN633; Fig. 69 is a diagram presenting 10 structures that have been predicted by performing the ChooseLD method ten times for KRN633, together with the 3-dimensional structure in the neighborhood of the binding site of VEGFR2; Fig. 70 is a diagram presenting a set of top ten ligands used in the docking with the neighborhood of the VEGFR2 binding site for the ligands that belong to the FP library used in the FP library of KRN951; Fig. 71 is a diagram presenting 10 structures predicted by performing the ChooseLD method 10 times for KRN951, together with the 3-dimensional structure in the neighborhood of the binding site of VEGFR2; Fig. 72 is a diagram presenting a graph for the ratio of predictive success when the Tc lower limit value obtained as a result of a docking performance testing of the ChooseLD method using the 133 sets, was set at 0.08, and the Tc upper limit value was varied, with the horizontal axis presenting the Tc upper limit value and the vertical axis presenting the success rate; Fig. 73 is a diagram showing the 3-dimensional structure of enoyl acyl carrier protein; Fig. 74 is a diagram presenting the structures of the top ten FPAScore as a result of performing in silico screening of enoyl acyl carrier proteins, using the MDL Comprehensive Medicinal Chemistry (MDL CMC) Library; Fig. 75 is a diagram showing the alignment between the amino acid sequence of AMPKhomoGAMMA1 and 2V9J_E; Fig. 76 is a diagram presenting the result list of the CMC pharmaceutical products in which a ligand is bound to the entirety of a receptor; Fig. 77 is a diagram collectively presenting the states of binding to 2V9J_E (AMPKhomoGAMMA1) receptor, listed from the 1$^{st}$ rank to the 10$^{th}$ rank.

BEST MODE(S) FOR CARRYING OUT THE INVENTION

The following describes an embodiment of an apparatus for in silico screening, and a method of in silico screening according to the present invention in detail with reference to the drawings. The embodiment is illustrative only, and is not intended to limit the present invention in any way.

[Outline of the Present Invention]

The following outlines the present invention, and then, a configuration and processing of the present invention are explained in detail.

Currently, 3-dimensional coordinates approximating 40,000 in number are registered on the PDB (Protein Data Bank). The 3-dimensional coordinates represent the state in which various compounds such as peptides, low molecular compounds or metals are directly interacting with target macromolecules, based on experiments such as X-ray analysis, an NMR experiment, an electron beam analysis experiment, or a high-resolution electron microscope photography. Furthermore, as a result of progress in the performance of computer and in the bioinformatics, a family macromolecular protein set, which has a 3-dimensional structure similar to that of a target macromolecular protein having various compounds bound thereto, is easily obtained, and can be extracted, by websites such as SCOP, or the program produced by the applicant of the present invention, which shows excellent results in the CASP.

Under such circumstances, the inventors of the present invention conceived an idea that if bioinformatics could be used by utilizing the collective overlapped state of various compounds bound to a target macromolecular protein, instead of the technique of determining the order of in silico screening of compounds from the results of the interaction energy obtained using the conformation, or the score value obtainable at that time, of the compounds that directly bind to a target macromolecular protein, which has been traditionally determined in general in a classical physical manner, it would be possible to determine the order by in silico screening of compounds from the results of the interaction energy using the conformation, or the score value obtainable at that time, of compounds based on the human intelligence.

The present invention was made as a result of the present application devotedly conducting investigation based on the above idea by the inventors, and the present invention roughly has the following fundamental features. That is, the present invention is an apparatus for in silico screening for performing screening of candidate compounds that bind to a target protein including at least a storage unit, and a control unit, wherein the storage unit includes a compound database produced by extracting a chemical descriptor that includes the atom type and the interatomic bonding rules as the fingerprint of a compound related to a plurality of atoms in the compound, for each of the candidate compounds.

Here, the term "fingerprint of compound" (fingerprint: FP) is more specifically a chemical descriptor that includes the atom type of atoms, such as two, three or four atoms, in a compound, and the interatomic bonding rules. The "atom type" is, for example, the Sybyl atom type, or the "valence type" and so on. The "interatomic bonding rules" represents the chemical bonding state between atoms, and for example, represents the bonding rules such as a single bond, a double bond or the bonding in aromatic rings, or the category classification according to the molecular orbital method.

The apparatus for in silica screening extracts the fingerprint of compound from a binding compound known to bind to a family protein, which has a 3-dimensional structure that is identical or similar to that of the target protein, along with 3-dimensional coordinates that have been converted into the coordinate system of the target protein, to thereby produce a fingerprint set of binding compound. That is, in the coordinate system of the target protein, the screening apparatus gathers the collective conformation of a group of the 3-dimensional structure of compounds binding to the target protein, and extracts the fingerprints of compound in correspondence to the 3-dimensional coordinates.

Here, the "family protein, which has a 3-dimensional structure that is identical or similar to that of a target protein," may be the target protein itself, or may be a protein having a partial structure (for example, an active site, a ligand-binding site or the like) that is identical or similar to that of the target protein. Alternatively, an identical or similar protein may also be used without analyzing the 3-dimensional structure of the target protein and thereby specifying the active site. To allow a stable conformation to have a higher score, there has been a need to analyze the 3-dimensional structure of the target protein in advance and to specify the active site, in the calculation of docking using existing docking software programs such as conventional DOCK, AutoDock and GOLD. However, in comparison thereto, the present invention has a superior effect that is different from that of the conventional techniques, and the invention is useful because it is not necessary to specify the active site through a study of the literature.

Also, a homology search from a protein database which stores the 3-dimensional structures and amino acid sequences of proteins that are bound to a compound, may be carried out using the amino acid sequence of the target protein as a query sequence, and a protein found to a certain value or higher for an index that represents similarity in the structure through the superimposition of conformations with the target protein, may be designated as the family protein. Here, the "binding compound (that is) already known to bind to a protein" may be a compound for which the 3-dimensional structure of the protein-compound complex has been experimentally confirmed by X-ray structural analysis, NMR structural analysis or the like. The binding compound may be acceptable merely the compound that is known to bind to a protein, or may be a compound predicted to have a stable conformation with respect to a target protein by a known docking algorithm (DOCK, AutoDock, GOLD, or the like) or any program for generating coordinates (Corina, or the like).

Here, in order to converts the 3-dimensional coordinates of the binding compound into the coordinate system of the target protein, an operation of superimposing the conformations of the family protein and the target protein may be performed by the apparatus for in silico screening, to thereby convert a binding compound that has been bound to the family protein, along with the coordinates of the binding compound from the coordinate system of the family protein into the coordinate system of the target protein. For example, the operation of superimposition of conformations may be carried out based on an algorithm for the superimposition of conformations between proteins (CE or the like), which does not take the kind of atom into consideration, or if the homology between the target protein and the family protein is high, the superimposition of conformations may also be performed, with the kind of atom taken into consideration.

Extraction of the fingerprint of compound is not limited to direct extraction from a binding compound, but an arbitrary fingerprint of compound may also be added as necessary, for the purpose of searching for candidate compounds with respect to the target protein. For example, the superimposition of conformations may be performed by referring to another compound that is different from the binding compound, to thereby produce a new fingerprint of compound that straddles between the atoms of the binding compound and the atoms of the other compound, and the new fingerprint of compound may be added to the fingerprint set of binding compound. Alternatively, the interaction energy with respect to the target protein may be calculated for a compound that is analogous to the binding compound on the basis of the Tanimoto coefficient, using a program ("Circle" or the like) that can evaluate the stability by interchanging the kind of atom between the atoms of the binding compound and the atoms of the relevant compound, to thereby newly produce a fingerprint of compound that is more stable in terms of local energy than the fingerprint of compound from the binding compound, as a "modified fingerprint of compound (modified FP)," and this modified fingerprint of compound may be added to the fingerprint set of binding compound. That is, in the case of using, as a lead compound, a low molecular compound that is ideal to the binding to a target protein, to add various substituted groups to improve the interaction energy, or in the case of finding an arbitrary low molecular compound whose Tanimoto coefficient, which is a quantifying function of the fingerprint of compound, is very similar to that of an ideal low molecular compound, that is, close to 1, the region of the fingerprint of compound is limited to 4 or 5 Angstroms, which is a region surrounding with an ideal low molecular compound that has been completely analyzed experimentally. Thereby, the docking structure of compounds having similar chemical structures, that is, very similar Tanimoto coefficients, and the interaction score thereof, can be easily calculated.

The apparatus for in silico screening computes, for the candidate compounds stored in the compound database, the 3-dimensional structures of the candidate compounds with respect to the target protein, so that the interaction scores that are based on the root-mean-square deviations (rmsd) of units of fingerprint of compound that have been calculated using the 3-dimensional coordinates of the fingerprint set of binding compound to be fixing coordinates as a basic, are optimized.

That is, in this process for optimization, for example, the apparatus for in silico screening determines, according to the Metropolis method, the interaction score that has been calculated, on the basis of the root-mean-square deviation, after repeatedly changing the conformation of a candidate compound, and repeatedly translating or rotating the candidate compound as a rigid body, for each of the conformations of the candidate compound, and modifies, increases or decreases the fingerprint of compound from the candidate compound according to the results of determination. Here, a fingerprint set of binding compound to be fixing coordinates that will serve as the basic, may be selected by randomly extracting several fingerprints of compound. Alternatively, instead of changing the conformation by randomly modifying the rotatable dihedral angle of the candidate compound, the conformations of the candidate compound may also be changed by storing the previous conformations in memory, such as in the case of a genetic algorithm.

The calculation of the interaction scores in the optimization process is carried out, for example, based on a function that takes into consideration of the collision state of the candidate compound with the target protein, the existential rate of the candidate compound in the region of interaction of the target protein, and the fraction of direct interaction of the candidate compound with the target protein, which function is based on the root-mean-square deviation of the unit of fingerprint of compound. More specifically, the interaction scores are calculated based on the following mathematical formula (1):

$$\begin{aligned} FPAScore &= F(aligned_fp, fp_rmsd, molecule) \\ &= BaseScore(aligned_fp, fp_rmsd) \\ &\times fp_volume(molecule) \\ &\times fp_contact_surface(molecule) \end{aligned} \quad (1)$$

wherein the FPAScore represents the interaction score, the F(aligned_fp,fp_rmsd, molecule) is a function using, as variables, the degree of alignment and the root-mean-square deviation of the unit of fingerprint of compound between the binding compound and the candidate compound, and the 3-dimensional structure of the candidate compound with respect to the target protein, the BaseScore(aligned_fp,fp_rmsd) is an index representing the degree of consistency and crowded degree of the unit of fingerprint of compound, the fp_volume(molecule) is an index representing the fraction occupied by the candidate compound in a space formed by the 3-dimensional coordinates of the fingerprint set of binding compound, and the collision state with the target protein, and the fp_contact_surface(molecule) is an index representing the contacting degree of the candidate compound with the target protein, and the degree of attribution to the 3-dimensional coordinates of the fingerprint set of binding compound.

Thus, the outline of the processing of the present invention is as discussed above. As such, the ranking of the candidate compounds for the interaction with respect to the target protein is determined on the basis of the interaction scores that have been calculated according to the optimization technique, and a significant candidate compound can be inferred from the compound database. Therefore, the binding between a protein and a compound can be predicted with high accuracy, and also, a large number of compounds that get a hit can be selected. Furthermore, it is possible to perform semiempirical screening while taking the information of biochemical experiments and the like into consideration, and the prediction efficiency can be increased.

In other words, the present invention has been achieved as a result of contemplating that the conformations of various low molecular compounds (binding compounds) that are collectively bound to a family protein, which has a 3-dimensional structure that is identical or similar to that of a target protein, are close to the most stable conformation that has made interaction with the target protein. Furthermore, the present invention can perform semiempirical in silico screening with higher prediction efficiency than conventional techniques, by scoring of appropriate interaction scores using an easily handlable fingerprint of compound as a unit, and optimizing, when a comparison is made between a binding compound and a candidate compound.

[Configuration of the Apparatus For in silico Screening]

The following first describes a configuration of the apparatus for in silico screening. Fig. 1 is a block diagram showing an example of a configuration of the apparatus for in silico screening to which the present embodiment is applied, and conceptually shows only parts related to the present invention.

In Fig. 1, in general, the apparatus for in silico screening 100 is composed of a control unit 102 such as a CPU that integrally controls the entire apparatus for in silico screening 100, a communication control interface 104 connected to a communication device (not shown) such as a router connected to a communication line, an input/output control interface 108 connected to an input device 112, and an output device 114, and a storage unit 106 that stores various databases and tables, and the units are communicably connected through an optional communication chancel. Further, the apparatus for in silico screening 100 is communicatably connected to a network 300 via a communication device such as a router and a wire or wireless communication line such as a special line.

The various databases and tables (such as a candidate compound DB 106a, a fingerprint set of binding compound 106b, and a medicinal chemical compound DB 106c) stored in the storage unit 106 are storage units such as fixed disk devices, and store various programs, various tables, various file, various databases, various web pages, and the like used in various processes.

Among these respective constituent elements of the storage unit 106, a candidate compound DB 106a is a candidate compound database unit that has been produced by extracting a fingerprint of compound for each of the compounds serving as candidates for in silico screening (referred to as "candidate compounds").

A fingerprint set of binding compound 106b is a storage means for fingerprints of binding compound, which stores a fingerprint set of binding compound produced by extracting fingerprints of compound for the compounds known to bind (referred to as "binding compounds") to a protein having a 3-dimensional structure that is identical or similar to that of a target protein (referred to as "family protein"), along with the 3-dimensional coordinates that have been converted into the coordinate system of the target protein.

A medicinal chemical compound DB 106c is a medicinal chemical compound database that stores a fingerprint set of medicinal chemical compound produced by extracting fingerprints of compound for known medicinal chemical compounds, such as the MDL CMC Library. That is, the Medicinal chemical compound DB 106c is used to produce the fingerprint set of binding compound 106b that is specialized in drug absorption, drug metabolism, drug excretion or drug toxicity, which have been organized in advance using drug absorption, drug metabolism, drug excretion, drug toxicity or the like as an index, and using the fundamental data units as the bases for the organization of fingerprints of compound, in order to take out compound information using the pharmaceutical database.

In Fig. 1, the communication control interface 104 controls communication between the apparatus for in silico screening 100 and the network 300 (or a communication device such as a router). That is to say, the communication control interface 104 has a function to communicate data to another terminal through a communication line.

In Fig. 1, the input/output control interface 108 controls the input device 112, and the output device 114. As for the output device 114, a monitor (including TV set), and a speaker can be used (the output device 114 may be described below as the monitor). As for the input device 112, a keyboard, a mouse, a recording medium reading device or the like can be used. The target proteins or candidate compounds, which are object of the in silico screening, are input through this input device 112.

In Fig. 1, the control unit 102 includes an internal memory that stores a control program such as an operating system (OS), programs specifying various processing procedures, and necessary data and performs information processing for executing various pieces of processing by using these programs. The control unit 102 functionally and conceptually includes a fingerprint of compound producing unit 102a, an optimizing unit 102b, a screening result output unit 102c, and a homology searching unit 102d.

The fingerprint of compound producing unit 102a is a unit that produces fingerprints of compound by extracting fingerprints of compound from compounds such as candidate compounds, binding compounds or medicinal chemical compounds. For example, the fingerprint of compound producing unit 102a produces a fingerprint set of candidate compound by extracting fingerprints of compound for candidate compounds that have been input via the input device 112, and stores the fingerprint set of candidate compound in the candidate compound DB 106a. The fingerprint of compound producing unit 102a also produces a fingerprint set of medicinal chemical compound by extracting fingerprints of compound from medicinal chemical compounds that have been acquired, and stores the fingerprint set of medicinal chemical compound in the medicinal chemical compound DB 106c.

The fingerprint of compound producing unit 102a also produces a fingerprint set of binding compound 106b by converting the 3-dimensional coordinates of atoms into the coordinate system of the target protein, and extracting fingerprints of compound for the binding compounds that are already known to bind to the family protein, along with the converted 3-dimensional coordinates. That is, the fingerprint of compound producing unit 102a gathers collective conformations for a group of the 3-dimensional structure of compounds binding to the target proteins on the coordinate system of the target protein, and extracts the fingerprints of compound in correspondence to the 3-dimensional coordinates. In other words, the fingerprint of compound producing unit 102a produces the fingerprint set of binding compound 106b by extracting, from the group of compound bound to the target protein, as many chemical descriptors as possible, which are called as fingerprints of compound, and which include the atom type of atoms, such as two, three or four atoms, and the interatomic bonding rules, together with the 3-dimensional coordinates of the chemical descriptors, and storing the chemical descriptors in the storage unit 106 in the form of a table of database.

Here, the fingerprint of compound producing unit 102a may perform, in order to convert the 3-dimensional coordinates of the binding compound into the coordinate system of the target protein, an operation of superimposing the conformations of the family protein and the target protein, and then convert the 3-dimensional coordinates of the binding compound that has been bound to the family protein, into the coordinate system of the target protein (from the coordinate system of the family protein). For example, the fingerprint of compound producing unit 102a may perform the operation of superimposition of conformations through an algorithm (CE or the like) for the superimposition of conformations between proteins (the target protein and the family protein), which does not take the kind of atom into consideration. If the homology between the target protein and the family protein is high, the superimposition of conformations may also be performed, with the kind of atoms taken into consideration.

The fingerprint of compound producing unit 102a is not limited to direct extraction of the fingerprint of compound from a binding compound, but may also add an arbitrary fingerprint of compound to the fingerprint set of binding compound 106b as necessary, for the purpose of searching for candidate compounds for the target protein. Here, the fingerprint of compound producing unit 102a includes, as shown in Fig. 1, a fingerprint of new compound adding unit 102e. That is, the fingerprint of new compound adding unit 102e is a unit for adding a new fingerprint of compound, which produces a new fingerprint of compound other than the fingerprints of compound extracted directly from the binding compounds, and adds the new fingerprint of compound to the fingerprint set of binding compound 106b. For example, the fingerprint of new compound adding unit 102e may perform the superimposition of conformations by referring to another compound which is different from the binding compound, to thereby produce a new fingerprint of compound that straddles between the atoms of the binding compound and the atoms of the other compound, and add the new fingerprint of compound to the fingerprint set of binding compound 106b. The fingerprint of new compound adding unit 102e may also calculate, for a compound that is similar to the binding compound on the basis of the Tanimoto coefficient, the interaction energy with respect to the target protein using a program ("Circle" or the like) that can evaluate the stability by interchanging the kind of atoms between the atoms of the binding compound and the atoms of the relevant compound, to thereby newly produce a fingerprint of compound that is more stable in terms of local energy than the fingerprint of compound from the binding compound, as a modified fingerprint of compound (modified FP), and add the modified fingerprint of compound to the fingerprint set of binding compound 106b.

The optimizing unit 102b is an optimizing unit that computes, for the candidate compounds stored in the candidate compound DB 106a, the 3-dimensional structures of the candidate compounds with respect to the target protein, so that the interaction scores that are based on the root-mean-square deviations (rmsd) of each unit of fingerprint of compound that have been calculated using the 3-dimensional coordinates stored in the fingerprint set of binding compound 106b as a basic, are optimized. For example, the optimizing unit 102b determines, according to the Metropolis method, the interaction scores calculated on the basis of the root-mean-square deviation, for each of the conformations of the generated candidate compounds and each of the 3-dimensional coordinates with respect to the target protein, and modifies, increases or decreases the fingerprints of compound of the candidate compounds according to the results of determination. Here, the optimizing unit 102b may randomly extract several fingerprints of compound from the fingerprint set of binding compound 106b, and thereby select a fingerprint set of binding compound to be fixing coordinates, which will serve as the basic. Here, the optimizing unit 102b includes, as shown in Fig. 1, an interaction score calculating unit 102f and a structure transforming unit 102g.

The interaction score calculating unit 102f is an interaction score calculating unit that calculates the interaction score, based on a function that takes into consideration of not only the root-mean-square deviation for the unit of fingerprint of compound but also the collision state of the candidate compound with the target protein, the existential rate of the candidate compound in the region of interaction of the target protein, and the fraction of direct interaction of the candidate compound with the target protein in the optimization process by the optimizing unit 102b. A concrete example of calculation of the interaction scores by the interaction score calculating unit 102f will be described below in detail in explanation of processing.

The structure transforming unit 102g is a structure transforming unit that repeatedly changes the conformation of the candidate compound in the optimization process by the optimizing unit 102b, and repeatedly translates or rotates the candidate compound as a rigid body, for each of the conformations of the candidate compound based on a simulated annealing method. The structure transforming unit 102g may also change the structure of the candidate compound by storing the previous conformations in memory, such as in the case of a genetic algorithm, instead of changing the conformation by randomly modifying the rotatable dihedral angle of the candidate compound.

The screening result output unit 102c is a unit for outputting results, which determines the ranking of the candidate compounds for the interaction with the target protein, based on the interaction scores that have been optimized by the optimizing unit 102b, and outputting the results of in silico screening.

The homology searching unit 102d is a homology searching unit that searches for the family protein and the binding compound from the protein database system, based on the homology of the target protein with the amino acid sequence. That is, the homology searching unit 102d performs a homology search, in order to acquire a binding compound, by making reference to a protein database such as an external system 200 using the amino acid sequence of the target protein as a query sequence, and thereby acquires a binding compound, for which the conformation bound to a protein having homology to the target protein is already known.

As shown in Fig. 1, the apparatus for in silico screening 100 may be constituted to be communicably connected to an external system 200 that provides an external database related to the amino acid sequence information or the protein 3-dimensional structure information, or an external program performing the alignment of sequences or 3-dimensional structures, via a network 300. The network 300 has function of connecting the apparatus for in silico screening 100 with the external system 200, and may include internet, or the like.

That is, in Fig. 1, the external system 200 is mutually connected to the apparatus for in silico screening 100 via the network 300, and has a function of providing an external database (PDB, PSI-Blast, or the like) such as a protein database relating to the amino acid sequence information or the protein 3-dimensional structure information, or an external program performing the alignment of sequences or 3-dimensional structures. Here, the protein database may store not only compounds that the 3-dimensional structures of protein-compound complexes are experimentally confirmed by X-ray structural analysis, NMR structural analysis or the like, but also compounds that are only known to bind to proteins. In this case, the fingerprint of compound producing unit 102a predicts the structure of a binding compound having a stable conformation with respect to the target protein through a known docking algorithm (DOCK, AutoDock, GOLD or the like) or any program for generating coordinates (Corina or the like), and thereby uses the structure in the production of the fingerprint set of binding compound 106b.

[Processing of the Apparatus For in silico Screening 100]

The following describes in detail one example of the processing executed by the apparatus for in silico screening 100 composed as above with reference to Fig. 2. Fig. 2 is a flow chart showing exemplifying processing performed by the apparatus for in silico screening 100.

As shown in Fig. 2, first, the homology searching unit 102d performs a homology search of a family protein whose 3-dimensional structure bound to a specific compound (binding compound) is already known, from a protein database of the external system 200 or the like, based on the amino acid sequence of the target protein input via the input device 112 (Step SA-1).

The fingerprint of compound producing unit 102a superimposes structure of the target protein and the structure of the family protein accompanied by the binding compound (Step SA-2). Here, the fingerprint of compound producing unit 102a may perform the superimposition of conformations between proteins without taking the kind of atom into consideration, or if the homology between the target protein and the family protein is high at a predetermined value or above, the unit may also perform the superimposition of conformations while taking the kind of atoms into consideration.

The fingerprint of compound producing unit 102a then converts the 3-dimensional coordinates of the binding compound from the coordinate system of the family protein into the coordinate system of the target protein (Step SA-3).

The fingerprint of compound producing unit 102a produces the fingerprint set of binding compound 106b by extracting the fingerprint of compound from the binding compound, along with the 3-dimensional coordinates of the binding compound that have been converted into the coordinate system of the target protein, and storing the fingerprint of compound in the storage unit 106 (Step SA-4). Here, the fingerprint of new compound adding unit 102e may add an arbitrary fingerprint of compound ("modified FP" or the like) as necessary, for the purpose of searching for a candidate compound with respect to the target protein. The fingerprint of compound producing unit 102a may also narrow structures by performing the search of those resembling a medicinal chemical compound, by determining an intersection of the fingerprint set of compound stored in the fingerprint set of binding compound 106b and the fingerprint set of compound stored in the medicinal chemical compound DB 106c.

The optimizing unit 102b selects, from the fingerprint set of binding compound 106b, a fingerprint of compound of fixing coordinates that serves as the basis of the calculation of the interaction score with respect to the candidate compounds stored in the candidate compound DB 106a (Step SA-5).

The optimizing unit 102b then calculates, for the candidate compounds, the root-mean-square deviations of each unit of fingerprint of compound using the 3-dimensional coordinates to be fixing coordinates of the selected fingerprints of compound as the basic, and performs the least square fitting, and computes the 3-dimensional structures of the candidate compounds with respect to the target protein, so that the interaction scores based on the calculated root-mean-square deviations are optimized (Step SA-6). That is, the optimizing unit 102b calculates, through the processing of the interaction score calculating unit 102f, the interaction scores based on the root-mean-square deviations of the 3-dimensional coordinates between fingerprints of compound, using, as the basic, the fingerprint of compound to be fixing coordinates of the target protein that has been arbitrarily selected from the fingerprint set of binding compound 106b. Then, the optimizing unit 102b performs a simulated annealing method based on the Metropolis method using the interaction scores as an index, so that the conformations of the candidate compounds converted by the processing of the structure transforming unit 102g and the structures with respect to the target protein will be optimized.

The screening result output unit 102c determines the order of interaction of the candidate compounds in the candidate compound DB 106a with respect to the target protein, based on the interaction scores that have been optimized by the optimizing unit 102b, and outputs the results of in silico screening to the output device 114 (Step SA-7). For example, the screening result output unit 102c sorts the group of candidate compounds in a descending order with respect to the interaction scores at the maximum point obtained for each of the candidate compounds by the optimizing unit 102b, and outputs the results.

This is the end of the processing of the apparatus for in silico screening 100.

[Calculation of the Interaction Score]

The following describes one example of the procedure for calculating the interaction score by the interaction score calculating unit 102f. The interaction score calculating unit 102f calculates the interaction score, based on a function that takes into consideration of not only the root-mean-square deviation for each unit of fingerprint of compound but also the collision state of the candidate compound with the target protein, the existential rate of the candidate compound in the region of interaction of the target protein, and the fraction of direct interaction of the candidate compound with the target protein. Specifically, the interaction score is calculated based on the following mathematical formula (1):

$$\begin{aligned} FPAScore &= F(aligned_fp, fp_rmsd, molecule) \\ &= BaseScore(aligned_fp, fp_rmsd) \\ &\times fp_volume(molecule) \\ &\times fp_contact_surface(molecule) \end{aligned} \quad (1)$$

wherein the FPAScore represents the interaction score, the F(aligned_fp,fp_rmsd, molecule) is a function using, as variables, the degree of alignment and the root-mean-square deviation of the unit of fingerprint of compound between the binding compound and the candidate compound, and the 3-dimensional structure of the candidate compound with respect to the target protein, the BaseScore(aligned_fp,fp_rmsd) is an index representing the degree of consistency and crowded degree of the unit of fingerprint of compound, the fp_volume(molecule) is an index representing the fraction occupied by the candidate compound in a space formed by the 3-dimensional coordinates of the fingerprint set of binding compound, and the collision state with the target protein, and the fp_contact_surface(molecule) is an index representing the contacting degree of the candidate compound with the target protein, and the degree of attribution to the 3-dimensional coordinates of the fingerprint set of binding compound.

More specifically, according to the present embodiment, the respective terms in the mathematical formula (1) are calculated based on the following mathematical formulas.

< BaseScore(aligned_fp,fp_rmsd) Term>

This term is a function taking into consideration of the degree of consistency and crowded degree of units of fingerprint of compound:

$$BaseScore(aligned_fp, fp_rmsd) = \frac{RawScore(aligned_fp)}{1 + \ln(fp_rmsd^{k1} + 1)} \quad (2)$$

wherein the RawScore(aligned_fp) is an index based on the number of atoms in the fingerprints of compound that are aligned between the binding compound and the candidate compound, and the fp_rmsd is the root-mean-square deviation.

The RawScore(aligned_fp) is specifically calculated based on the following mathematical formula (3):

$$RawScore(aligned_fp) = \sum_{i=1}^{n} assigned_score(i) \quad (3)$$

wherein the assigned_score(i) is the score given in advance to the fingerprint of compound aligned on the *i*th order, based on the following formula.

The assigned_score(i) is calculated in more detail based on the following mathematical formula (4):

$$assigned_score(i) = \begin{cases} \sum_{j=1}^{total_atom(i)} Case1_S + \ln(n_neighbor_atom(i)+1) \\ \sum_{j=1}^{total_atom(i)} Case2_S + \ln(n_neighbor_atom(i)+1) \\ \sum_{j=1}^{total_atom(i)} Case3_S + \ln(n_neighbor_atom(i)+1) \end{cases} \quad (4)$$

wherein the total_atom(i) is the number of atoms constituting the fingerprint of compound aligned on the *i*th order, and for example, in the case of a fingerprint of compound formed from four atoms, the number is 4; Casel_S, Case2_S and Case3_S are the scalar values given when the conditions described below are satisfied; and the n-neighbor_atom(i), which will be described later, is the number of atoms belonging to the same fingerprint of compound that is adjacent to the *i*th atom set.

For example, in regard to the term Case1_S, the depth-first search (see "Complete Course of C Algorithm: From Basics to Graphics, ISBN4-7649-0239-7, Kindai Kagaku Sha Co., Ltd.") is performed up to four atoms for a binding compound present in the fingerprint set of binding compound (for example, fingerprint of compound such as C.ar-N.arC.ar-C.ar). In the present embodiment, since the search is ended with up to four atoms, the number of ring structure is not taken into account. That is, a benzene ring and a naphthalene ring are not distinguished. If the search is successful, a score (Casel_S) is given to each of the atoms constituting the fingerprint of compound. Herein, the scalar value per one atom is defined as 5.0. That is, a fingerprint of compound composed of four atoms is given 20.0, while a fingerprint of compound composed of three atoms is given 15.0.

The term Case2_S is a constant score given to each atom when a new fingerprint of compound is produced using the fingerprint of compound obtained in Case1, by selecting two arbitrary fingerprints of compound that superimpose at a certain distance, and linking the atoms through imaginary bonds to produce a new fingerprint of compound. A default of 2.5 may be used.

The term Case3_S is an arbitrary scalar value that can be given when there is a possibility of the presence of an atom based on biochemical information or energy calculation. Here, the Case3_S was not used in the validation calculation using a training set.

Here, the fingerprints of compound obtained from the process for producing the Case1_S and Case2_S must belong to the fingerprint set of compound obtained from a known pharmaceutical database that is capable of discriminating the information on bonding rules and the atom type. Furthermore, in the process for producing the Case1_S, Case2_S and Case3_S, between the coordinates that belong to a same fingerprint of compound, the natural logarithm of the number of atoms for which the distance between an atomic coordinate set and another atom is within the value of dist (default is 1.0Å), is added to the score for the coordinates of fp. In regard to the binding compounds, when most of the compounds are peptides that amino acid residues links up, the correspondence relationship of a fingerprint of compound having many peptide groups becomes complicated. Therefore, the correspondence relationship may be underestimated during the process of calculating the interaction scores, and the part corresponding to the mathematical formula (3) of the fingerprint of compound for the peptide moiety in the mathematical formula for RawScore given above, may be replaced with the underestimated number such as zero .

The denominator of right side in the above mathematical formula (2) is calculated based on the following mathematical formula (5):

```
1.0 + ln(fp_rmsd**k1 + 1.0 )   (5)
```

wherein In is natural log; k1 uses 4.0 as the optimized result; fp_rmsd is the rmsd for least square superimposition; k1 is the scaling factor determining how strict the accuracy of the superimposition of FP should be, and is a constant which makes such that a large value of k1 results in a large rmsd (bad), and therefore decreases the RawScore (score) of the formula (3).

<fp_volume(molecule) Term>

This term is a function that evaluates the fraction occupied by a candidate compound in a space formed by the 3-dimensional coordinates of the fingerprint set of binding compound, that is, how much space formed from the fingerprint of compound obtained from the fingerprint set of binding compound is filled, and the collision with the target protein:

$$fp_volume(molecule) = \ln\frac{1.0 + nafp^{k2}}{1.0 + nap^{k3}} \qquad (6)$$

wherein the nafp (Number of Ligand Atom covering Fingerprint) is the number of lattice points occupied by the 3-dimensional coordinates of the candidate compound in a region of proper grid based on the 3-dimensional coordinates of the fingerprint set of binding compound, the nap (Number of Ligand Atom covering Protein) is the number of lattice points to which the 3-dimensional coordinates of the candidate compound fall into the region of proper grid of the atoms in the 3-dimensional structure of the target protein, and the k2 and k3 are each coefficient, and an arbitrary constant that can be modified by the biochemical information of the target protein, the degree of induced fit, or the like. In the present embodiment, 1.0 is used as the default.

<fp_contact_surface(molecule) Term>

This term is a function taking into consideration of the contacting degree of the candidate compound with the target protein, and the degree of attribution of the fingerprint set of binding compound to the there-dimensional coordinates:

$$fp_contact_surface(molecule) = \frac{\sum_{i=1}^{n} density_of_atom(atom(i))}{total_density_of_atom(molecule)} \qquad (7)$$

wherein n is the number of atoms of the candidate compound, atom(i) is the 3-dimensional coordinates of the *i*th atom in the candidate compound, the density_of_atom(atom(i)) is a function that reciprocates the sum of the number of atoms in the target protein contacting with the atoms of the fingerprint of compound at a predetermined distance and the number of atoms in the binding compound falling into the same lattice points of the fingerprint of compound when the 3-dimensional coordinates of the atom belong to the fingerprint of compound of the fingerprint set of binding compound, and the total_density__of_atom(molecule) is the number obtained by reordering the distribution of the density_of_atom in a descending order, and summing the numeric values in order as many times as the number of atoms in the candidate compound.

The density_of_atom(atom(i)) is expressed in more detail based on the following mathematical formula (8):

$$density_of_atom(atom(i)) = \begin{cases} 0 \\ \ln(nfpcontact + natom + hi) \end{cases} \qquad (8)$$

In this formula, if the coordinates of an atom that constitutes the candidate compound do not fall into the fingerprint of compound derived from the fingerprint set of binding compound, the crowded degree becomes 0, while if the coordinates do fall into the fingerprint of compound, the score is calculated according to the formula described above.

That is, nfpcontact is the number of atoms of the candidate compound that are contacting with the atoms belonging to the fingerprint of compound at a certain distance (default is 3.8). natom is the number of atoms constituting a compound derived from the binding compound set, which atoms fall into the same lattice point. In the case of identical binding compounds having different ID code in the PDB, the value may be appropriately changed, but in the present embodiment, the number may be counted, with overlapping being allowed. hi is used in the case of having particularly important biochemical information, and 0 is used as default. That is, the value occurs depending on the modified FPs, which is introduced according to the 3D-ID method of the "Circle" or the like, when stable contact with the target protein is suggested.

The formula of total_density_of_atom(molecule) is described below:

$$total_density_of_atom(molecule) = \sum_{i=1}^{total} sort_density_of_atom(i) \quad (9)$$

wherein total is the number of atoms (atoms in a molecule) of the compound; sort_density_of_atom is the result of arranging the distribution of density_of_atom in order, starting from larger values. That is, if the molecule is large, a larger value is added, and therefore, the total_density_of_atom is increased.

This is the end of explanation of one example of the method for calculating the interaction score by the interaction score calculating unit 102f.

[Interaction Score Maximization by Simulated Annealing]

An example of the processing of optimizing the conformation and configuration of candidate compounds according to simulated annealing by the optimizing unit 102b, based on the interaction scores calculated by the method for calculating interaction scores as described above, will be explained in the following.

Initially, the structure transforming unit 102g changes the conformation by randomly varying the rotatable dihedral angle of the candidate compound. In the present embodiment, the change of conformation is performed 1000 times. If this number is larger, there is a possibility of obtaining better results. However, since there is a need to perform docking calculation for the large number of low molecular compounds included in the virtual candidate compound DB 106a, it is necessary to limit the finite number of runs. It is also speculated that even though the number may depend on the degree of freedom of rotation of the candidate compound, this number of runs will be sufficient in the preliminary calculation. The initial conformation may be the binding conformation with respect to a family protein, which conformation is registered on the candidate compound DB 106a. The optimizing unit 102b uses the coordinates of the candidate compound in the following processing, for each of these changed conformations.

The optimizing unit 102b randomly selects ten fingerprints of compound from the bands of fingerprint of compound (fp bands) of the fingerprint set of binding compound 106b. When there are fewer than ten fingerprints of compound, half the maximum number of the bands of fingerprint of compound is used. More specifically, the atomic coordinates of the fingerprints of compound of the candidate compound and the fingerprint set of binding compound 106b are randomly selected from the selected bands of fingerprint of compound. This state is referred to as fingerprint alignment. In the correspondence relationship thereof, least square fitting is performed, and using the root-mean-square deviation (rmsd) of the superimposition in that case and the atomic coordinates of the candidate compound after the superimposition, the interaction score is calculated by the formula described above.

After the second round of repetition, the structure transforming unit 102g stores the state of the previous round in the storage unit 106, and performs translation and rotation while maintaining the conformation of the candidate compound, that is, while dealing with the candidate compound as a rigid body. Thereby, the structure transforming unit 102g performs an increase or decrease of one fingerprint of compound, and modification and addition of the correspondence relationship of the atomic coordinate set. In the present embodiment, this step is carried out 10,000 times.

In this process, the optimizing unit 102b performs the Metropolis decision. That is, the optimizing unit 102b accepts the configuration of the relevant candidate compound if the interaction score of this round is larger than the interaction score of the previous round. On the contrary, if the interaction score is smaller, the adopting probability, Paccept, is calculated based on the following mathematical formula:

$$\Delta Score = Score(this\ round) - Score(previous\ round)$$

$$Paccept = \exp \frac{\Delta Score}{T}$$

That is, since the scope of the adopting probability, Paccept, is such that 0<Paccept<=1, the optimizing unit 102b simultaneously generates uniform random numbers in the range of 0<=r<=1 in this case. If r<Paccept, even if the interaction score is smaller than that of the previous round, the interaction score is accepted. In the simulated annealing process, T (temperature) starts from 30 K and is decreased to 0.07 K.

As such, the optimizing unit 102b calculates the maximum value of the interaction score of one conformation, makes a comparison for 1000 conformations that have been initially generated, and thereby predicts the structure with the maximum interaction score as the optimal target protein-candidate compound complex (Protein-Ligand complex) structure. At this time, in the process of ranking the 1000 conformations, the previous conformation may be stored, to thereby keep changing the ligand structure according to a certain algorithm, by using a genetic algorithm or the like instead of randomly generating the conformations, and the calculation time or the search of the maximum value may also be subjected to devising. In the process of calculation for 1000 runs, it is possible to shorten the calculation time, or to obtain the minimum score having a possibility of the ligand conformations approaching more closely to the true conformation, in order to determine the order of the ligand conformations, by using a genetic algorithm or the like, such as that employed in the GOLD program.

The explanation of the maximization of the interaction score by simulated annealing is as discussed above.

[Tanimoto Index]

Upon producing a fingerprint set of compound, as a scale for measuring the similarity between compounds, for example, a low molecular compound set having a Tanimoto coefficient (Tc) of 0.08 or more may be used. In the case of determining the fingerprint (fp) of compound from a chemical descriptor, which is the fingerprint of compound from each compound, such as the Sybyl atom type, the Tanimoto coefficient (Tc) is calculated as follows:

$$Tc = \frac{a}{a+b+c}$$

wherein a is the number of fingerprints of compound that are present in the FP bands of both the binding compound and the candidate compound; and b and c are each the number of FP that are present in only one side of the FP bands.

To explain the same matter using the concept of assembly, if the assembly of fingerprints of compound possessed by the respective FP bands is designated as A and B, the following formula may be obtained:

$$Tc = \frac{number_of_fp(A \cap B)}{number_of_fp(A \cup B)}$$

wherein number_of_fp(assembly) is the number of fingerprints of compound that belong to a certain assembly.

Thus, the explanation of the Tanimoto index is as described above.

[First Example]

Hereinafter, a first example of the present embodiment to which the present invention is applied, will be described below in detail while referring to Fig. 3 to Fig. 29. In the following example, the fingerprint set of binding compound 106b may be referred to as "CElib" (FP(fingerprint)set extracted from collected ligands in the binding site).

[Development of choosing biological information semiempirically on the Ligand Docking]

In recent years, owing to the enhancement in the speed of computing machines, the methods for predicting the 3-dimensional structures of proteins and the evaluation method of the 3-dimensional structures in the field of pharmaceutical development [reference literature (Terashi G, Takeda-Shitaka M, Kanou K, Iwadate M, Takaya D, Hosoi A, Ohta K, Umeyama H Proteins, 2007; 69(Suppl 8): 98-107)] have improved. For example, in Homology Modeling, which is one of the methods for predicting 3-dimensional structures of proteins, the accuracy of prediction is ascentive, as a result of an increase in the structures registered to the Protein Data Bank (PDB) [reference literature (Westbrook et al. Nucleic Acids Res. 2003 Jan 1; 31(1): 489-91)], an increase in the templates that are referred to, except for membrane proteins, and blind tests in the Critical Assessment of Techniques for Protein Structure Prediction (CASP) [reference literature (Takeda-Shitaka, M., Terashi, G., Takaya, D, Kanou, K., Iwadate, M., Umeyama, H. Protein structure prediction in CASP6 using CHIMERA and FAMS. Proteins 2005; 61: 122-127)]. Furthermore, in regard to this homology modeling, the scope of application of the 3-dimensional structure prediction method is extending to the prediction of activity changes under the effect of mutation [reference literature (Nakamachi Y et al. Computer modeling analysis of Antithrombin with Ala54Thr and Ala249Glu mutations (P04-08). ], drug design [reference literature (Takede-Shitaka, M., Takaya, D., Chiba, C., Tanaka, H., Umeyama, H. Curr. Med. Chem. 2004; 11: 551-558)], and the like.

Together with the increase in the protein 3-dimensional structures registered on the PDB, the results of X-ray structural analysis of protein-ligand complexes are also increasing, and in many cases, multiple X-ray structures that have been analyzed may exist in a single family protein [reference literature (Edgar R. Wood et al. CANCER RESEARCH 2004; 64: 6652-6659), (Jennifer et al. J. Bio. Chem. 2002; 277 (48): 46265-46272)]. Even in the CASP described above, tests for predicting the residues at the binding site of proteins are performed [reference literature (Lopez, G, Rojas, A, Tress, M, Valencia, A Proteins 2007; 69(S8): 165-174)]. Thus, the importance of improving the accuracy of prediction of protein-ligand complexes is ever raising.

Meanwhile, in recent years, experimental determination of causative proteins of diseases is becoming popular [reference literature (Nature, or the like)], and the necessity of the design of inhibitors inhibiting those proteins is more and more rising.

As a potent method for the design of inhibitors, there may be mentioned inhibitor design (SBDD) based on the 3-dimensional structures of target proteins, and in-silico screening using software for protein-ligand complex prediction (so-called docking software) is performed. Here, Fig. 3 is a situation diagram representing the docking method according to the present example, based on a conventional docking software program and bioinformatics making effective use of a number of X-ray structures or NMR structures of protein-ligand complexes.

As shown in Fig. 3, in regard to the existing docking software programs, the AutoDock [reference literature (Goodsell et al. J. Mol. Recognit 1996; 9: 1-5)], DOCK [reference literature (Ewing et al. J Comput Aided Mol Des. 2001; 15(5): 411-428)], GOLD [reference literature (Gareth et al. J. Mol. Biol. 1997; 267: 727-748)] and the like employ the Ab-initio Approach, which uses a potential function of classical physics called hydrogen bond, hydrophobic interaction, and electrostatic interaction. These existing software programs have been proven by various verifications to perform docking with high accuracy (for example, a fraction that can predict to the correct structure at an rmsd of 2.0 or less has been verified by a blind test concealing the correct structure) [reference literature (Onodera et al. J. Chem. Inf. Model. 2007; 47: 1609-1618), (Michael et al. J. Med. Chem. 2007; 50: 726-741)].

In order to perform the docking of compounds having many rotatable bonds with high accuracy, a method of disposing fragments of the compound at the ligand binding site in advance using a potential function has also been designed [reference literature (Budin et al. Biol Chem. 2001; 382 (9) : 1365-1372)].

There have been a number of reports on the attempts to perform re-evaluation for selecting many hit compounds, in order to select the hit compounds, performing the calculation of the distance between the protein and the ligand, the energy of classical physics, and the like from the structure of a known protein-ligand complex, to extract the information on the interaction, after docking an inhibitor candidate compound from a library of virtual compounds to a target protein using an existing docking software program to predict the structure of the protein-ligand complex [reference literature (Sukumaran et al. Eur. J. Med. Chem. 2007; 42: 966-976), (Zhan et al. J. Med. Chem. 2004; 47: 337-344)].

However, it is implied by the series of studies described above that the existing docking software programs can predict protein-ligand complexes with high accuracy, but this prediction does not coincide with (is not directly connected to) direct selection of many hit compounds from the library of virtual compounds.

In other words, currently, the development of a system that is capable of predicting the structure of protein-ligand complexes with high accuracy and also capable of detecting many hit compounds from a virtual library, is highly requested, and such a system is absolutely indispensable in drug discovery.

Under such circumstances, the inventors of the present application developed the system CHOOse information SemiEmpirically on the Ligand Docking (ChooseLD), which can predict the structure of protein-ligand complexes by efficiently picking out effective information from the biochemical information of those protein-ligand complexes, of which interactions are already known that are registered on the PDB, and performing docking, and which can detect many hit compounds, without using the potential functions of classical physics in the evaluation of the interaction of protein-ligand complexes. Furthermore, the method of the inventors of the present invention does not use the potential functions of classical physics in the evaluation of the interaction of protein-ligand complexes. Therefore, the method of the present invention is expected that physical approaches such as CHARMM [reference literature (Brooks, R. B, Bruccoleri, E. R., Olafson, D. B., States, J. D., Swamlnathan, S., Karplus, M. CHARMM. A program for macromolecular energy, minimization, and dynamics calculations. J. Comp. Chem. 1983; 4: 187-217)], AMBER [reference literature (Case, A. D., Cheatham III, E. T., Darden, T., Gohike, H., Luo, R., Merz Jr., M. K. Onufriev, A., Simmerling, C., Wang, B., Woods, J. R. The Amber Biomolecular Simulation Programs. J. Comput. Chem. 2005; 26: 1668-1688)], and quantum chemistry [reference literature (Fedorov, G. D., Kitaura, K. Extending the Power of Quantum Chemistry to Large Systems with the Fragment Molecular Orbital Method J. Phys. Chem. 2007; 111: 6904-6914)] may function effectively on the optimization of the structures of protein-ligand complexes, for which it cannot be said that the physical interaction energy has been optimized.

[Outline of the Present Example 1]

The following describes an outline of the present example with reference to Fig. 4. Fig. 4 is a block diagram showing a principle of docking between a protein and a ligand according to the present example (ChooseLD).

Here, in the present example, the LIBRARY LIGANDS corresponds to a set of binding compounds, and the CElib corresponds to the fingerprint set of binding compound 106b.

Here, in Fig. 4, each column represents a set of data, while an ellipse represents the input information, and a rectangle represents the output structure. A parallelogram represents the fingerprint (FP) of compound as a chemical descriptor. Since the all process is carried out with a computing machine (apparatus for in silico screening 100), the information that is input is a file as electronic information. That is, files of the 3-dimensional coordinates of target proteins that are described in a format such as that represented by the PDB format, and files of the 3-dimensional coordinates of ligands that are docked, may be supposed.

In Fig. 4, the arrow means a conversion operation mainly involving extraction of a set of data or modification of input information, and detailed conditions can be specified for the conversion operation. However, such a conversion operation have pre-defined values set up, so that if the input information is normal in terms of the file format, and if the input coordinates of the protein are physicochemically normal, the outputs can be obtained full-automatically. That is, if a file of the 3-dimensional coordinates of a target protein and a file of those of the candidate ligand docked to the protein were input, a file of 3-dimensional coordinates of a protein-ligand complex structure would be output. The 3-dimensional coordinates and amino acid sequences of proteins are used as the 3-dimensional coordinates of protein ternary structures for the homology search, the establishment of an FP library that corresponds to the fingerprint set of binding compound 106b, and the calculation of docking, and the target candidate ligand, which corresponds to the candidate compound, is used in the search of candidate protein-specific FP bands and the 3-dimensional conformations of ligands.

That is, as shown in Fig. 4, first, the apparatus for in silico screening 100 according to the present embodiment performs homology search for the target protein on the protein structure database such as the PDB, by the processing of the homology searching unit 102d, performs fitting by structural alignment with a homologous protein by the processing of the fingerprint of compound producing unit 102a, extracts a fingerprint of compound along with the 3-dimensional coordinates converted into the coordinate system of the target protein, and thereby produces a group of ligands oriented to target proteins (C), which corresponds to the fingerprint set of binding compound 106b.

The apparatus for in silico screening 100 refers the group of ligands oriented to target proteins (C) to a druggable FP database (D), which corresponds to the medicinal chemical compound DB 106c, and obtains a target protein-specific FP band (L) as union (C)^ (D). Here, the group of ligands oriented to target proteins (C) may be added with virtual FPs such as modified FPs, through the processing of the fingerprint of new compound adding unit 102e.

Subsequently, the apparatus for in silico screening 100 extracts fingerprints of compound from the candidate ligands of a library of virtual ligand or a benchmark set, which is ligands docked to the target protein, and thereby produces an FP band (R) of the candidate ligands, which corresponds to the candidate compound DB 106a.

The apparatus for in silico screening 100 changes the conformations of the candidate ligands through the processing of the structure transforming unit 102g, and performs FP alignment between the FP bands (R) of the ligands oriented to target proteins (C) and the candidate ligands.

When the apparatus for in silico screening 100 performs docking of the candidate ligands to the binding site of the target protein using an interaction scoring function through the processing of the optimizing unit 102b, the apparatus for in silico screening 100 performs the prediction of 3-dimensional structures of the target protein-candidate ligand complexes, while optimizing the interaction scores using the simulated annealing (SA) method. This is the outline of the present example.

[LIBRARY LIGANDS]

LIBRARY LIGANDS corresponds to sets of binding compounds. That is, the apparatus for in silico screening 100 performs alignment between the target protein and a homologous protein using CE [reference literature (Shindyalov et al. Protein Engineering 1998; 11(9): 739-747)], a 3-dimensional structure alignment generating program, when a protein among the proteins detected by the homology search using the PSI-Blast [reference literature (Altschul et al. Nucleic Acids Res. 1997; 27(17): 3389-3402)] is a protein-ligand complex, and the apparatus superimposes the homologous protein to the target protein by a least square fitting method. The LIBRARY LIGANDS is a group obtained, when the Z-score resulting from the least square fitting is 3.7 or more, by converting the coordinate system of the binding ligands into it of the target protein, and picking out only the binding ligands.

In the present example, if the Z-score is less than 3.7, the ligand is not used as a binding compound. This is because the basis of this numerical value is "3.7 to 4.0 - twilight zone where some similarities of biological significance can be seen" according to the CE, and thus, 3.7 or more is accepted. According to the present example, the lowest homology of the homology search is defined as a homology of 0.1% or more. That is, most of the similar proteins detected by the homology search are superimposed using the CE.

[Definition of an FP and Construction of an FP Band]

The following describes in detail a method to make an FP band with reference to Fig. 5. Here, before defining the fingerprint of compound (fp) used in the present example, the interpretation of fingerprints of compound, will be explained. The fingerprint of compound (hereinafter, referred to as "FP") is one method of formula with regard to computing machines, used for the calculation of similarity between vectors representing the features of compounds, or between compounds, in the field of chemintormatics [reference literature (Swamidass, S. J. Baldi, P. Mathematical Correction for Fingerprint Similarity Measures to Improve Chemical Retrieval. J. Chem. Inf. Model. 2007; 47: 952-964)].

The present example is not aimed at a precise interpretation of FPs, but in order to avoid confusion, the following terms will be unified. When a molecule is expressed as a vector that has, as an element, a combination taking into consideration of the atom form (or atom type), the order of atomic bonds and the like, the element of the vector is called "FP," while the vector is called an "FP vector." According to the present example, information more than or equal to simple description of the character string of atom form may be merely added to the element of vector, but the additional information may also be interpreted as one feature expressing the molecule. If the information means to be an element of the vector, the information, is also referred to as "FP," while a vector having the FP as an element is distinguished from the typical "FP vector," and is referred to as an "FP band." This implies that in addition, the "FP band" has a nature such as that the respective elements in the "FP vector," are atom type. Here, Fig. 5 is a diagram depicting an example of the method of producing an FP (fingerprint).

In the ChooseLD method, which is the present example, the purpose of the method is to predict an unknown ligand structure which docks so as to satisfy the minimization of free energy, using a protein-ligand complex structure of which interaction is already known, and in order to achieve this purpose, the FP (fingerprint) has been defined, which is a component maintaining the partial free energy of binding from a ligand known to have interaction. The substance name of the chemical substance presented in Fig. 5 as an example is AZD2171 [reference literature (Cancer Res 2005; 65(10) May 15, 2005)]. As shown in Fig. 5, the FP is produced by tracing atoms using the given information of bonding rules. The number of atoms to be traced is 2, 3 or 4 (there is a reason for these numbers, and the reason will be described later). The respective surrounding lines mean FPs that are calculated. The FP represented by a implies the case of tracing two atoms, while the FP represented by b is an example of tracing three atoms. The FPs represented by c and d are respectively the cases of four atoms, and although they pass over the same atoms, this case is also allowable. The FP represented by e is in different coordinates but traces the same atom species, and thus the multiplicity of FP in the interaction scoring function that will be described later, is added.

That is, the parts surrounded by the line along the bonding of the compound in Fig. 5 mean the description of atom type of the FP that is used in the ChooseLD method as well as a comparison of similarity of compounds. The depth-first search method [reference literature (Chiba et al. C algorithm ZENKA 1995 ISBN4-7649-0239-7)] is used while taking an arbitrary atom on the compound as a base point, and the lines pass over atoms according to the information on interatomic bonding of the given ligand, but the number of bonds that are passed over is defined to be 1, 2 or 3. That is, a benzene ring and a naphthalene ring are supposed to have the same description of atom type established, and thus, the difference in the ring structure is undistinguishable. Individual atoms are represented using Sybyl Atom Type (Tripos Inc., 1699 South Hanley Road, St Louis, MO 63144-2913, USA (http://www.tripos.com)), and the atomic weight, the atomic radius and the number of bindable bonds are defined, which are obtained by referring to AMBER99 [reference literature (J. Comput. Chem. 2005; 26: 1668-1688)]. At this point, only the atom type of the FP is taken into consideration, and the coordinates of the passed atoms are not taken into consideration. Here, Fig. 6 is a chart presenting the list of character strings of the atoms used in the present example.

[Calculation of Similarity between Compounds Based on the Tanimoto Coefficient]

The method for calculating similarity between compounds based on the Tanimoto coefficient will be explained in the following. Here, Fig. 7 is a schematic diagram depicting the method for calculating similarity between compounds based on the Tanimoto coefficient.

According to the present example, the Tanimoto coefficient (hereinafter, referred to as Tc) was introduced so as to calculate similarity between compounds [reference literature (J. Chem. Inf. Comput. Sci. 2000; 40: 163-166)]. In general, the Tc is a result of numerical conversion of the degree of similarity of vectors consisted of two bits, that is, 0 or 1. As shown in Fig. 7, according to the present example, an FP vector was produced for one low molecular compound as a subject of processing, using the method for FP establishment introduced as described above. If the defined FP existed on the vector, the bit was given a value of 1, whereas if the defined FP did not exist, the bit was given a value of 0. The similarity between compounds was evaluated based on two vectors thus produced, which had the same length, and implied FPs having the same corresponding components.

The Tc was calculated by the following mathematical formula. Here, when the corresponding bits of both vectors are all on, 1 is added to a. If the bit of only one of the both vectors is on, 1 is added to b or c. That is, when the bits of both vectors are off, nothing is added to d, and d is taken out of consideration in the Tc calculation. For example, between the two bit columns shown in Fig. 7, the following is established: a=9, b+c=7, and Tc=9/(9+7)=0.5625.

$$Tc = \frac{a}{a+b+c}$$

According to the present example, the FP bands were defined such that when any two FP bands derived from low molecular compounds, which are obtained from a set of low molecular compounds belonging to the LIBRARY LIGANDS of binding compounds and form a set, are compared, the Tanimoto coefficient (Tc) must be 0.08 or more. In other words, the term a in the above mathematical formula is the number of FPs existing in each FP bands. The terms b and c are the numbers of FPs existing in the other FP bands.

To explain the same matter using the concept of assembly, if the assembly of FP possessed by the respective FP bands are designated as A and B, the following formula may be obtained:

$$Tc = \frac{number_of_fp(A \cap B)}{number_of_fp(A \cup B)}$$

wherein number_of_fp(assembly) is the number of FP that belong to the set "assembly."

[Construction of an FP Library]

An FP library corresponds to a set of binding compounds, and is the source of acquisition of the description of atom type of FPs that are used in the ChooseLD method of the present example, as well as a ligand group serving as the origin of the atomic coordinates registered on the established FPs. Conventionally, the FP library is collected from family proteins that have been detected by a homology search or the like using the primary structure, that is, the amino acid sequence, of a target protein as a query; however, the object is not limited to family proteins, and even ligands, or proteins, peptides and the like, which are considered to bind to a target site, such as an active site of the target protein, may also be added if necessary.

According to the ChooseLD method of the present example, the FP library was established mainly from family proteins. When proteins that have been detected by a homology search using the PSI-Blast [reference literature (Nucleic Acids Res. 1997; 27: 3398-3402)], of which 3-dimensional coordinate structures are already known, and which is protein-ligand complexes, 3-dimensional structure alignment between the target protein and a family protein is performed using CE [reference literature (Protein Engineering 1998; 11: 739-747)]. The CE is a program equipped with an algorithm which performs alignment of two proteins using similar parts in terms of the 3-dimensional structure, without depending on the similarity in the amino acid sequence, and other programs for 3-dimensional structure alignment include Dali [reference literature (J. Mol. Biol. 1993; 233: 123-138), TOPOFIT[reference literature (Protein Science 2004; 13: 1865-1874)], and the like. To mention the main differences of these programs, it is possible with the CE to obtain results quickly, as a result of ameliorations such as performing superimposition of amino acid sequences sequentially from the N-terminal, but when the subject proteins have domain swapping or the like, it is difficult to perform alignment with high accuracy. In that case, high accuracy may be obtained by using Dali or the like, which performs alignment without depending on the order of the amino acid sequence.

The ChooseLD method of the present example used CE, which takes shorter calculation time, since the family proteins detected by the PSI-Blast were mainly superimposed. The family proteins were superimposed to the target protein based on least square fitting, using the alignments output by the CE. When the Z-score of the alignment from CE, was 3.7 or more, the binding ligands were converted based on the coordinate system of the target protein, and thereby only the binding ligands were picked out. That is, according to the present example, only the proteins that are structurally similar to the target protein, will be used as family proteins.

[Construction of the FP Band]

The FP band is a vector of FP correlated with one or multiple atomic coordinates as additional information, and is obtained from a set of binding ligands that belong to the FP library. The binding ligands that belong to the obtained set (FP library) include the coordinates in the coordinate system of a target protein; the atom type expressed as Sibyl atom type; and the information of bonding rules such as a single bond, a double bond and the bonding in aromatic rings. Here, Fig. 8 is a schematic diagram presenting the FP in the case of docking a ligand to the binding site of a target protein as an example. In Fig. 8, the semi-transparent part composed of several geometrical shapes (rectangle, diamond shape or ellipse) represents various FPs.
The "intra-molecule FP" (rectangle in Fig. 8) is an FP established using only the intramolecular information of a ligand, and is an FP produced using the atom type information and the bonding information obtained only from the inner part of one ligand that belongs to the FP library. A single FP constitutes four atoms at the maximum, which pass over bound atoms once, twice or three times, based on the method for establishing the description of the atom type of FP previously mentioned while taking one of the atoms inside a ligand molecule as a base point, and which do not branch as shown in Fig. 8. The smallest FP in the present example consists of two atoms. During the implementation of FP establishment for once, an atom that has once been traced is not passed over twice during the implementation, and when there are no more bonds to be passed over, the description of the atom type of FP and the atomic coordinates at that time point are registered on the FP band. The instance in which the FP has already been registered on the FP band is not excluded, but multiple atomic coordinates are registered for a single FP. Here, Fig. 9 is a diagram depicting an example of the process of obtaining atomic coordinates from the traced path and registering the atomic coordinates on an FP band.
In Fig. 9, the matrix below means the atomic coordinates, and the number of the rows represents the number of atoms constituting an FP. For example, a matrix consisting of four rows and three columns represents that the FP includes four atomic coordinates.
The "modified FP" (diamond shape in Fig. 8) is an FP produced based on given information on bonding and an assumption that an imaginary bond exists between adjacent atoms. An FP is established that consists of four atoms at the maximum, which are bonded, and which are actually not bonded but are judged to be imaginarily bonded if atoms are present within 1Å, unless particularly defined otherwise, and which pass over the bond once, twice or three times and do not branch. According to the present example, the smallest FP is composed of two atoms. In the same manner as in the operation of establishing the "Intra-molecule FP," during the implementation of FP production for once, those atoms that have once been traced are not to be passed over two times, and if there are no more bonds to be passed over, the description of the atom type of FP and the atomic coordinates at that point are registered on the FP band. Thereby, an FP including the intermolecular bonding of a ligand in addition to the intramolecular bonding of a ligand is produced, and therefore an FP, which is not likely to actually exist, is obtained. That is, it is speculated that an FP of bonds that cannot exist physicochemically (FP such as N.am, N.am, N.am, N.am) is established.
Thus, according to the present example, from MDL Comprehensive Medicinal Chemistry (MDL CMC) Library, which is a database for 3-dimensional coordinates of physicochemically existing pharmaceuticals (corresponding to the medicinal chemical compound DB 106c), a drug-like FP vector is produced and compared with the FP vector part of the FP band obtained from the FP library, so that the description of the atom type of FP that is included in both parties, remains in the target protein-specific FP band. In the process of calculation using an arbitrary FP (fingerprint), when a pharmaceutical database or a compound database is used to take out compound information, a pharmaceutical database or a compound database specialized in drug absorption, drug metabolism, drug excretion or drug toxicity, which has been arranged in advance using an index for drug absorption, drug metabolism, drug excretion, drug toxicity or the like in this foundational database and using fundamental data units which use fingerprints (FP) or the like as the basic for arrangement, is produced, and the same series of operations are carried out.
Specifically, when a intersection between the FP vectors derived from the ligand library and the FP vectors derived from the pharmaceutical library is determined, only the FPs present in the medicinal chemical compound DB 106c are registered on the FP bands, and the FPs not present in the medicinal chemical compound DB 106c are neglected in the present example, thereby the fingerprint set of binding compound 106b being established. Fig. 10 is a diagram depicting an example of the method step of arranging to be decreasing fingerprint band in the present example.
As shown in Fig. 10, the FP bands (A) obtained from the MDL CMC Library and the FP bands (B) obtained from a group of ligands oriented to target proteins are compared, and FPs are removed from the FP band of (A) or (B), excluding the cases where an FP is present in both FP bands (represented by symbol X in Fig. 10). As a result, the library ligand-derived FPs essentially have 3-dimensional coordinates.
Thus, the explanation of the method for establishing FP bands according to the present example is completed. According to the present example, it is allowed that one atom belongs to multiple FPs in the overall process of establishing FP bands. If the FPs obtained from the FP bands has already been registered, the coordinates of FPs are added. If FPs are not present, new FPs are added to the FP bands, and coordinates are added. It is allowed that one atom belongs to multiple FPs. The same operation is carried out for the candidate ligands that serve as the target of docking (docked ligands), and FP bands derived from candidate ligands (fp bands of docked ligand) are produced.

[Alignment of FP Bands]

The FP band is correlated with the coordinates of an atomic set, and when two FP bands are compared, not only the atom type is used, but also the correlated coordinates are used. That is, the alignment of FP bands means that a comparison of the FP band obtained from a candidate ligand and an FP band obtained from the FP library of the binding ligands is carried out. The comparison is carried out through the following processes of (1) and (2).

(1) Comparison of complete correspondence of character strings for description of atom type constituting FP
In regard to the FP vector (bit column (1)) derived from the FP band obtained from a candidate ligand to be docked, and the FP vector (bit column (2)) derived from the FP band obtained from an FP library including binding compounds, the existence or nonexistence of FP is expressed in each bit, and a combination in which the bits in both vectors are on is selected (see Fig. 7).
(2) Process of defining correspondence relationship between coordinate vectors for atoms registered on selected FP

Fig. 11 is a schematic diagram presenting an example of the process of defining a correspondence relationship between coordinate vectors. One FP is composed of an atomic coordinate vector (1) derived from a candidate ligand molecule to be docked, and an atomic coordinate vector (2) derived from a binding ligand of the FP library, and a correspondence relationship between these atomic coordinates is defined.
Carrying out these two processes (1) and (2) constitutes the alignment of FP according to the present example. Furthermore, the phrase "FP alignment is different" means that at least one of the following is different:

1. the total number of FPs having on for both of the two bits,
2. the type of FP to be correlated, and
3. the correspondence relationship of coordinates within FPs.

That is, the phrase "FP alignment is changed" means that at least one among these is changed. The significance of "at least one" is due to that when the atom type of FP changes, the correspondence relationship between the coordinates of FPs before change is lost, and because the correspondence relationship is redefined for FPs after change, the correspondence relationship of the coordinates is also necessarily changed.

[Interaction Score (FPAScore)]

The interaction score FPAScore is explained below in detail in the present example. The FPAScore (fingerprint alignment score) is defined in the present example such that a higher FPAScore better satisfies the structure of family protein-binding ligand complex of which interaction is already known, based on the assumption of the ChooseLD method saying that the FP is a set of partial binding free energy. The FPAScore evaluates a target protein-candidate ligand complex structure by considering the accuracy of the superimposition of FPs, the number of FPs used in alignment, the crowded degree of FP, and the protein-ligand complex interaction at the same time. According to the present example, the optimal target protein-candidate ligand complex was predicted by searching for the optimal alignment of the FP bands obtained by the operation described above.
That is, according to the present example, the interaction score, FPAScore, is defined by the following mathematical formula. Here, aligned_fp means FPs that have been aligned; fp_rmsd means the rmsd calculated by least square fitting using the alignment; and molecule means the coordinates of the complex after the candidate ligand has docked to the target protein. Each of the terms will be explained below in detail.

```
        FPA Score = F(aligned_fp, fp_rmsd, molecule
    coordinate.)
                  = BaseScore(fp_rmsd, aligned_fp)
                    * fp_volume(molecule)
                    * fp_contact_surface(molecule)
```

<1. BaseScore(fp_rmsd, aligned_fp) Term>

This term is defined as a function taking into consideration of the degree of consistency and crowded degree of FP, that is, a function for evaluating the strength of using already known FP, and can be represented by the following mathematical formula:

$$\text{BaseScore}(\text{fp_rmsd},\ \text{aligned_fp}) = \frac{\text{raw_score}(\text{aligned_fp})}{1.0 + \ln(\text{fp_rmsd}^{**}\text{k1} + 1.0)}$$

wherein In is natural log (natural logarithm); and k1 is a scaling factor for determining how strict the accuracy of superimposition of FPs should be. If the rmsd for the superimposition of aligned FPs is large, the denominator is increased, resulting in a smaller BaseScore. This implies exclusion of the cases where, even if the degree of consistency of FP is large, the rmsd that represents the accuracy of overlapping of the FP atomic coordinates registered on the FP is large (bad). According to the present example, k1 was set at 4.0; fp_rmsd is the rmsd calculated by least square fitting using the alignment; and aligned_fp is the correspondence relationship of FPs in that case, that is, aligned FPs.

Here, in the mathematical formula described above, the term raw_score(aligned_fp) is represented by the following formula. Here, assigned_score(i) is the score given in advance to the FP aligned on the ith order. n is the total number of aligned FPs. The term aligned FPs means the atom type and atomic coordinate set for a target protein-specific FP band (see, "alignment of FP bands" and Fig. 11). That is, in regard to the alignment of FPs, although the FPs have the same atom type, if there is a difference in the atomic coordinates, this means different FPs.

$$\text{raw_score}(\text{aligned_fp}) = \sum_{i=0}^{n} \text{assinged_score}(i)$$

Here, in the mathematical formula described above, assigned_score(i) is the score given in advance to the FP aligned on the ith order, and is represented by the following mathematical formula. This score is given as follows, to the FP obtained from a ligand library such as CElib.

$$\text{assinged_score}(i) = \begin{cases} \sum_{j=0}^{\text{total_atom}} \text{Case1_S} + \ln(N+1) & \ldots\ \text{case1} \\ \sum_{j=0}^{\text{total_atom}} \text{Case2_S} + \ln(\text{Neighbor_atom} + 1) & \ldots\ \text{case2} \end{cases}$$

Here, total_atom(i) in the formula represents the number of atomic coordinates constituting the FP; and Case1_S, Case2_S and Case3_S (not described above) are scores that are given in advance to the atoms constituting the FP, and are respectively used in the following cases.

Case1_S is a score that is given to each atom when the "Intra-m-olecule FP" as described above has been constructed. If the value is not particularly designated, 5.0 is used. For example, when the search is successful, the score Case1_S (the default of 5.0 was used) is given to each atom that constitutes the FP, so that an FP constituted of four atoms is given 20.0 points, while an FP constituted of three atoms is given 15.0 points.

Next, Case2_S will be explained. This is a score that is given to each atom when the "Modified FP" as described above has been established. If the value is not particularly designated, 2.5 is used.

Finally, Case3_S is an arbitrary scalar value given when there is a possibility of the presence of atoms based on biochemical information or calculation of energy ("Circle" or the like). The Case3_S is not used in the present example, and is not used in the calculation for verifying the docking performance (binding mode prediction performance) using a benchmark set, and the in silico screening performance.

According to the present example, the natural logarithmic value of the crowded degree of atoms belonging to the FP library was added to the score, in addition to the score of the sum of Case1_S, Case2_S and Case3_S. This involves adding the natural logarithm of the number of atoms (n_neighbor_atom(i)) of an atomic coordinate set that belongs to another FP, which are within 1.0Å from the atoms of an atomic coordinate set that belongs to the FP, to the score of FP, and thus this term can be said to be a term which gives preference to a dense FP. That is, in regard to the easel and case2, the natural logarithm of the number of atoms (Neighbor_atom) of an atomic coordinate set, which are within a distance of dist (default 1.0Å) between the coordinates that belong to the same FP, is added to the score for the coordinates of FP.

<2. fp_volume(molecule) Term>

This term is a function for evaluating the complex structure, after a candidate ligand has been docked to a target protein using aligned FPs. That is, it is a function for evaluating the number of the molecular coordinates of a candidate ligand after docking which occupy a space formed from the FPs obtained from the bound ligands of an FP library (that is, how much space formed from the FPs derived from an FP library is filled), and the collision with the target protein. The term is represented by the following mathematical formula. Here, the term molecule represents the atomic coordinates of the candidate ligand after docking.

```
                                      1.0 + nafp ** k2
fp_volume(molecule) = ln ----------------------------
                                      1.0 + nap ** k3
```

Here, nafp (Number of Ligand Atom covering Fingerprint) is the number of the coordinates of a molecule occupying in a region of proper grid that is produced using the atoms of small molecules constituting the LIBRARY LIGAND, that is, the number of the coordinates of candidate ligands occupying in a region of proper grid produced using the atoms of binding ligands that constitute the FP library. nafp represents how much a molecule of a candidate ligand satisfies an FP (fingerprint) of fixing coordinates. nap (Number of Ligand Atom covering Protein) is the number of the coordinates of a molecule (molecule of a candidate ligand after docking) falling into a region of proper grid produced from the atomic coordinates of the target protein, and represents the collision state with the constituent atoms of the target protein.

k2 and k3 are each a coefficient, and if the value is not particularly designated (default), 1.0 is used, respectively. However, they can be respectively modified depending on the biochemical information of the target protein and the degree of induced fit. That is, k2 is a constant which makes a point of attaching importance to the region occupied a space of a group of binding ligands of a family protein to be identical or similar to the target protein, and if the coefficient is increased, a larger ligand may obtain a larger score. The k2 value is possible to be grouped, even based on the size of the binging region of the target protein. k3 is a tolerance factor for the collision of a candidate ligand in a region occupied by the target protein, and is a coefficient which makes a point of attaching importance to the collision between the atoms of a candidate ligand and the atoms of the target protein. If the value of k3 is increased, the collision between the target protein and the candidate ligand may not be allowed. In regard to k3, there is a possibility of grouping the flexibility at the active site of a protein, and the like. Fig. 12 is a diagram depicting a specific example of nafp and nap by using the ligand having the number of atoms of 31.

As shown in Fig. 12, when the number of atoms in a candidate ligand colliding with a target protein is 10, the atoms fall into 21 sites of the FP library-derived lattice points, and the k2 value and the k3 value are each 1.0, the term of fp_volume(molecule) obtains a value of ln(22/11)=0.693. In view of the nature of the function of this term, the ratio of change is highest when the nafp is 31 to 30, that is, the number of collisions is 0 to 1. When nearly half the ligand atoms are subject to collision, the value becomes negative, and therefore, it becomes difficult to accept the value. That is, the FPAScore is defined to be corresponding to the Lennard-Jones potential expressing in intermolecular attraction force-repulsion term, which is an empirical physical function. In addition, the results of an example of the optimization of the k2 value and the k3 value will be described later, in a section related to the performance of in silico screening using EGFR as the target protein.

<3. fp_contact_surface(molecule) Term>

The term of fp_contact_surface is a function taking into consideration of the contacting degree of the atomic coordinates with a target protein in the after-docking structure of a candidate ligand, and the degree of attribution of the coordinates to the FP library. The term is represented by the following mathematical formula. Here, the term molecule means the atomic coordinates of the candidate ligand after docking; atom(i) means the *i*th atomic coordinates after docking; and n means the number of atoms. That is, this formula is calculated with respect to the complex structure obtained after the docking of a candidate ligand to a target protein, as in the case of the mathematical formula for fp_volume described above, and is a function taking into consideration of the contacting degree of the atomic coordinates of the candidate ligand with the surface of the target protein, and the degree of attribution of the atomic coordinates of the candidate ligand to the FP atoms obtained from the FP library.

$$\text{fp_contact_surface(molecule)} = \frac{\sum_{i=0} \text{density_of_atom(atom(i))}}{\text{total_dense_of_atom(molecule)}}$$

In the above formula, density_of_atom is represented by the following mathematical formula. Here, nfpcontact is the number of atoms of the target protein that get in contact with the atomic coordinates of an FP that belongs to the FP library, at 3.8Å or less if there is no particular designation (at default); and natom is the number of atoms of an FP library-derived binding ligand compound, that fall into a same lattice point. In this case, a plurality of ligand molecules of the same atom type may be present, and even in the case of the same ligand molecule with different ID codes for the PDB, the present examples include all of such molecules. hi is a variable used in the case of having particularly important biochemical information, and if the value is not particularly designated (at default), 0 is used. It is envisaged that the variable be used when an FP (Modified FP, Creative FP, or the like) that does not depend on the family protein is input as a result of the 3D-1D score value of CIRCLE [reference literature (Terashi G, Takeda-Shitaka M, Kanou K, Iwadate M, Takaya D, Hosoi A, Ohta K, Umeyama H Proteins 2007; 69(S8): 98-107)] or the like. The following mathematical formula becomes 0 when atomic coordinates x of the ligand do not fall into the FP obtained from the FP library (not in contact at a distance of 3.8Å or less). Otherwise, the score is calculated according to the formula given above.

$$\text{density_of_atom(x)} = 0 \text{ or } \ln(\text{nhpcontact} + \text{natom} + \text{hi})$$

Fig. 13 is a diagram depicting an example of the location of a ligand derived from the FP library in the neighborhood of the binding site of the target protein. As shown in Fig. 13, since the neighboring FP surrounded by an ellipse (a circle of chain-dashed line) near the target protein is in contact with the target protein, nfpcontact is treated preferentially. In the neighborhood of the black circle, atoms of FP library-derived binding ligand are densely present, and thus natom is treated preferentially. That is, when the atomic coordinates of a docked candidate ligand is near to these divisions, the score is treated preferentially because of the formula described above.

In the mathematical formula for the fp_contact_surface, total_dense_of_atom(molecule) is represented by the following mathematical formula. Here, the term total is the number of atoms of the candidate ligand molecule. sort_density_of_atom is the result of arranging the distribution of the scalar values of density_of_atom in order of great numerical value, starting from larger values. That is, if the molecule of the candidate ligand is large, the total_dense_of_atom is increased.

$$\text{total_dense_of_atom(molecule)} = \sum_{i=0}^{\text{total}} \text{sort_density_of_atom(i)}$$

This is the end of the explanation of the interaction score FPAScore in the present example.

[Interaction Score Maximization and Conformation Change by Simulated Annealing]

Next, in order to maximize the FPAScore function defined as described above, the method for performing simulated annealing (hereinafter, referred to as "SA") according to the present example will be explained, while referring to Fig. 14 [reference literature (J. Mol. Graphics Mod. 2000; 18: 258-272, 305-306)]. Fig. 14 is a conceptual diagram depicting an example of the process of simulated annealing.

First of all, one cycle of steps 1. to 3. from the conformation change of the candidate ligand to the obtainment of a docking structure resulting in the maximum FPAScore for the structure, will be described.

<Step 1.>

First, the conformation is changed by randomly modifying the rotatable dihedral angle that is present in the candidate ligand to be docked (docked ligand). According to the present example, values of the van der Waals radius of the candidate ligand atoms obtained by referring to AMBER99 was used.

<Step 2.>

The candidate ligand with changed conformation is used as a rigid body, to dock to the ligand binding site (the binding site). The following operation of translation and rotation is carried out for a single conformation generated in the step 1.

First, ten atom types of FP are randomly selected from an FP band described above. If there are fewer than 10, half the maximum number of the size of the FP vector in the FP band was used. The atomic coordinate sets registered for the selected FPs are randomly selected. These are used as aligned FPs, and for the correspondence relationship, least square fitting is performed to calculate the rmsd between the atomic coordinates of the candidate ligand and the atomic coordinates derived from the FP library. The translation and rotation matrices thus obtained are operated with respect to the target ligand, to thereby obtain one target protein-candidate ligand complex structure. The FPAScore is then calculated using the aligned FPs, the rmsd, and the target protein-candidate ligand complex structure. Here, Fig. 15 is a diagram schematically depicting the FP alignment and the least square fitting for calculating the FPAScore.

As shown in Fig. 15, the FP alignment is performed between the coordinate matrices for each type of FPs of (D) and (E) as described above in the section of the alignment of FP bands, and <1> a combination in which the bits in both of the ligand library-derived FP vector (D) and the candidate ligand-derived FP vector (E) are on, is selected. Any FP that is not consistent in this process of selection is excluded from the alignment. <2> For one FP, the coordinates of the atomic coordinate vector (1) derived from the candidate ligand molecule, and the atomic coordinate vector (2) derived from the binding ligand of the FP library are correlated, and the interaction score is calculated based on least square fitting.

The change in state caused by simulated annealing is a process of modifying, increasing or decreasing the FP. That is, this change in state is carried out by repeating a process of selecting the coordinates belonging to the FP, from the FPs derived from the candidate ligands to be docked and the ligand library-derived FPs. Simulated annealing changes alignment by increasing by one or maintaining the atom type of FP, with respect to the aligned FPs, and performing modification or addition of the correspondence relationship of the atomic coordinate set registered with the FP, and reduction of FP, and thus maximizes the FPAScore. When one or more atomic coordinate sets are selected from one FP, or when the FPA score is decreased regardless of the presence of coordinates, Metropolis decision is carried out, and if the score is accepted, the state is maintained. That is, Metropolis decision is carried out in the process of SA, and if the score of this round is larger than the score of the previous round, the score is accepted. Otherwise, the adopting probability, Paccept, is calculated based on the following mathematical formula. In this case, if uniform random numbers in the range of 0<=r<=1 are generated simultaneously, and r<Paccept, even a low score is accepted. In the present example, T (temperature) was started from 30.0 K and decreased down to 0.07 K. Thus, the maximum value of the FPAScore is calculated for one conformation.

$$\Delta Score = Score(after) - Score(before)$$

$$Paccept = \exp(\Delta Score/T)$$

Using the FP bands thus obtained, the FPAScore is optimized according to the SA method. In the present example, SA was performed 10,000 times.

<Step 3.>

The maximum FPAScore obtained for one conformation in the step 2, is stored in the structure pool of the storage unit, together with the structure.
The processing of one cycle for the maximization of FPAScore for one conformation is as discussed above.

<Step 4.>

According to the present example, since it has been set up to perform the change of conformation 1000 times, if the process is performed fewer than 1000 times, it should be controlled such that the steps 1. to 3. as described above are carried out again. A larger number of generated conformation may lead to a possibility of obtaining better results, but since it is needed to perform docking calculation for a large number of low molecular compounds that are included in the virtual compound database, the calculation must be stopped after a limited number of cycles. Thus, although the number may depend on the degree of freedom of rotation of the compound, this number of runs was sufficient in the preliminary calculation of the present example.
When the maximum value of the interaction score, FPAScore, has been calculated for each of the 1000 conformations generated, the process of the repetition of cycle is terminated, and the maximum FPAScores of the 1000 conformations stored in the structure pool are compared. Thus, a predictive structure of the target protein-candidate ligand complex (Protein-Ligand complex), which is the docking structure having the largest score, is output as the optimal conformation for the candidate ligand.

[Results and Discussion (Materials), and method related]

The "Results and Discussion (Materials)" in regard to the present example will be described in the following. In the construction of the FP library described in the present example, development was achieved by combining shell script languages such as Perl (http://www.perl.com/), Ruby (http://www.ruby-lang.org/), and bash (http://www.gnu.org/software/bash), and so on. The algorithm described as the method of the present example, for searching for a protein-ligand complex structure that is likely to maximize the FPAScore by changing the conformation of candidate ligands to be docked, was written in C/C++. As for the compiler, Intel (registered trademark) C++ Compiler 10.0 was used. To mention the configuration of the computing machine used, up to 200 non-shared memory-type computing machine clusters having different configurations of computing machines, such as Red Hat Linux or Scientific Linux for the OS; Pentium 4, Core2Duo or Opteron for the CPU; and 512MB, 1024MB or 2048MB for the memory, were used. To mention about the calculation time for reference, when in silico screening of 20,000 compounds of the MDL Available Chemicals Directory (MDL ACD) Library (Symyx Technologies, Inc. Corporate Address: 3100 Central Expressway, Santa Clara, CA 95051) is carried out for the kinase domain of EGFR that will be described later, in regard to the time for implementation of calculation per CPU for the docking of one candidate ligand to one target protein, the median value was 10.2 minutes, and the average value was 18.6 minutes. The minimum calculation time was 4.8 minutes, and the maximum calculation time was 1077 minutes. Here, Fig. 16 is a diagram presenting the distribution of calculation time in the in silico screening of EGFR.
As shown in the distribution of calculation time in the in silico screening of EGFR of Fig. 16, it may take a very long time depending on the ligand to be docked. As one of the reasons therefore, the case of docking a ligand which causes difficulty in the search of conformations that are likely to avoid intramolecular collision, was conceived. It was understood that random selection of rotatable bonds was the cause, and it was necessary to perform rotation such that it would be difficult for intramolecular collision to occur. The calculation time for ChooseLD of the present example depends on the size of the target protein, the number of ligands included in the FP library, the molecular weight of the ligand, the molecular weights of candidate ligands, and the number of rotatable bonds of those. Thus, when the ligand binding site of the target protein was narrowed, and selection of the FP library was performed, it was possible to obtain a predictive structure more rapidly.
According to the present example, protein-ligand complex structures were acquired from the Protein Data Bank [reference literature (Nucleic Acids Res. 2003; 31: 489-491)], to test the docking performance of the ChooseLD.
A bench mark used in the present example is explained with reference to Figs. 17 and 18. Fig. 17 is a diagram depicting an example of the outline of benchmark. Fig. 18 is a diagram presenting the yearly distribution of the number of registrations on the PDB.
As shown in Fig. 17, the number of benchmark sets used was 218 species of proteins respectively having a ligand. The PDB structures of 85 species (on the left side of Fig. 17) were used to produce a score equation. The PDB structures of 133 species (on the right side of Fig. 17) were used to make a comparison with other docking methods (DOCK, AUTODOCK, GOLD and the like) (PDBIDs are presented below).

85 PDB structures; 1G9V IGKC IGM8 1GPK 1HNN 1HP0 1HQ2 1HVY 1HWI 1HWW 1IA1 1IG3 1J3J 1JD0 IJJE 1JLA 1K3U 1KE5 1KZK 1L2S 1L7F 1LPZ 1LRH 1M2Z 1MEH 1MMV 1MZC 1N1M 1N2J 1N2V 1N46 1NAV 10F1 1OF6 1OPK 1OQ5 1OWE 1OYT 1P2Y 1P62 1PMN 1Q1G 1Q41 1Q4G 1R1H 1R55 1R58 1R9O 1S19 1S3V 1SG0 1SJ0 1SQ5 1SQN 1T40 1T46 1T9B 1TOW 1TT1 1TZ8 1U1C 1U4D 1UML 1UNL 1UOU 1V0P 1V48 1V4S 1VCJ 1W1P 1W2G 1X8X 1XM6 1XOQ 1XOZ 1Y6B 1YGC 1YQY 1YV3 1YVF 1YWR 1Z95 2BM2 2BR1 2BSM

133 PDB structures; 1AAQ 1ABE 1ACJ 1ACK 1ACM 1ACO 1AEC 1AHA 1APT 1ASE 1ATL 1ARM 1BAF 1BBP 1BLH 1BMA 1BYB 1CBS 1CBX 1CDG 1CIL 1COM 1COY 1CPS 1CTR 1DBB 1DBJ 1DID 1DIE 1DR1 1DWD 1EAP 1EED 1EPB 1ETA 1ETR 1FEN 1FKG 1FKI 1FRP 1GHB 1GLP 1GLQ 1HDC 1HDY 1HEF 1HFC 1HRI 1HSL 1HYT 1ICN 1IDA 1IGJ 1IMB 1IVE 1LAH 1LCP 1LDM 1LIC 1LMO 1LNA 1LPM 1LST 1MCR 1MDR 1MMQ 1MRG 1MRK 1MUP 1NCO 1NIS 1PBD 1PHA 1PHD 1PHG 1POC 1RDS 1RNE 1ROB 1SLT 1SNC 1SRJ 1STP 1TDB 1TKA 1TMN 1TNG 1TNI 1TNL 1TPH 1TPP 1TRK 1TYL 1UKZ 1ULB 1WAP 1XID 1XIE 2ADA 2AK3 2CGR 2CHT 2CMD 2CTC 2DBL 2GBP 2LGS 2MCP 2MTH 2PHH 2PK4 2PLV 2R07 2SIM 2YHX 3AAH 3CLA 3CPA 3GCH 3HVT 3PTB 3TPI 4CTS 4DFR 4EST 4FAB 4PHV 5P2P 6ABP 6RNT 6RSA 7TIM 8GCH

The two circles in Fig. 17 represent category classification of the PDBID according to the feature of the protein-ligand complex, and all of those PDBID are presented. The set of the circle on the right side in the drawing may be the target proteins for pharmaceutical development, but the ligands that are binding are rich in variety, including druggable compounds, peptides, sugar chains and the like. On the other hand, for the PDBIDs of the circle on the left side, proteins that serve as the targets of pharmaceutical development are selected as in the case of the circle on the right side, but they are different from the PDBIDs of the circle on the right side and are composed of druggable ligands. To describe in more detail, the set of the circle on the right side is the result eventually manually selecting those judged to be druggable ligands according as the determination criteria based on the molecular structure of ligands such as the existence or nonexistence of heteroatoms, of hydrogen donor, of hydrogen acceptor, and of hydrophobic group, whether Lipinskis Rule of Five [reference literature (Adv Drug Deliv Rev 46 (1-3): 3-26)] is satisfied [reference literature (J. Med. Chem. 2007; 50: 726-741)].

That is, the breakdown of these benchmark sets are such that 85 benchmark sets are collected by selecting target proteins that serve as targets of drug discovery among those registered on the PDB after August 11, 2000, and finally manually selecting those judged to be druggable ligands according to the determination criteria such as whether the ligand to be docked also has heteroatoms, a hydrogen bond donor, a hydrogen bond acceptor , and a hydrophobic group and the like respectively, or whether the Lipinski's Rule of Five is satisfied. Furthermore, the Riken Benchmark [reference literature (Onodera et al. J. Chem. Inf. Model. 2007; 47: 1609-1618)] uses the benchmark of GOLD [reference literature (Gareth et al. J. Mol. Biol. 1997; 267: 727-748)]. This benchmark uses the target proteins registered on the PDB before August 2000, as mentioned above. However, since this benchmark compares AutoDock or DOCK in addition to the GOLD, it was conceived that comparing with the results of this benchmark would be very useful to find out the placement of the ChooseLD in the docking software program. There is no overlapping in the PDB ID for the two benchmarks. Thus, setup of default parameters for the ChooseLD was performed with 85 sets, and the performance evaluation of ChooseLD based on the parameters was performed with the Riken Benchmark, Here, Fig. 18 is a diagram plotting the year in which the PDBIDs proposed with 85 sets (circle on the left side) and 133 sets (circle on the right side) were registered, on the horizontal axis, and the number of total registrations in the years on the vertical axis.

The years of registration in PDB in these benchmark sets are distributed as shown in Fig. 18. To mention about the indication of colors of the groups of protein-ligand complexes of the two benchmark sets of Fig. 18, the mount on the left side of the graph represents the distribution of the year of registration in the case where the target protein is druggable (meaning that the target protein can be a subject of drug development), and where the ligands are various low molecular compounds (Green plane:133 benchmark set Gold Benchmark [reference literature (Jones et al. J. Mol. Biol. 1997; 267: 727-748), Onodera et al. J. Chem. In. Model. 2007; 47: 1609-1618)]. The mount on the right side of the graph represents the distribution of the year of registration in the case of the target protein and the ligand being all druggable compounds (Blue plane:85 benchmark set [reference literature (Hartshom et al. J. Med. Chem. 2007; 50: 726-741)]. The black line represents the average of number of PDB of each (green, blue) plane, and the average values are 9.5, and 14.2 for the green plane, and the blue plane respectively.

Fig. 19 is a table summarizing the rmsd between the prediction and the experimental results (Table. Summary of r.m.s deviation between predictions and experimental results). In order to evaluate the accuracy of the prediction structure for binding mode, the rmsd between the predictive structure and the experimental structure were calculated. A large rmsd means that the difference between the predictive structure and the experimental structure is large, that is, a failure in the prediction. Thus, the upper limit value of the rmsd to judge that a predictive structure is correct, was decided. The table in Fig. 19 presents the relationship between the rmsds between the prediction structures for binding mode and the experimental structure, and human recognition, namely, Good, Close, Errors and Wrong, as implemented by Jones et al. When the rmsd is 2.0Å or less, the predictive structure is as good as compared to the experimental structure, that is, the predictive structure gets the grade Good. When the rmsd is 2.5Å or less, since it implies that the predictive structures which are close to the experimental structure are included, there include good predictive structures. In other words, this is Close. Thus, the case of obtaining a predictive structure having an rmsd of 2.0Å or less was defined as successful prediction. When the rmsd is 2.0 or more and 2.5 or less, the visual evaluation is Good, Close, Errors or Wrong [reference literature (Jones et al. J. Mol. Biol. 1997; 267: 727-748)]. That is, when the rmsd is 2.0Å or less, the structure is good as a ligand model as compared to the correct structure. When the rmsd is 2.5Å or less, the structures include both the grade Close and the grade Good as a ligand model, as compared to the correct structure.

[Results and Discussion (1): Optimized k1 in FPA Score function]

As discussed above, the k1 value of the FPAScore is a coefficient that controls the degree of consistency between the atomic coordinates registered on the FP library and the atomic coordinates of the candidate ligand. The k1 value is possible to be changed in accordance with the target, but upon considering the case of performing in silico screening with respect to a large quantity of target proteins, or the case of being used by other researchers, determining the optimal parameter serves as one of data for judgment of employing the technique. Therefore, the optimal value of k1 of the FPAScore function was determined as the optimal value in the docking performance testing of the ChooseLD method using 85 sets [reference literature (Michael et al. J. Med. Chem. 2007; 50: 726-741)].

The 85 sets collect many of drug-like target proteins, and are therefore subjected to the performance evaluation of GOLD [reference literature (Gareth et al. J. Mol. Biol. 1997; 267: 727-748)]. This is because the 85 sets do not overlap with the 133 sets in regard with the PDBID, that is, the 85 sets do not use the information of the 133 sets in this process of optimization. Furthermore, the 85 sets are subjected only to the benchmarking of GOLD, and the success rate of GOLD was 75.2±0.4% in the case of docking the structure of Corina to the target protein; 80.5±0.5% in the case of defining the binding site as 6Å by using the ligand structure of the experimental structure; 86.9±0.3% in the case of defining the binding site as 4Å by using the ligand structure of the experimental structure; and 98.6±0.1% in the case of including the water of crystallization that is present in the X-ray crystallographic structure [reference literature (J. Med. Chem. 2007; 50: 726-741)]. That is, in the case of performing evaluation only by GOLD, the placement of ChooseLD in the existing docking software programs can be not known, and therefore, the 85 sets were used in the optimization of the k1 value. Here, the optimization of k1 described with the FPA score was performed.

The docking conditions were as described below. Similarly to other benchmarks, the ligand binding site was defined because it is advantageous to narrow the scope of search for the ligand binding site, or the like. That is, the benchmark of the docking performance testing of ChooseLD is not about predicting the amino acid residues at the ligand binding site of a protein, but is about testing the accuracy of the conformation of a candidate ligand at the ligand binding site. The size of the binding site was set at 4Å from each atom of the ligand of the correct structure of the protein-ligand complex. Furthermore, in order to test the influence of the similarity to candidate ligands of a ligand included in the FP library, the Tc with the ligand that belongs to the FP library was calculated, and thus the ligands included in the FP library were limited. The Tanimoto coefficient between the ligands to be docked and the ligands belonging to the LIBRARY LIGANDS was calculated using a drug like fingerprint (FP). The Tc range for the FP bands was set at 0.96 for the maximum value, 0.76, 0.56, and 0.08 as the minimum value.

In regard to the initial conformation, a conformation obtained by randomly rotating the dihedral angle, and a structure having the largest rmsd and sufficiently separating from the binding site among the initial ligands, was used. Using that ligand, 10 times of docking was performed for one target. Among the 85 sets, 84 sets could be docked. Here, Fig. 20 is a chart presenting a list of ratio of predictive success (relationship between k1 and Tc range) in the 85 sets.

k1 in the table of Fig. 20 is the coefficient mentioned for the FPAScore. The following numerical values are k1 values obtained by calculation. The Tc range was set at 0.96 for the maximum, 0.76, 0.56, and 0.08 for the minimum. The numerical values in the column represent the success rate (%), and the average is the average value of the range described above.

As a result, when k1=4.0, the average value had the highest success rate, as high as 62.1%, and then the results were good in order of 6.0, 3.0, 5.0, and 2.0. When the k1 value was 1.0, the success rate was poorer than the success rates of other k1 values in regard to all of the Tc ranges. The case of the k1 value being 4.0 and the case of the k1 value being 6.0 were almost equal, but the case of the k1 value being 4.0, for which the average value was slightly more excellent, was used as the optimum value. Thus, this value was used for the benchmarking of the 133 species [reference literature (Onodera et al. J. Chem. Inf. Model. 2007; 47: 1609-1618)].

Here, Fig. 21 is chart presenting the fractions capable of prediction within the $10^{th}$ rank with an rmsd of 2.0 or less. The right side diagram of Fig. 21 is a plot of the success rate at that time, but when the ranking to be accepted based on the FPAScore is extended, it was found that the probability of obtaining the predictively successful structure increases. That is, when not only one of but also a plurality of the predictive structures of higher ranking FPAScore is used, the probability of obtaining a structure close to the correct structure is increased. That is, it is conceived that it is good to use multiple predictive structures of higher ranking FPAScore as the initial structure in the optimization of complex structure by molecular dynamics calculation or quantum chemistry calculation. When the rmsd with an experimental structure regarded as successful was set at 2.0Å, it was shown that success ratio of 82.9% at the maximum was shown as far as prediction within the 10$^{th}$ rank.

Fig. 22 is chart presenting the fractions capable of prediction within the 10$^{th}$ rank with an rmsd of 2.5 (Close) or less. As shown in Fig. 22, when the rmsd with an experimental structure regarded as successful was set at 2.5Å, it was shown that success ratio of 87.6% at the maximum was shown as far as prediction within the 10$^{th}$ rank.

Fig. 23 is a chart representing the case of performing with a value other than 2.0Å for the rmsd with a correct structure that is regarded as successful. The right side diagram of Fig. 23 is a plot showing the rmsd with the experimental structure regarded as successful on the horizontal axis, and the ratio of predictive success on the vertical axis.

As explained above, at 2.5Å, approximately 70% was successful, but it was shown that in order to obtain a success rate equivalent to the success rate of the binding mode prediction, 75.2% [reference literature (Michael et al. J. Med. Chem. 2007; 50: 726-741)], in the case of not using the ligand generated by Corina, which was one of the ratio of predictive success of GOLD in the 85 set benchmarks, that is, the conformation of an experimental structure, a Tc range of 3.2 to 3.3 at 0.56 to 0.08, a Tc range of 2.8 at 0.76 to 0.08, and a Tc range of 2.6 to 2.7 at 0.96 to 0.08, should be used. In addition, when the general covalent-bond length of 1.5Å is defined as successful, about 40% of the prediction is considered successful. Within 3.5Å, which is close to the limit value of van der Waals interaction, about 80% of prediction is considered successful. Here, Fig. 24 is a chart presenting the results of benchmarking of the Dock, AutoDock and GOLD as compared to the results of the ChooseLD.

Fig. 24 is a diagram presenting the results of 116 species of PDBID, having removed of targets failed in the coordinate generation by Corina and of the targets with which docking by the DOCK or GOLD end in failure upon benchmarking by Onodera, et al. [reference literature (Onodera et al. J. Chem. Inf. Model. 2007; 47: 1609-1618)]. In Fig. 24, the success rate represents the fraction of structures having an rmsd of 2.0Å or structures better than that.

Here, the Docking method means the name of each docking software program (Docking soft). ChooseLD performs a performance evaluation on three Tc ranges. The values of GOLD GOLDScoreSTD, GOLDScoreLib, GOLD ChemScoreSTD, AutoDock, and DOCK are the average values of Corina and MINI, while the standard deviation with respect to the success rate of each docking software program is represented by an error bar.

As shown in the graph of Fig. 24, the performance of predicting a structure having an rmsd of ChooseLD of 2.0Å or better (success rate) according to the present example is almost equal to that of GOLD, when the Tc range is 0.96 to 0.08. When the Tc range is 0.76 to 0.08, the performance is nearly equal or slightly inferior to that of GOLD. It was shown that when the Tc range is 0.56 to 0.08, the performance is not comparable to that of GOLD, but is better than the performance of DOCK or AutoDock.

Here, Fig. 25 is a diagram presenting the frequency distribution of collisions of with the respective target proteins when the rmsd between the predictive structure of the FPAScore and the experimental structure in the 85 sets is 2.0Å or less. Since the structure of zero collision is 75.0%, and the structure of one collision is 17.3%, and thus the total is 92.3%, it was shown that the function of collision decision of the FPAScore functions equivalently to the decision of collision of the Lennard-Jones type function, which is an empirical physical function.

Fig. 26 and Fig. 27 present the counting of the number of successes in performing docking total 10 times with respect to each target. Fig. 26 presents the frequency distribution of predictively successful structure in the benchmarking of 85 sets. In addition, the symbol "*1" in Fig. 26 presents the fraction of the PDBIDs having the predictively successful number of 5 to 10, to the total PDBIDs. For all Tc ranges, the fractions of 10 successes and 10 failures are large. Furthermore, the target succeeded 5 times out of 10 times occupied 62.7 to 65.5%. When the upper limit of the Tc range was made small, there was a tendency that the number of structures making all 10 failures increases. This is because the ChooseLD method depends on the protein-ligand complex structures that are already known as the FP library, it is conceived that the ligands belonging to the FP library are reduced in number, and that therefore the accuracy goes down.

[Results and Discussion (2): Results of benchmarking of 133 species]

According to Onodera, et al. [reference literature (Onodera et al. J. Chem. Inf. Model. 2007; 47: 1609-1618)], benchmarking is performed in a state close to the initial state of each docking software program as received. According to them, in regard to the target proteins, the protein-ligand complex used in the benchmarking of GOLD [reference literature (Gareth et al. J. Mol. Biol. 1997; 267: 727-748)] are total 116 species, obtained by removing the targets that could not dock with GOLD or DOCK, and the targets that could not generate 3-dimensional coordinates by Corina, from 133 species. The removed PDBIDs are 1TPH, 1TRK, 1XID, 4FAB, 6RSA, 1BBP, 1CTR, 1HYT, 1PHG, 1POC, 1SNC, 1TMN, 1CDG, 1DR1, 1LDM, 4CTS, 4EST (Virtual Screening [reference literature (Onodera et al. J. Chem. Inf. Model. 2007; 47: 1609-1618)]).

For the parameters of the respective docking software programs, those parameters provided by the respective docking software programs are used, and the parameters are not optimized for targeting. It is conceived that if optimization of the parameters is carried out, the success rate would be definitely changed. However, the same also applies to the ChooseLD, and since the parameters k1, k2 and k3 values that are variable in accordance with the target protein are defined also in the ChooseLD method, there is still room for optimization. Thus, in the performance evaluation of ChooseLD, the values mentioned in the terms in the method, and the k1 value optimized with the 85 sets, that is, 4.0 were used.

Here, the docking conditions used by ChooseLD were defined as follows, with respect to each target.

1. Binding site
The binding site was defined, similarly to the case of conventional benchmarking [reference literature (Onodera et al. J. Chem. Inf. Model. 2007; 47: 1609-1618)], as a sphere of atoms of the protein present in a distance of within a radius of 5.0Å from each atom of the ligand of the native protein-ligand complex.
2. Conformation change of ligand
In the benchmarking of 133 sets, three ligands for docking are prepared. That is, the three include the ligand generated by Corina, ligand with the minimum energy structure (hereinafter, referred to as MINI) among the ligands generated by Corina, and ligand with the structure as registered on the PDB, and these are respectively subjected to 1000 predictions with respect to 116 target proteins (Virtual Screening [reference literature (Onodera et al. J. Chem. In. Model. 2007; 47: 1609-1618)]). In the docking performance test of the ChooseLD method, the conformation was randomly changed, and a ligand having a structure with the largest rmsd from the ligand of the protein-ligand complex of experimental structure, and being sufficiently far from the ligand binding site defined above, was used. That is, it was equivalent to that 10 predictions were performed with respect to each of the 116 target proteins without using the experimental structure, and thus the predictions were performed under almost the same conditions as those for the benchmarking using the 133 sets. In this process, the hydrogen atoms were eliminated in case of a ligand having hydrogens in the molecule.
3. Scope of Tanimoto coefficient with ligand
In regard to the LIBRARY LIGAND used, the values 0.96, which is the maximum value, 0.76 and 0.56 in the range of Tc with a candidate ligand (docked ligand) correspond to the implications that a compound very similar to the docking ligand exists, that a compound similar to the docking ligand exists, and that a compound slightly similar to the docking ligand exists, respectively. Thus, for the scope of Tc, the values corresponding to 0.96 to 0.08 (that is, solution not included), 0.76 to 0.08, and 0.56 to 0.08 were used.
4. Onodera, et al. performs docking runs 1000 times with respect to one ligand [reference literature (Onodera et al. J. Chem. Inf. Model. 2007; 47: 1609-1618)]. In this performance evaluation of ChooseLD, the candidate ligand (docked ligand) was docked 10 times. That is, docking runs for 1160 times was performed for each Tc range, so that docking was performed 3480 times in all. It was considered successful if the rmsd between the docking structure predicted in one docking run, and the ligand of the native protein-ligand complex was 2.0Å or better.

Fig. 28 and Fig. 29 are diagrams presenting the results of the rmsd distribution of the predictive structures of DOCK, AutoDock and GOLD and the results of the ChooseLD method in the benchmarking of 133 sets. The Docking method means the respective names of the docking software programs. ChooseLD performs a performance evaluation with respect to three Tc ranges. GOLD used the three parameters of 'Standard Default Settings' with GOLDScore (GOLDScoreSTD), 'Library Screening Settings' with GOLDScore (GOLDScoreLib), and 'Standard Default Settings' with ChemScore (GOLDChemScoreSTD) (Virtual Screening[reference literature (Onodera et al. J. Chem. Inf. Model. 2007; 47: 1609-1618)]), and AutoDock and DOCK used the average values of Corina and MINI. From this graph, the performance of ChooseLD to predict a structure having an rmsd of 2.0Å or less is almost equivalent to GOLD when the Tc range is 0.96 to 0.08. When the Tc range is 0.76 to 0.08, the performance is almost equivalent or slightly inferior to that of GOLD. When the Tc range is 0.56 to 0.08, the performance is shown to be better than that of DOCK or AutoDock.

Fig. 30 and Fig. 31 indicate the number of successes in performing docking with respect to each target for total 10 times. The symbol "*1" in Fig. 30 represents the ratio of the number of predictive successes being 5 to 10, to the total number of the PDBID. Similarly to the case of 85 sets, polarization in the ratio of ten successes and ten failures occurs, but the case of ten failures was shown to be most frequent. Furthermore, in comparison to the case of 85 sets, the success rate for 10 times is decreased by near 20%. From these results, it is speculated that the 133 sets include many targets that are difficult to dock, as compared to the 85 sets. Since the druggable compounds in the 85 sets have the molecular weight, the number of rotatable bonds, the number of hydrogen donor, and the number of hydrogen receptor, limited by Lipinskis' Five Rule or the like, it is conceived that the 85 sets include many compounds that are likely to make docking easily under the influence of the narrowed selection.

Fig. 32 and Fig. 33 are diagrams presenting the probability of obtaining a structure having an rmsd with an experimental structure of 2.0Å or less from the distribution of FPAScore ranking in the FP library that has been limited to the Tc range. That is, the first rank coincides with the success rate of the comparison with other docking software programs described above. This result also implies a decrease in the overall success rate, similarly to the case of 85 sets.

Fig. 34 is a diagram presenting the frequency distribution of collisions of the predictively successful structures, and presents the frequency distribution of collisions with each target protein for those structures having an rmsd between the predictive structure and the experimental structure of 2.0Å or less in the 133 sets. The ratio of the structures having zero collision was 56.0%, and the ratio of the structures having one collision was 28.7%, so that the total was 84.6%. Thus, it was shown that the function of collision decision by the FPAScore functions to be equivalent to the decision of collision performed by the Lennard-Jones type function, which is an empirical physical function. Since the 85 sets and the 133 sets all show the same tendency, it is speculated that the decision of collision is functioning satisfactorily.

Fig. 35 is a diagram presenting the performance in the case of further lowering the upper limit value of the Tc range of the ligands used in the FP library to be 0.16, 0.24, 0.36, and the lower limit value to be 0.08, and the ratio of predictive success in the Tc range described above, namely, 0.56, 0.76, 0.96 as the upper limit value, and 0.08 as the lower limit value. It was shown that when the upper limit value of Tc was lowered, the prediction accuracy was equivalent to that of DOCK (21.1%) in the case of benchmarking 133 sets at 0.24 to 0.08, while the prediction accuracy was equivalent to that of AutoDock (26.6%) in the case of benchmarking 133 sets at 0.36 to 0.08.

(Comparison with GOLD)

Two examples with an rmsd of 2.0 or less, that could dock in the method of the present inventors, although GOLD failed in the Riken benchmarking, will be presented.

Fig. 36 is a diagram showing the predicted protein-ligand complex structure for 1DR1.

Conditions and values in Fig. 36 are described below:

PDBID: 1DR1
TITLE: CHICKEN LIVER DIHYDROFOLATE REDUCTASE
DOCKED LIGANSD: NADP
RMSD: 1.743
FPA: Score 1295.553
CYAN (central cyan parts in the figure): experimental (X-ray crystallographic analysis ) structure (Answer) (same in the following figures)
GREEN (central deep green parts in the figure): predicted ligand Structure (same in the following figures)
The other: the binding site (same in the following figures).

That is, Fig. 36 shows the predictive structure of the present example with respect to PDBID; 1DR1. This is a target protein that GOLD has failed predicting, that is, a target excluded from the benchmarking of the 133 sets (Virtual Screening[reference literature (Onodera et al. J. Chem. Inf. Model. 2007; 47: 1609-1618)]). ChooseLD of the present example found that the rmsd between the predictive structure and the experimental structure was 1.74Å, and thus succeeded in prediction. It is speculated that this is because the ring structure present in the ligand is also included in the FP library in a large quantity.

Fig. 37 is a diagram showing the predicted protein-ligand complex structure for 4EST.

Conditions and values in Fig. 37 are described below:

PDBID: 4EST
TITLE: CRYSTAL STRUCTURE OF THE COVALENT COMPLEX FORMED BY A PEPTIDYL ALPHA, ALPHA-DIFLUORO-BETA-KETO AMIDE WITH PORCINE PANCREATIC ELASTASE AT 1.78-ANGSTROMS RESOLUTION DOCKED LIGAND: INHIBITOR ACE-*ALA-*PRO-*VAL-*DIFLUORO-*N-*PHENYLETHYLACETAMIDE
RMSD: 1.729
FPASCORE: 451.291.

That is, Fig. 37 presents the predictive structure of the present example with respect to PDBID; 4EST. This is a target protein that GOLD has failed predicting, that is, a target excluded from the benchmarking of the 133 sets (Virtual Screening[reference literature (Onodera et al. J. Chem. Inf. Model. 2007; 47: 1609-1618)]). ChooseLD found that the rmsd between the predictive structure and the experimental structure was 1.73Å, and thus succeeded in prediction. It is speculated that this is because the docking ligand is often a peptidic ligand, and carbon, nitrogen and oxygen in the main chain of the peptidic ligand included in the FP library have been mainly used.

[Results and Discussion (2.1): results of predicted structure]

Four examples of all the docking instances that existing docking software programs (GOLD and DOCK) have failed will be presented.

Here, Fig. 38 to Fig. 41 are diagrams presenting the targets that GOLD has failed, but ChooseLD has succeeded in prediction.

Conditions, and the like in Fig. 38 are described below:

PDBID: 1CDG

TITLE: NUCLEOTIDE SEQUENCE AND X-RAY STRUCTURE OF CYCLODEXTRIN GLYCOSYLTRANSFERASE FROM BACILLUS CIRCULANS STRAIN 251 IN A MALTOSE-DEPENDENT CRYSTAL FORM.

Conditions, and the like in Fig. 39 are described below:

PDBID: 1DR1
TITLE: 2.2 ANGSTROMS CRYSTAL STRUCTURE OF CHICKEN LIVER DIHYDROFOLATE REDUCTASE COMPLEXED WITH NADP+ AND BIOPTERIN.

Conditions, and the like in Fig. 40 are described below:

PDBID: 1LDM
TITLE: REFINED CRYSTAL STRUCTURE OF DOGFISH M4 APO-LACTATE DEHYDROGENASE.

Conditions, and the like in Fig. 41 are described below:

PDBID: 4EST
TITLE: CRYSTAL STRUCTURE OF THE COVALENT COMPLEX FORMED BY A PEPTIDYL ALPHA, ALPHA-DIFLUORO-BETA-KETO AMIDE WITH PORCINE PANCREATIC ELASTASE AT 1.78-ANGSTROMS RESOLUTION.

(Comparison Including GLIDE)

Glide [reference literature (J. Med. Chem. 2004; 47: 1739-1749)] is a flexible ligand docking software program, and performs a comparison of prediction accuracy with GOLD or the like, in the method of the present example. Fig. 42 is a chart presenting the ratio of predictive success for 90 targets in the 133 sets. However, the method for calculating the ratio of predictive success in the above table varies with each of the docking software programs. That is, GOLD gave the results of performing 20 runs of optimization by a genetic algorithm for each target (the best of GA 20 run) (http://www.ccdc.cam.ac.uk/products/life_sciences/validate/ gold_validation/value.html), while ChooseLD selected two higher FPAScores by performing 10 runs of docking for each target, and selected the best structure. Since there is no description verifying the docking performance of Glide, the performance is considered to be equivalent to that of GOLD. In regard to the results of benchmarking the 133 sets, from the fact that the ratio of predictive success of GOLD was about 45%, it is conceived that the ratio of predictive success fluctuates to a large extent depending on the docking conditions and the method of selecting the predictive structure.

(Distribution of predictive successful target proteins)

Fig. 43 is a chart presenting the degree of similarity of the PDBIDs of a successfully predicted target protein between the docking software programs, calculated in terms of Tc (Tanimoto coefficient). Here, for the 90 sets among the 133 sets, if both of the docking software programs succeeded in prediction for the respective target proteins, the numerical value is added to a in the formula for Tc, calculation, and if only one succeeded in prediction, the numeric value is added to b or c.

As shown in Fig. 43, the Tc between Glide, GOLD and FlexX [reference literature (J. Mol. Biol. 1996; 261: 470-489)] were 0.61 to 0.65, while the Tc between CHooseLD and another docking software program were about 0.47 to 0.55. It was conceived that since there was no significant difference in the ratio of predictive success among GOLD, Glide and ChooseLD, this ChooseLD, has originality in the distribution of target proteins with which prediction is succeeded, as compared with other docking software programs.

Fig. 44 is a cross table showing the distribution of success and failure of prediction by the respective docking software programs with respect to one target protein among the 90 targets. There are many targets that can be predicted by one docking software program, and thus in the current situation, it is said that there is no docking software program that succeeds in the prediction of all target proteins. Under such circumstances, research is more frequently conducted, on the assumption that multiple docking software programs are used, not by selecting predictive structures based on the scores given by the docking software programs, but by acquiring information on the interaction with proteins such as hydrogen bond, from predicted target protein-ligand complex structures, and selecting a predictive structure that is closer to the experimental structure [reference literature (Eur. J. Med. Chem. 2007; 42: 966-976), (J. Med. Chem. 2004; 47: 337-344)].

Here, Fig. 45 to Fig. 47 are diagrams presenting the targets that DOCK failed but ChooseLD succeeded in prediction.

Conditions, and the like in Fig. 45 are described below:

PDBID: 1HYT
TITLE: RE-DETERMINATION AND REFINEMENT OF THE COMPLEX OF BENZYLSUCCINIC ACID WITH THERMOLYSIN AND ITS RELATION TO THE COMPLEX WITH CARBOXYPEPTIDASE A.

Conditions, and the like in Fig. 46 are described PDBID: below:

1PUG
TITLE: CRYSTAL STRUCTURES OF METYRAPONE-AND PHENYLIMIDAZOLE-INHIBITED COMPLEXES OF CYTOCHROME P450-CAM.

Conditions, and the like in Fig. 47 are described below:

PDBID: 1TMN
TITLE: BINDING OF N-CARBOXYMETHYL DIPEPETIDE INHIBITORS TO THERMOLYSIN DETERMINED BY X-RAY CRYSTALLOGRAPHY. A NOVEL CLASS OF TRANSITION-STATE ANALOGUES FOR ZINC PEPTIDASES.

[Results and Discussion (3): results of accepted rankrange]

Fig. 48 is a diagram presenting the fraction for which the structure with an rmsd of 2.0 can be collected not only for the 1st rank but also within the 10th rank. As shown in Fig. 48, when the structure is collected to the 10th rank, 60% or more is capable of docking with an rmsd of 2.0 or less.

Fig. 49 is a diagram presenting the fraction for which the structure with an rmsd of 2.5 (Close) can be collected not only for the 1st rank but also within the 10th rank.

[Results and Discussion (4): result_rmsd_regard_as_suceess]

The rmsd defined as successful is changed. At the time of comparing with Riken benchmarking, the rmsd between the predictive structure defined as successful and the correct structure was defined to be 2.0Å, but numerical values other than that (1.5, 2.5, 3.0 and 3.5) may use as well. It is because when the rmsd is 3.5Å, the predictive ligand structure is in most cases thought to be present in the neighborhood of the ligand binding site, and thus the structure can be used as the initial structure for the calculation of molecular dynamics or quantum chemistry. Fig. 50 is a chart presenting the instance of changing the rmsd that is defined as successful.

As shown in Fig. 50, the fraction of the structure that could be predicted within 3.5Å was 68.9% for the Tc range of 0.56 to 0.08 (that is, when slightly similar ligands are present in the library). That is, it is implied that if the experimental structures of similar compounds exist, the docking structure can be predicted to be at least in the neighborhood of the ligand binding site, with this degree of accuracy.

It is also implied that in regard to the Tc range of 0.96 to 0.08 (that is, when fairly similar ligands are present in the library), about 70% is present at the ligand binding site.

Here, the value of rmsd of 2.0 as the definition of successful docking is a basic evaluation criterion for various benchmarks and the like [reference literature (Gareth et al. J. Mol. Biol. 1997; 267: 727-748), (Michael et al. J. Med. Chem. 2007; 50: 726-741), (Onodera et al. J. Chem. Inf. Model. 2007; 47: 1609-1618)]. However, in fact, even in the cases of having an rmsd larger than 2.0, when optimization such as MD or QM is performed, the structure of a protein-ligand complex can be predicted with good accuracy. That is, presenting such an rmsd defined as successful serves as useful data when an investigator using MD or QM selects the initial structure for the optimization of the complex structure. That is, it is speculated that the data may be instructive for the supposition of the time taken for the optimization (shot time 100 ps, long time 1 ns and so on) or the scope of the ligand binding site to be optimized (5Å, 10Å and so on).

[Results and Discussion (5): method_ideal]

Discussion will be mainly described below, while referring to Fig. 8 again.

In the present example, an assumption is made such that the conformation of FP, which is a part of the ligand, is most stable as a structure subjected to interaction. In regard to the interaction with the target protein of an FP according to the present example, an FP in a short distance from the protein is interpreted as enthalpic interaction including hydrophobic interaction, hydrogen bond interaction, and van der Waals interaction, while an FP in a long distance from the protein is interpreted as entropic interaction such as interaction with a solvent.

That is, according to the present example, it is assumed, finally using the conformation of an FP, that when a chemical compound takes the most stable docking structure as the basic, this is equivalent to the structure taking the most stable free energy for the protein-ligand interaction.

That is, an FP conformation extracted from a group of binding ligands derived from homologous proteins showing satisfactory the overlap, includes the free energy interaction with protein.

Here, when there is one target protein, a homologous protein having low homology or low e-value is used in order to gather many ligands, but a family protein in a broad sense, which is not bound by these functional classifications, is accompanied by slight structural changes and changes in the amino acid residue at the neighborhood of the binding site. Thus, the possibility that the FP extracted from the family protein does not satisfy the assumption of stabilizing free energy, should also be considered.

Therefore, this drawback needs to be compensated, and thus the FP extracted from a family protein is changed to an FP having more stable free energy with respect to the interaction with the target protein to be a "Modified FP," which is accepted as a slightly less reliable FP. This copes with modifying the program of the 3D-1D method. When this Modified FP is produced for a target protein, this is equivalent to the considering of an undiscovered ligand having a new skeleton. Thus, there is a possibility of finding a compound having higher activity than the known ligands that are bound to the target protein.

Meanwhile, the FP in the common region of atomic interaction of a plurality of binding compounds attaches importance to the overlap of the family proteins binding to a plurality of similar chemical compounds. Thus, it is speculated that an FP to which experimental information has been more reflected than the "Creative FP," which is given when there is a possibility of the atomic presence by the biochemical information or energy calculation can be obtained.

[Protein-Ligand Complex Optimize for Other Method (MD, QM)]

With respect to the structure of a protein-ligand complex predicted by conventional energy of classic physics, ranking the docking structures obtained by the method described above, and clustering is performed, using the known information of the structure of a protein-ligand complex [reference literature (Zhan et al. J. Med. Chem. 2004; 47: 337-344)]. This implies that the output by an existing docking software program is equivalent to outputting a structure which does not certainly reflect the experimental information.

Meanwhile, there have been attempts to optimize the predicted structure of a protein-ligand complex with MD using AMBER or CHARMM [reference literature (Case, A. D., Cheatham III, E. T., Darden, T., Gohlke, H., Luo, R., Merz Jr., M. K., Onufriev, A., Simmering, C., Wang, B. Woods, J. R. The Amber Biomolecular Simulation Programs, J. Comput. Chem. 2005; 26: 1668-1688), (Brooks, R. B, Bruccoleri, E. R., Olafson, D. B., States, J. D., Swaminathan, S. & Karplus, M. CHARMM A program for macromolecular energy, minimization, and dynamics calculations. J. Comp. Chem. 1983; 4: 187-217)], or QM [reference literature (Kamiya K, Sugawara Y, Umeyama H. J. Comput. Chem. 2003; 24: 826-841)]. With these methods of MD or QM, performing docking or in silico screening involves an excessively large amount of calculation, it is needed to performing docking of a ligand at a location somehow close from the native structure of a protein-ligand complex, and use this as the initial structure.

Existing docking software programs are used to obtain the initial structure, but since these programs are based on the physical energy previously mentioned, optimization based on physical energy is repeated.

On the other hand, the technique according to the present example mainly uses known information of a protein-ligand complex, and thus it is possible to take into consideration of the viewpoint of bioinformatics and the viewpoint of physical energy. Since the bioinformatics information called the structure information of PDB used in the present example is accumulated every year, the protein-ligand complexes attracting the public interest from a medical viewpoint are studied by many researchers, and are also conceived to be useful for such optimization of predictive structures.

[Conclusion: Performance of the Present Technique]

Fig. 51 is a cross table showing the result of processing according to the present example. As shown in Fig. 51, when the technique according to the present example was used, in the case of docking a drug-like ligand with a druggable protein for the T85 set, when the Tc range was 0.56 to 0.08, 0.76 to 0.08, and 0.96 to 0.08, the probability of obtaining a structure graded Good was 58.9, 62.1 and 65.2%, respectively, and the probability of obtaining a structure graded Close was 68.6, 72.1, and 72.4%, respectively.

In regard to the performance in the case of docking various ligands with a druggable target protein, when the Tc range was 0.56 to 0.08, 0.76 to 0.08, and 0.96 to 0.08, the probability of obtaining a structure graded Good was 40.1, 44.8, and 46.4%, respectively, and the probability of obtaining a structure graded Close was 53.2, 57.8, and 59.3%, respectively. It was shown that these performances were almost equivalent to the performances of existing docking software programs.

From the results of training calculation including compounds that are all druggable as the target protein and the ligand, when the conformations for the top ten interaction scores between a target protein and an arbitrary ligand are contemplated, the ligand structure including the solution in the range of 2.0Å, which is said to give a good model for the solution, is found for at least one time in 83% of the entire target proteins (values down to the $10^{th}$ rank, 0.96 to 0.08 of Fig. 21). Thus, it is worthwhile to find a good structure by visual inspection.

On the other hand, when the conformations for the top ten interaction scores between a target protein and an arbitrary ligand are contemplated, the ligand structure including the solution in the range of 2.5Å, which is said to give a model similar to the model that is good for the solution, is found for at least one ligand structure in 88% of the entire target proteins (values within the $10^{th}$ rank, 0.96 to 0.08 of Fig. 22). Thus, it is good to perform visual inspection. It is worthwhile to find a structure or a similar model structure.

From the results of training calculation including a druggable target protein, and various low molecular compounds as the ligand, when the conformations within the top ten interaction scores between a target protein and an arbitrary ligand are contemplated, the ligand structure including the solution in the range of 2.0Å, which is said to give a good model for the solution, is found for at least one ligand structure in 65% of the entire target proteins (values within the $10^{th}$ rank, 0.96 to 0.08 of Fig. 48). Thus, it is worthwhile to find a good structure by visual inspection.

On the other hand, when the conformations within the top ten interaction scores between a target protein and an arbitrary ligand are contemplated, the ligand structure including the solution in the range of 2.5Å, which is said to give a model similar to the model that is good for the solution, is found for at least one ligand structure in 76% of the entire target proteins (values down to within the $10^{th}$ rank, 0.96 to 0.08 of Fig. 49). Thus, it is worthwhile to find a good structure or a similar model structure by visual inspection.

Traditionally, this interaction between a target protein and a low molecular compound from a library of virtual compounds has been calculated by a physical interaction function, but the present example differs from the conventional techniques in view of semiempirically calculating using the information of bioinformatics. Furthermore, the present example also has excellently high effects in terms of the success rate of structure prediction, as compared to the globally acknowledged docking software program, GOLD. Also, since the accumulation of information that is increasing every year leads up to obtaining better results for the interaction calculation of the semiempirical bioinformatics technique, the technique of the present example is highly useful and gives effects that are different from those of conventional techniques.

According to the present example, the conformations obtained by scoring of the interactions between a target protein and various low molecular compounds, can be used in the DOCK, AutoDock or GOLD, which are docking programs involving the calculation formulas of molecular dynamics, and also can be used as the initial conformations of existing docking software programs such as Amber or Charm, which are molecular dynamics calculation programs. In this regard, since the initial conformations obtained in the present example can be conveniently obtained, and the accuracy of reproducing the experiments is high, useful results can be obtained by combination with other software programs.

Furthermore, the present example can be carried out by a method that does not require analyzing the 3-dimensional structure of the target protein and designating the active site in the process of calculation using an arbitrary FP (fingerprint) based on the CElib (FP (fingerprint) set extracted from collected ligands in the binding site), which is a database of various low molecular compounds bound to a family macromolecular protein set having a 3-dimensional structure similar to that of the target protein. In the conventional techniques, it was necessary to analyze the 3-dimensional structure of the target protein and to designate the active site in advance, in the docking calculation using existing docking software programs such as DOCK, AutoDock or GOLD, so that stable conformations would receive higher scores. However, compared to these, the present example has high effects that are different from those of the conventional techniques, and does not require designation of the active site through learning from the literature, thus being useful.

[Conclusion]

The method according to the present example succeeded in using the score which defines the information on the interaction of a protein-ligand complex that is already known from the viewpoint of bioinformatics, and reflecting this appropriately in docking simulation.

Attempts to enhance the accuracy in the docking simulation by adding the known information of protein-ligand complex to the output of existing docking software programs have been traditionally carried out. However, these methods depend on the intelligence and action of the researcher, and thus lack generality.

The technique according to the present example automatically performs the homology search and the superimposition of 3-dimensional structure. Furthermore, by using the scoring functions suggested by the present technique, docking structure could be obtained with high accuracy.

Based on these, the technique can be widely used without requiring too much human intervention by the researcher. The scoring functions suggested by the present technique can also be combined with existing docking software programs.

That is, the method according to the present example is extremely useful for the following three aspects.

The technique of the present example differs from conventional techniques in that the information of the interaction of protein-ligand complex that is already known from the viewpoint of bioinformatics can be appropriately reflected in the docking simulation. Furthermore, the technique of the present example exhibits excellent effects of being capable of automatically adding the parameters of the physical amount of suitable for the ligand and the constraint on distance, while taking into consideration of the complementarity with the receptor, and the conformation and atomic species of the known ligand. Of course, these aspects are very useful in the search of pharmaceuticals of new skeletons or similar skeletons, because the bioinformatics information on the interaction between target protein and ligands, which is important from neomedicinal and biological viewpoints, is accumulated every year. Furthermore, due to the coming of the age of tailor-made medicine, drug design of target proteins with rich experimental information will be required, and the method according to the present example is very useful.

[Second Example]

As a second example, the optimization of k2 and k3 and in silico screening in the case that the target protein is Epidermal growth factor receptor (EGFR) will be described below. Here, Fig. 52 is a diagram presenting an intracellular signal transduction pathway starting from EGFR.

The k2 and k3 values in the score of FPAScore defined in the ChooseLD method of the first example have been defined as coefficients capable of optimizing in accordance with the target protein. Thus, verification was performed on whether the coefficients would function effectively with respect to the target protein. EGFR, which is an epidermal growth factor receptor family, serves as an important inhibitory target in cancer therapeutic [reference literature (J. Biol. Chem. 2002; 277: 46265-46272, Cell 2006; 125: 1137-1149)]. Therefore, in silico screening was performed using EGFR as the target protein.

(Construction of 3-dimensional structure of EGFR)

For the amino acid sequence of EGFR, ACCESSION ID P00533 in NCBI [reference literature (Wheeler, D. L. et al. Nucleic Acids Res. 2007 Nov 27)] was used, and the A chain of PDBID 1M17 was used as the template. The alignment used was presented in Fig. 53. Fig. 53 is a diagram presenting the alignment of the amino acid sequence of EGFR.

Homology is about 99%, and the purpose is to append the lack in residues at the C-terminus of 1M17, rather than predicting the 3-dimensional structure. A model was constructed using the alignment with a homology modeling software, FAMS Ligand & Complex[reference literature (Proteins, 2005; Suppl 7: 122-127)]. Here, Fig. 54 is a diagram presenting constructed model of EGFR.

The CIRCLE score [reference literature (Terashi, G. et al. Proteins, 2007)] was 71.367. The score of the template 1M17_A was 82.110. The CIRCLE score is a statistical potential obtained from the X-ray structure of a protein which belongs to the experimental structure coordinate database obtained by the PDB or the like. As the score increases more positive, the environment of the known protein X-ray structure is more satisfied, that is, it can be said that the model is closer to the X-ray structure.

(Construction of FP library specific to EGFR)

The PDBID of the ligands used as the FP library obtained according to the ChooseLD method of the second example is as follows.
1AD5,1AGW,1BYG,1E9H,1FGI,1FIN,1FPU,1FVV,1GAG,1H1P,1H1Q,1H24 ,1H25,1H26,1H27,1I44,1IEP,1IR3,1JPA, 1JQH,1K3A,1KSW,1M17,1M5 2,1MP8,1MQB,1OEC,1OGU,1OI9,1OIU,1OPJ,1OPK,1OPL,1PF8,1PKG,1Q CF,1QMZ, 1QPC,1QPD,1QPF,1QPJ,1ROP,1RQQ,1SM2,1SNU,1T46,1U4D,1 U54,1U59,1UWH,1UWJ,1VYW,1XBB,1XBC, 1XKK,1Y57,1Y6A,1Y6B,1YKR, 1YOL,1YOM,1YVJ,1YWN,2B54,2B7A,2BDF,2BDJ,2BKZ,2BPM,2C0I,2C0O ,2C0T, 2C4G,2C5N,2C5O,2C5P,2C5T,2C5V,2C5X,2DQ7,2E2B,2ETM,2EV A,2EXM,2F4J,2FB8,2FGI,2FO0,2G1T,2G2F, 2G2H,2G2I,2G9X,2GNF,2G NG,2GNH,2GNI,2GQG,2GS6,2GS7,2H8H,2HCK,2HEN,2HIW,2HK5,2HWO,2 HWP, 2HYY,2HZ0,2HZ4,2HZI,2HZN,2I0V,2I0Y, 2I1M, 2I40, 2ITN,2ITO, 2ITP,2ITQ,2ITT,2ITU,2ITV,2ITW,2ITX,2ITY,2ITZ, 2IVS,2IVT,2IVU ,2IVV,2IW6,2IW8,2IW9,2J0J,2J0K,2J0L,2J0M,2J5F,2J6M,2NRU,2NR Y,2OF2,2OF4,2OFU,2OFV, 2OG8,2OIQ,2OJ9,2OO8,2OSC,2OZO,2P0C,2P 2H,2P2I,2P4I,2SRC,2UUE

(Acquisition of compounds with known IC50)

Eleven 2-dimensional structures of compounds that competitively inhibit EGFR, with known IC50 value were obtained from the website of BIOMOL (http://www.biomol.com/). Fig. 55 is a diagram showing the 2-dimensional structure of the obtained eleven inhibitors. In Fig. 55, the IC50 values are shown correspondingly to the 2-dimensional structure of the respective compounds. The 3-dimensional coordinates of these compounds, which were performing the energy minimization calculation attached to the Chem3D after generating 3-dimensional structures using Chem3D, were used.

(Optimization of k2 and k3 values for in silico screening of EGFR)

An experiment was performed by changing the k2 value of the FPAScore in the range of 0.5 to 5.0, assuming the MDL Comprehensive Medicinal Chemistry (MDL CMC) Library (Symyx Technologies, Inc. Corporate Address: 3100 Central Expressway, Santa Clara, CA 95051) to be dummy compounds having inactivity toward EGFR, and examining whether a known inhibitor would rank highly as compared to those compounds.

Fig. 56 is a diagram presenting a line chart of harvest rate when the k2 value defined for the FPAScore was changed in the range of 0.5 to 5.0. At this time, the k3 value was set at 1.0. The straight line of random is a straight line for the assumed ranking of obtaining known inhibitors in the case of randomly selecting compounds from a population. If a broken line can be drawn at a lower position than this straight line, this implies that the ability to detect an inhibitor is more highly in the ranking of the FPAScore, in other words, the performance of in silico screening is good. When the k2 value was 0.5, 1.0 or 5.0, the broken line started to increase from the position of the 6$^{th}$ rank in the appearance of compound. When the broken lines for k2 values of 2.0 and 3.0 were compared, the line at 2.0 had a more satisfactory harvest rate for the 9$^{th}$ and 10$^{th}$ ranks. Thus, the k2 value was determined to be 2.0.

Fig. 57 is a diagram presenting a line chart of harvest rate when the k3 value defined for the FPAScore was changed in the range of 0.5 to 2.0. At this time, the k2 value was set at 1.0. For any k3 value, approximately similar straight lines were obtained. But when the k3 value was 0.5 or 2.0, the broken lines turned up for the 10$^{th}$ and 11$^{th}$ ranks, and therefore, a k3 value of 1.0 was designated as the optimum value.

(Optimization of Tc lower limit value)

The lower limit value of Tc for the ligands that may be included in the FP library was determined. By defining the lower limit value of Tc, compounds that are not similar to the docking ligand can be excluded. The Tc lower limit value with which the line chart of harvest rate would become optimal, was determined.

Fig. 58 is a diagram presenting the results of in silico screening for the respective Tc ranges, when the Tc upper limit value was set at 1.00, and the range of the Tc lower limit value was changed from 0.08 to 0.32 at an increment of 0.08. The horizontal axis represents the number of appearances of known compounds with activity, while the vertical axis represents the ranking in the FPAScore. For the case of the Tc lower limit value being 0.24, since the broken line is satisfactory, which appears like creeping near the bottom along the x-axis, for the number of appearances of 1 to 6, this value was designated as the optimum Tc lower limit value. In addition, the broken line for a Tc lower limit value of 0.32 rapidly increases from near the number of appearance of 2. This is considered to be because those ligands that should be used in the FP library have been excluded by the refined selection based on the Tc lower limit value. Thus, it is conceived that in the in silico screening, mere inclusion of those ligands having FPs similar to that of the docking ligands means that the screening is unsuccessful.

Fig. 59 is a diagram presenting the PDBIDs for which the protein-ligand complex structures are registered on the PDB are already known, and the ranking of their ligands according to FPAScore. Fig. 60 is a diagram of corresponding the ligand IDs and the compound names in Fig. 59. As shown in Fig. 59, EGFR inhibitors as well are included in the ranking of the ligands. Since these ligands are included in the FP library, it is conceived that the FPs derived therefrom are mainly used in the FP alignment, and thus the FPAScore is raised to be ranked highly. In the in silico screening with the Tc lower limit value being 0.24, as compared to the case of 0.08, the ranking of appearance of these ligands dispersed. However, since those compounds having known IC50 values against EGFR, for which the protein-ligand complex structures have not been clarified, drew a satisfactory curve of harvest rate when the Tc lower limit value was 0.24, it was conceived that the Tc lower limit value of 0.24 was the optimum.

(Results of in silico screening)

The results for the in silico screening of EGFR under the conditions of k2=2.0, k3 value=1.0, and Tc lower limit value=0.24, will be presented below. Among the one hundred high-ranking structures, 97 structures were ATP derivatives including phosphoric acids. Thus, refined selection as follows was carried out.

(1) Including molecules having a molecular weight of 350 or more and 800 or less, and excluding molecules including phosphorus from those;
(2) Excluding molecules that do not involve important hydrogen bond (nitrogen in the main chain of MET); and
(3)Excluding docking ligand molecules for which collision between a protein atom and a ligand atom occurs at 2.0Å or less.

Fig. 61 and Fig. 62 are diagrams presenting the protein-ligand complexes of high ranking to the 10th rank, as a result of refined selection by in silico screening of Kinase. Fig. 62 is a view of Fig. 61 from another angle. A structure satisfying complementarity to the 3-dimensional structure within the space of the Kinase domain, and filling the hydrogen bond that is important for interaction, was found to be present in the ranking based on the FPAScore. Thus, it was found that the ChooseLD method of the present example is also useful for the search of inhibitors based on in silico screening. Additionally, these reagents are commercially available, and it is possible to measure the activity values of these. However, since the ranking based on the FPAScore is not a score which directly represents the intensity of the inhibitory activity of target protein, it is conceived that the score that is given to the FP, should not be uniformly given in a manner dependent on the method for FP establishment, but the score can be improved on a score that is likely to reflect the size of the binding constant as well.

[Example of the Application]

The results of applying the ChooseLD method according to the examples 1 and 2 to various target proteins, are shown below. These results require demonstration by experiment. A first example relates to the search for an inhibitor of dimmer formation of EGFR. A second example is related to the prediction of the complex structures of KRN633 and KRN951 with VEGF2, and the prediction of the protein-ligand complex structure requires demonstration by X-ray structural analysis. A third example is related to in silico screening against malaria, and requires demonstration by a binding experiment.

(In silico screening of inhibitor for TGFα-binding domain of EGFR)

As shown in Fig. 52, EGFR is known to be involved in signal transduction by forming a dimmer [reference literature (Nat. Rev. Cancer. 2004; 4: 361-370)]. Transforming Growth Factor α (TGF-α), which binds to EGFR as a ligand, is a peptide needed to form a complex with EGFR. That is, development of an inhibitor for the TGF-α binding domain of EGFR becomes an target object of drug discovery. Thus, in silico screening for the TGF-α binding domain of EGFR was performed using the ChooseLD method. PDBID IMOX was used as the reference protein for 3-dimensional structure modeling of EGFR. Its model was constructed on condition that the peptide of a TGF analog was located in the neighborhood of the TGF-α binding domain using FAMS Ligand & Complex [reference literature (Proteins 2005; 61: 122-127)], and the side chain was cut out.

Fig. 63 is a diagram presenting the neighborhood of the TGF-α binding domain, and the yellow color represents only side chain cut out from the peptide of a TGF-α analog. Thus, this was used as the FP library of the ChooseLD method. This was performed for the purpose of preventing a peptidic inhibitor from appearing in the high ranks of the FPAScore.

Fig. 64 is a diagram presenting the results of in silico screening for the TGF-α binding domain of EGFR using the MDL Comprehensive Medicinal Chemistry (MDL CMC) Library, and Fig. 65 is a diagram presenting the results of the same in silico screening using the MDL ACD Library. Thereby, it was shown that docking using the information of protein-protein interaction is made possible by the present example.

(Prediction of complex structures of KRN633 and KRN951 with Vascular endothelial growth factor Receptor-2 (VEGFR2)).

VEGFR2 is a kinase participating in vascularization, and is one of the proteins that are overexpressed at the development of cancer such as lung cancer. A compound which specifically inhibits this protein serves as a therapeutic drug for cancer. As the inhibitors, KRN633 [reference literature (Mol. Garxoer. Ther. 2004; 3: 1639-1649)] and KRN951 [reference literature (Cancer Res. 2006; 66: 9134-9142)] is known. However, X-ray crystallographic structure analysis of these complex has not been achieved in December 2007. Thus, the complex structures of VEGFR2 and KRN633 and the complex of VEGFR2 and KRN951 were predicted. Here, Fig. 66 is a diagram showing the 2-dimensional structure of KRN633 (IC50 = 1.16nm/L), and Fig. 67 is a diagram showing the 2-dimensional structure of KRN951 (IC50 = 0.16nm/L).

The 3-dimensional structure of VEGFR2 was constructed using PDBID 2P2H A chain as the reference protein. To describe the conditions for the docking of KRN633 and KRN951, the ligand used in the FP library was obtained by a homology search based on PSI-Blast, and the top ten compounds in the FP library used in docking were, for the KRN633, PDBID: 2HZN_A, 1YWN_A, 2J5F_A, 2IVU_A, 2H8H_A, 20H4_A, 1GAG_A, 1FPU_A, 2COI_A, 2P4I_A, and for the KRN951, PDBID: 2I0V_A, 2HZN_A, 2OH4_A, 1FGI_A, 1YWN_A, 1FPU_A, 2OFU_A, 2C0I_A, 2H8H_A, 2FGI_A.

Figs. 68 to 71 are diagrams presenting the 3-dimensional structure of the neighborhood of the VEGFR2 binding site. The red ribbon on the protein means an α-helix, and the cyan ribbon means a β-sheet. Fig. 68 presents a set of top ten ligands used in the docking for the ligands that belong to the FP library used in the docking with the neighborhood of the EGFR2 binding site of KRN633, and Fig. 70 similarly presents a set of top ten ligands used in the docking with the neighborhood of the EGFR2 binding site for the ligands that belong to the FP library used in the FP library of KRN951. Fig. 69 presents 10 structures that have been predicted by performing the ChooseLD method ten times for KRN633, together with the 3-dimensional structure in the neighborhood of the binding site of VEGFR2. In the case of using Tc for the degree of similarity to KRN633 among the ligands of the FP library, the maximum value was 0.45. For 10 runs, almost the same structure could be obtained. Fig. 71 presents 10 structures predicted by performing the ChooseLD method ten times for KRN951, together with the 3-dimensional structure in the neighborhood of the binding site of VEGFR2. Eight out of the 10 predicted structures had almost the same structure. In the case of using Tc for the degree of similarity to KRN951 among the ligands of the FP library, the maximum value was 0.29.

(Calculation of successful rate of docking prediction for VEGFR-2)

In order to evaluate the reliability of the predictive complex structures of KRN633 and KRN951, a statistical success rate calculated from 133 sets was calculated using the Tc maximum value of the docking ligands included in the FP library. Fig. 72 is a diagram presenting a graph for the ratio of predictive success when the Tc lower limit value obtained as a result of a docking performance test of the ChooseLD method using the 133 sets, was set at 0.08, and the Tc upper limit value was varied, with the horizontal axis presenting the Tc upper limit value and the vertical axis presenting the success rate.

That is, it is possible to statistically calculate the accuracy for predictive success at the time of applying the ChooseLD method by interpolating the Tc upper limit value in the graph. However, this statistical ratio of predictive success does not take into consideration of the 3-dimensional structure and amino acid sequence of the target protein. Among the ligands included in the FP library used in the docking of KRN633, the ligand with the maximum Tc was 0.45. Thus, when the ratio of predictive success in the case of 0.36 and 0.56 were interpolated, the success rate was 34.7%. Similarly, for KRN951, the ratio of predictive success was interpolated from the ratio of predictive success in the case of 0.24 and 0.36, was 24.3%. From the ratio of predictive success in the 133 sets, GOLD Score STD, which results in the highest ratio of predictive success, was 46.0%, DOCK was 21.1%, and AutoDock was 26.6%. For KRN633, an accuracy of ChooseLD can be predicted better than that of AutoDock but insufficient compared to that of GOLD. It was conceived that for KRN951 an accuracy of ChooseLD can be predicted similar to that of AutoDock.

(Docking with Plasmodium falciparum enoyl acyl carrier protein reductase under condition of existing a low molecular compound (NAD))

Plasmodium falciparum enoyl acyl carrier protein is a pathogenic protein for malarial fever, and is a protein participating in the synthesis of lipids. However, since this pathway for lipid synthesis does not exist in human being, it is conceived that inhibiting the function of this protein leads to the treatment of malarial fever [reference literature (J. Biol. Chem. 2002; 277: 13106-13114)].

Fig. 73 is a diagram showing the 3-dimensional structure of enoyl acyl carrier protein. As shown in Fig. 73, triclosan and the like are included as compounds inhibiting this protein, and X-ray crystallographic structure analysis with a plurality of inhibitors has been achieved [reference literature (J. Biol. Chem. 2002; 277: 13106-13114)], and these inhibitors bind via NAD. When these are used as the FP library, in silico screening was performed for the search of lead compounds of new inhibitors.

Fig. 74 is a diagram presenting the top ten structures on the basis of the FPAScore as a result of performing in silico screening of enoyl acyl carrier proteins, using the MDL Comprehensive Medicinal Chemistry (MDL CMC) Library. The part surrounded by circle on the upper side represents the results obtained by in silico screening, and docking is performed while taking into consideration of the space occupied by NAD represented by the circle in the lower side. In silico screening was also achieved with respect to the MDL Available Chemicals Directory (MDL ACD) Library, but according to the ChooseLD method of the present example, it can be seen that it is possible to perform docking while taking into consideration of low molecular compound that are present in the neighborhood of the binding site of target proteins, such as NAD or $H_2O$.

(Conclusion)

According to the present example, a ligand docking and method of in silico screening, the ChooseLD method based on bioinformatics using a method for optimizing newly defined FPAScores by simulated annealing, were developed.

By performing optimization of the k1 value in the 85 sets, the optimum value was determined to be 4.0 by considering the use in the high-throughput screening or the like. In the case of taking this k1 value, when the fraction capable of predicting the experimental structure at an rmsd of 2.0Å or less for the 133 sets was used as an index, the docking performance of the ChooseLD method of the present example was equal to that of GOLD which performs docking using existing classical physical function, and when the Tc upper limit value was low, the performance was the same as that of DOCK or AutoDock. This implies that the assumption that an FP obtained according to the FP construction method from the ligands included in the FP library established from ligands derived from a family protein, is the coordinates such as having decreasing free energy, was right.

However, since conventionally existing docking software programs are not necessarily be able to search for a structure with the minimum free energy, this means that there is still room for the conventional techniques to undergo improvement. Furthermore, for 133 set, from the viewpoint of the distribution of the PDBIDs successfully predicted, the ChooseLD method was compared with Glide, GOLD and FlexX, to calculate the degree of similarity of the distribution of PDBID based on Tc. As a result, the targets successfully predicted had originality, and it showed a possibility that the accuracy of in silico screening could be increase when the ChooseLD method of the present example and a conventional method are used in combination. Furthermore, as described above, in the second example, it was shown that the k2 and k3 values of the FPAScore are variables that can be optimized in accordance with the target protein, using the kinase domain of EGFR as the target protein. From these results, it was conceived that when the k1, k2 and k3 values of the FPAScore according to the ChooseLD method of the present second example are optimized in accordance with the target protein, in silico screening of more inhibitors and lead compounds can be achieved.

[Third Example]

Third example will be described below. In the third example, in silico screening was performed for the purpose of developing an antagonist and an agonist of AMPKhomoGAMMA1 enzyme.

First, a homology search for the amino acid sequence of AMPKhomoGAMMA1 enzyme was performed, which is a target protein. Thus, modeling of AMPKhomoGAMMA1 was carried out using FAMS Ligand including the following ligands, of which the template is 2V9J_E (E chain of 2V9J) having 99.7% homology. Here, Fig. 75 is a diagram showing the alignment of the amino acid sequence between AMPKhomoGAMMA1 and 2V9J_E. In the result, binding ligands were 3 ligands of 2V8Q_E (AMP_E_1327, AMP_E_1328, AMP_E_1329), 3 ligands of 2V92_E (ATP_E_1327, ATP_E_1328, AMP_E_1329), 3 ligands of 2V9J_E and two magnesium ions (ATP_E_1327, ATP_E_1328, AMP_E_1329, MG_E_1330, MG_E_1331), and 1 ligand of 2QRE_E (AMZ_E_1002).

Subsequently, ligands except 2V9J_E were superimposed to the coordinate system of 2V9J_E by fitting based on CE (structural superimposition between proteins without considering the kind of atoms). Screening of an antagonist and an agonist was attempted on AMP_E_1329 binding site, which does not depend on MG ions among the three ATP (AMP) binging sites of the 2V9J_E model.

Upon performing ChooseLD of the present example, amino acid residues within 18Å from the binding site of AMP_E_1329 was cut out to be used as a receptor model for 2V9J_E. During the CHooseLD screening, the ligands and MG ions except the receptor binding site were included in the receptor as cofactors. Also, as the FPs of ChooseLD of the present example, three adenosines having a phosphate group (PO3) removed from the ligand molecules at the receptor binding site, and 1-(5-amino-4-carboxamide-1H-imidazole-yl)-ribose were used, but the phosphate group moiety is unsuitable for the functional group of the candidate compound. Therefore, phosphate was not used directly as FP, but the relative distance between the pair of His151 and His298 (His150 and His297 in the 2V9J_E of the template protein) that are hydrogen bonded with the oxygen atom of the phosphate group, is calculated, and the structural difference was calculated by GDT_TS (0.5Å, 1.0Å, 1.5Å, and 2.0Å). Thus, a ligand which is a residue pair having a GDT_TS of 70% or more (modifiable) and which exists within 3.0Å (modifiable) from the residue pair, was extracted as HETATM from the 95%NR_PDB. In addition, not only two amino acid residues, but also three amino acid residues can also be assigned.

GDT_TS represents the fraction of residues that can be overlapped to the native structure at XÅ or less. As a result, 1061 ligands could be extracted. For these ligands, collision with the 2V9J_E receptor was checked, and thereby 18 ligands or parts of ligands were added to the FP, so as to perform screening of the CMC [reference literature (Comprehensive Medicinal Chemistry, 2006.1, Elsevier MDL)] database based on total 22 FPs.

Screening result for CMC database were set up as follows: atomic collision between the receptor site and the ligand (2.0Å one atom or less, 2.2Å three atoms or less, 2.4Å five atoms or less), the ligand molecular weight was 200 to 500, LogP of the ligand was -1 to 5, and the number of rings in the ligand, the number of hydrogen donor atoms, and the number of hydrogen acceptor atoms were 0 to 5, respectively. Here, Fig. 76 is a diagram presenting the result list of the CMC pharmaceutical products in which a ligand is bound to the entirety of a receptor.

Here, Fig. 77 is a diagram collectively presenting the states of binding to 2V9J_E receptor, listed from the $1^{st}$ rank to the $10^{th}$ rank. The green ball and stick model represents two HIS residues, and the yellow stick model represents three Adenosines and 1-(5-amino-4-carboxamide-1H-imidazole-yl)-ribose. Among them, 10 pharmaceuticals are docked. In addition to the three adenosines and 1-(5-amino-4-carboxamide-1H-imidazole-yl)-ribose, 27 pharmaceutical compounds of which docking structures with 2V9J_E receptor are obtained by CMC screening may be used as fingerprints, so that total 31 FPs are used to perform screening of ACD (Available Chemicals Directory, 2008.1, Elsevier MDL), and to thereby obtain candidate compounds for the antagonist and agonist of the AMPKhomoGAMMA1 enzyme.

[Other Embodiments]

Although the invention has been described with respect to a specific embodiment for a complete and clear disclosure, the appended claims are not to be thus limited but are to be construed as embodying all modifications and alternative constructions that may occur to one skilled in the art which fairly fall within the basic teaching herein set forth.

For example, in the embodiment, the apparatus for in silico screening 100 performs various processes as a stand-alone device. However, the apparatus for in silico screening 100 can be configured to perform processes in response to request from a client terminal, which is a separate unit, and return the process results to the client terminal.

All the automatic processes explained in the present embodiment can be, entirely or partially, carried out manually. Similarly, all the manual processes explained in the present embodiment can be, entirely or partially, carried out automatically by a known method.

The process procedures, the control procedures, specific names, information including registration data for each process and various parameters such as search conditions, example of display screen, and database construction, which are mentioned in the description and drawings, can be changed as required unless otherwise specified.

The constituent elements of the apparatus for in silico screening 100 are merely conceptual and may not necessarily physically resemble the structures shown in the drawings., that is, the apparatus need not necessarily have the structure that is illustrated.

For example, the process functions performed by each device of the apparatus for in silico screening 100, especially the each process function performed by the control unit 102, can be entirely or partially performed by CPU and a computer program executed by the CPU or by a hardware using wired logic. The external system 200 can be configured as a web server or ASP server or the like. Hardware configurations of the external system 200 can be configured by the information processing device such as generally commercially available personal computer, workstation, and attachment devices thereof. Each functions of the external system 200 can be operated by such as CPU, disc device, memory device, input device, output device, and communication control device in the hardware configurations of the external system 200, and programs or the like that controls these devices.

The computer program, which are recorded on a recording medium to be described later, can be mechanically read by the apparatus for in silico screening 100 on demand. In other words, the storage unit 106 such as read-only memory (ROM) or hard disk (HD) stores the computer program that can work in cooperation with OS to issue commands to the CPU and cause the CPU to perform various processes.

The computer program is first loaded to the random access memory (RAM), and the instruction is executed at a control unit in collaboration with the CPU. Alternatively, the computer program can be stored in any application program server such as the external system 200 connected to the apparatus for in silico screening 100 via the network 300, and can be fully or partially loaded on demand.

The computer-readable "recording medium" on which the computer program can be stored may be a "physical medium of portable type" such as flexible disk, magneto optical (MO) disk, ROM, erasable programmable ROM (EPROM), electrically erasable programmable ROM (EEPROM), compact disk-read-only memory (CD-ROM), digital versatile disk (DVD), or a "communication medium" that stores the computer program for a short term such as communication channels or carrier waves that transmit the computer program over the network 300 such as local area network (LAN), wide area network (WAN), and the Internet.

"Computer program" refers to a data processing method written in any computer language and written method, and can have software codes and binary codes in any format. The "computer program" can be not only a single component but also a dispersed construction in the form of a plurality of modules or libraries, or can perform various functions in collaboration with a different program such as the OS. In the each device according to the embodiment, any known configurations for reading the recording medium, any known process procedure for reading or the following installing the computer program can use any known configuration and procedure.

The storage unit 106 is a memory device such as RAM, ROM, and a fixed disk device such as HD or flexible disk, optical disk, and stores therein various programs, tables, databases, and web page files required for various processes and provided by websites.

The apparatus for in silico screening 100 can also be connected to information processing device such as any existing personal computer, workstation, etc. and can be operated by executing software (that includes computer program, data, etc.) that implements the method according to the present invention in the personal computer or workstation.

The distribution and integration of the apparatus for in silico screening 100 are not limited to those illustrated in the figures. The device as a whole or in parts can be functionally or physically distributed or integrated in an arbitrary unit according to various loads .

INDUSTRIAL APPLICABILITY

The information of which compound would significantly interact with a target macromolecular protein and would be docking to the protein, is an important factor in the development of new pharmaceutical products, and tailor-made medicine means developing pharmaceutical products that conventionally do not work, in correspondence with substitution of at least one amino acid residue. Therefore, the information of compounds bound to target macromolecular proteins is richer in the number of compounds for which experimental determination has been completed, and development of new drugs is more highly accelerated. Thus, the apparatus for in silico screening and the method of in silico screening described in the present invention have high industrial applicability.

## Claims

1. An apparatus for in silico screening for performing screening of candidate compounds that bind to a target protein comprising:

   a storage unit, and a control unit, wherein
   the storage unit includes:

      a compound database produced by extracting a chemical descriptor that includes the atom type and the interatomic bonding rules as the fingerprint of a compound related to a plurality of atoms in the compound, for each of the candidate compounds, and
      the control unit includes:

   a fingerprint of compound producing unit that extracts the fingerprint of compound from a binding compound known to bind to a family protein, which has a 3-dimensional structure that is identical or similar to that of the target protein, along with 3-dimensional coordinates that have been converted into the coordinate system of the target protein, to thereby produce a fingerprint set of binding compound; and
   an optimizing unit that computes, for the candidate compounds stored in the compound database, the 3-dimensional structures of the candidate compounds with respect to the target protein, so that the interaction scores that are based on the root-mean-square deviations of each unit of fingerprint of compound that have been calculated using the 3-dimensional coordinates of the fingerprint set of binding compound as a basic, are optimized.

2. The apparatus for in silico screening according to claim 1,
   the apparatus being connected to a protein database apparatus that stores the respective 3-dimensional structure and amino acid sequence of the proteins bound to a compound, wherein
   the control unit further includes:

   a homology searching unit that searches for the family protein and the binding compound from the protein database system, based on the homology of the target protein with the amino acid sequence, and
   the fingerprint of compound producing unit extracts the fingerprint of compound from the binding compound to bind to the family protein searched by the homology searching unit, along with 3-dimensional coordinates that have been converted into the coordinate system of the target protein to thereby produce the fingerprint set of binding compound.

3. The apparatus for in silico screening according to claim 1, wherein
   the fingerprint of compound producing unit converts the 3-dimensional coordinates of the binding compound that binds to the family protein, into the coordinate system of the target protein, through the superimposition of conformations of the family protein and the target protein, and extracts the fingerprints of compound along with the converted 3-dimensional coordinates of compound, to thereby produce the fingerprint set of binding compound.

4. The apparatus for in silico screening according to claim 1, wherein
   the fingerprint of compound producing unit further includes:

a fingerprint of new compound adding unit that performs superimposition of conformations by referring to another compound that is different from the binding compound, extracts a fingerprint of compound that straddles between the atoms of the binding compound and the atoms of the other compound, and adds the fingerprint of compound to the fingerprint set of binding compound.

**5.** The apparatus for in silico screening according to claim 1, wherein the fingerprint of compound producing unit further includes:

a fingerprint of new compound adding unit that, in regard to the compound that is similar to the binding compound on the basis of the Tanimoto coefficient, interchanges the kind of atom between the atoms of the binding compound and them of the compound, calculates the interaction energy with respect to the target protein to thereby produce a fingerprint of compound that is more stable in terms of local energy than the fingerprint of compound from the binding compound, and adding the produced fingerprint of compound to the fingerprint set of binding compound.

**6.** The apparatus for in silico screening according to claim 1, wherein the binding compound is a compound that is predicted by a known docking algorithm to have a stable conformation with respect to the target protein.

**7.** The apparatus for in silico screening according to claim 1, wherein the optimizing unit further includes:

an interaction score calculating unit that calculates the interaction score, based on a function that takes into consideration of not only the root-mean-square deviation for a unit of fingerprint of compound but also the collision state of the candidate compound with the target protein, the existential rate of the candidate compound in the region of interaction of the target protein, and the fraction of direct interaction of the candidate compound with the target protein.

**8.** The apparatus for in silico screening according to claim 1, wherein the optimizing unit optimizes the interaction score by determining the interaction score based on the Metropolis method, and modifying, increasing or decreasing the basal fingerprint of compound from the candidate compound according to the results of determination.

**9.** The apparatus for in silico screening according to claim 1, wherein the optimizing unit further includes:

a structure transforming unit that repeatedly changes the conformation of the candidate compound in the process of optimizing the interaction score, and repeatedly translates or rotates the candidate compound as a rigid body, for each of the conformations of the candidate compound based on a simulated annealing method, and
the optimizing unit calculates the interaction score of the candidate compound for each of the conformations translated or rotated by the conformation transforming unit.

**10.** The apparatus for in silico screening according to claim 1, wherein the optimizing unit calculates the interaction score based on the following mathematical formula (1):

$$\begin{aligned} FPAScore &= F(aligned_fp, fp_rmsd, molecule) \\ &= BaseScore(aligned_fp, fp_rmsd) \\ &\quad \times fp_volume(molecule) \\ &\quad \times fp_contact_surface(molecule) \end{aligned} \qquad (1)$$

wherein the FPAScore represents the interaction score; the F(aligned_fp,fp_rmsd, molecule) is a function using, as variables, the degree of alignment and the root-mean-square deviation of the unit of fingerprint of compound between the binding compound and the candidate compound, and the 3-dimensional structure of the candidate compound with respect to the target protein; the BaseScore(aligned_fp,fp_rmsd) is an index representing the degree of consistency and crowded degree of the unit of fingerprint of compound; the fp_volume(molecule) is an index representing the fraction occupied by the candidate compound in a space formed by the 3-dimensional coordinates of the fingerprint set of binding compound, and the collision state with the target protein; and the fp_contact_surface(molecule) is an index representing the contacting degree of the candidate compound with the target protein, and the degree of attribution to the 3-dimensional coordinates of the fingerprint set of binding compound.

**11.** The apparatus for in silico screening according to claim 10, wherein
the BaseScore(aligned_fp,fp_rmsd) in the mathematical formula (1) is calculated based on the following mathematical formula (2):

$$BaseScore(aligned_fp, fp_rmsd) = \frac{RawScore(aligned_fp)}{1 + \ln(fp_rmsd^{k1} + 1)} \quad (2)$$

wherein the RawScore(aligned_fp) is an index based on the number of atoms in the fingerprints of compound that are aligned between the binding compound and the candidate compound; and the fp_rmsd is the root-mean-square deviation;
the fp_volume(molecule) is calculated based on the following mathematical formula (6):

$$fp_volume(molecule) = \ln\frac{1.0 + nafp^{k2}}{1.0 + nap^{k3}} \quad (6)$$

wherein the nafp is the number of lattice points occupied by the 3-dimensional coordinates of the candidate compound in a region of proper grid based on the 3-dimensional coordinates of the fingerprint set of binding compound; the nap is the number of lattice points to which the 3-dimensional coordinates of the candidate compound fall into the region of proper grid of the atoms in the 3-dimensional structure of the target protein; and the k2 and k3 are arbitrary constants; and
the fp_contact_surface(molecule) is calculated based on the following mathematical formula (7):

$$fp_contact_surface(molecule) = \frac{\sum_{i=1}^{n} density_of_atom(atom(i))}{total_density_of_atom(molecule)} \quad (7)$$

wherein n is the number of atoms of the candidate compound; atom(i) is the 3-dimensional coordinates of the $i$th atom in the candidate compound; the density_of_atom(atom(i)) is a function that reciprocates the sum of the number of atoms in the target protein contacting with the atoms of the fingerprint of compound at a predetermined distance and the number of atoms in the binding compound falling into the same lattice points of the fingerprint of compound when the 3-dimensional coordinates of the atom belong to the fingerprint of compound of the fingerprint set of binding compound; and the total_density_of_atom(molecule) is the number obtained by reordering the distribution of the density_of_atom in a descending order, and summing the numeric values in order as many times as the number of atoms in the candidate compound.

**12.** An method of in silico screening executed by an apparatus for in silico screening for performing screening of candidate compounds that bind to a target protein including:

a storage unit and a control unit, wherein
the storage unit includes:

a compound database produced by extracting a chemical descriptor that includes the atom type and the interatomic bonding rules as the fingerprint of a compound related to a plurality of atoms in the compound, for each of the candidate compounds,
the method comprising:

a fingerprint of compound producing step of extracting the fingerprint of compound of a binding compound known to bind to a family protein, which has a 3-dimensional structure that is identical or similar to that of the target protein, along with 3-dimensional coordinates that have been converted into the coordinate system of the target protein, to thereby produce a fingerprint set of binding compound; and

an optimizing step of computing, for the candidate compounds stored in the compound database, the 3-dimensional structures of the candidate compounds with respect to the target protein, so that the interaction scores that are based on the root-mean-square deviations of each unit of fingerprint of compound that have been calculated using the 3-dimensional coordinates of the fingerprint set of binding compound as a basic, are optimized,

wherein the steps are executed by the control unit.

FIG.1

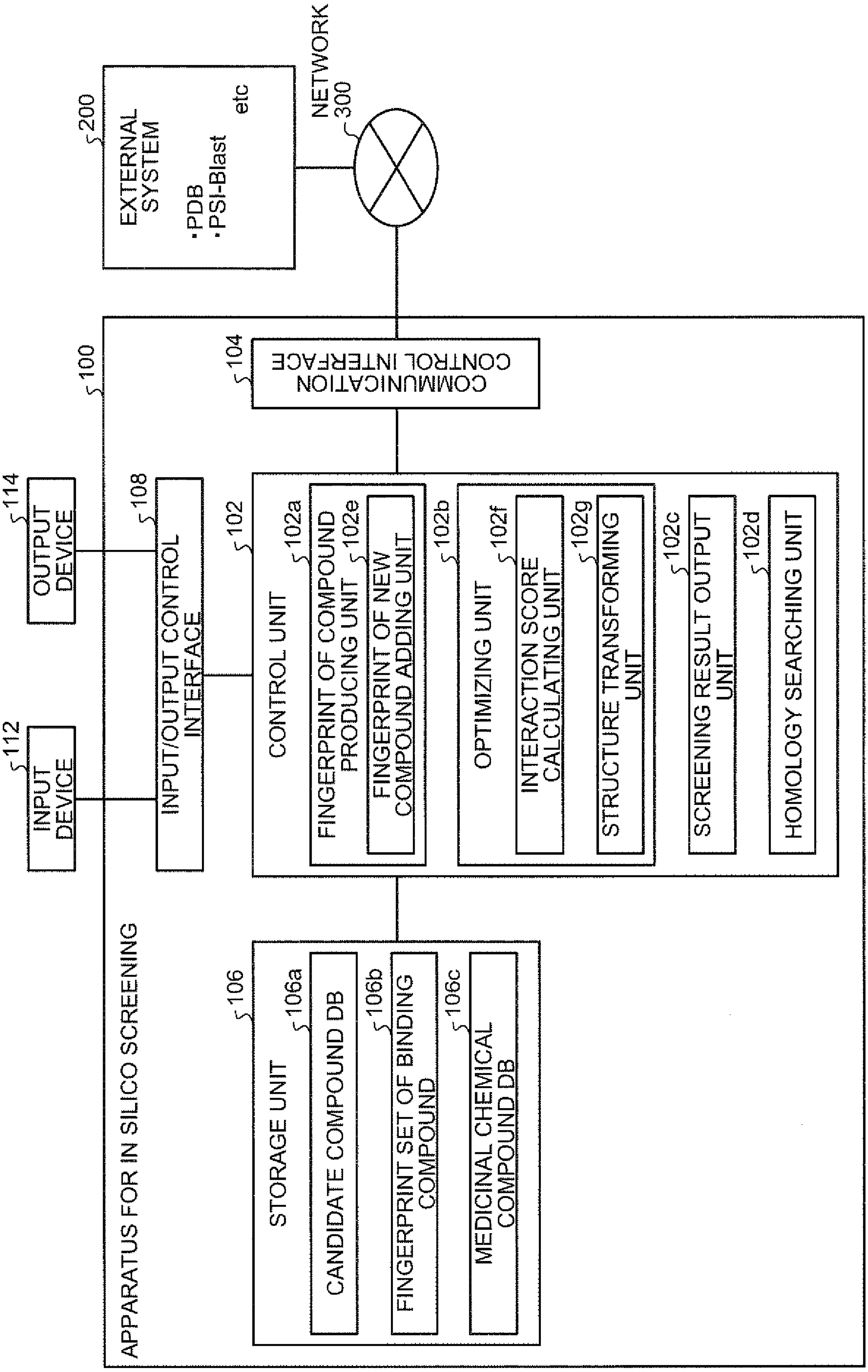

200
EXTERNAL SYSTEM
·PDB
·PSI-Blast
etc
NETWORK
300
100
APPARATUS FOR IN SILICO SCREENING
104
COMMUNICATION CONTROL INTERFACE
112
INPUT DEVICE
114
OUTPUT DEVICE
108
INPUT/OUTPUT CONTROL INTERFACE
102
CONTROL UNIT
102a
FINGERPRINT OF COMPOUND PRODUCING UNIT
102e
FINGERPRINT OF NEW COMPOUND ADDING UNIT
102b
OPTIMIZING UNIT
102f
INTERACTION SCORE CALCULATING UNIT
102g
STRUCTURE TRANSFORMING UNIT
102c
SCREENING RESULT OUTPUT UNIT
102d
HOMOLOGY SEARCHING UNIT
106
STORAGE UNIT
106a
CANDIDATE COMPOUND DB
106b
FINGERPRINT SET OF BINDING COMPOUND
106c
MEDICINAL CHEMICAL COMPOUND DB

FIG.2

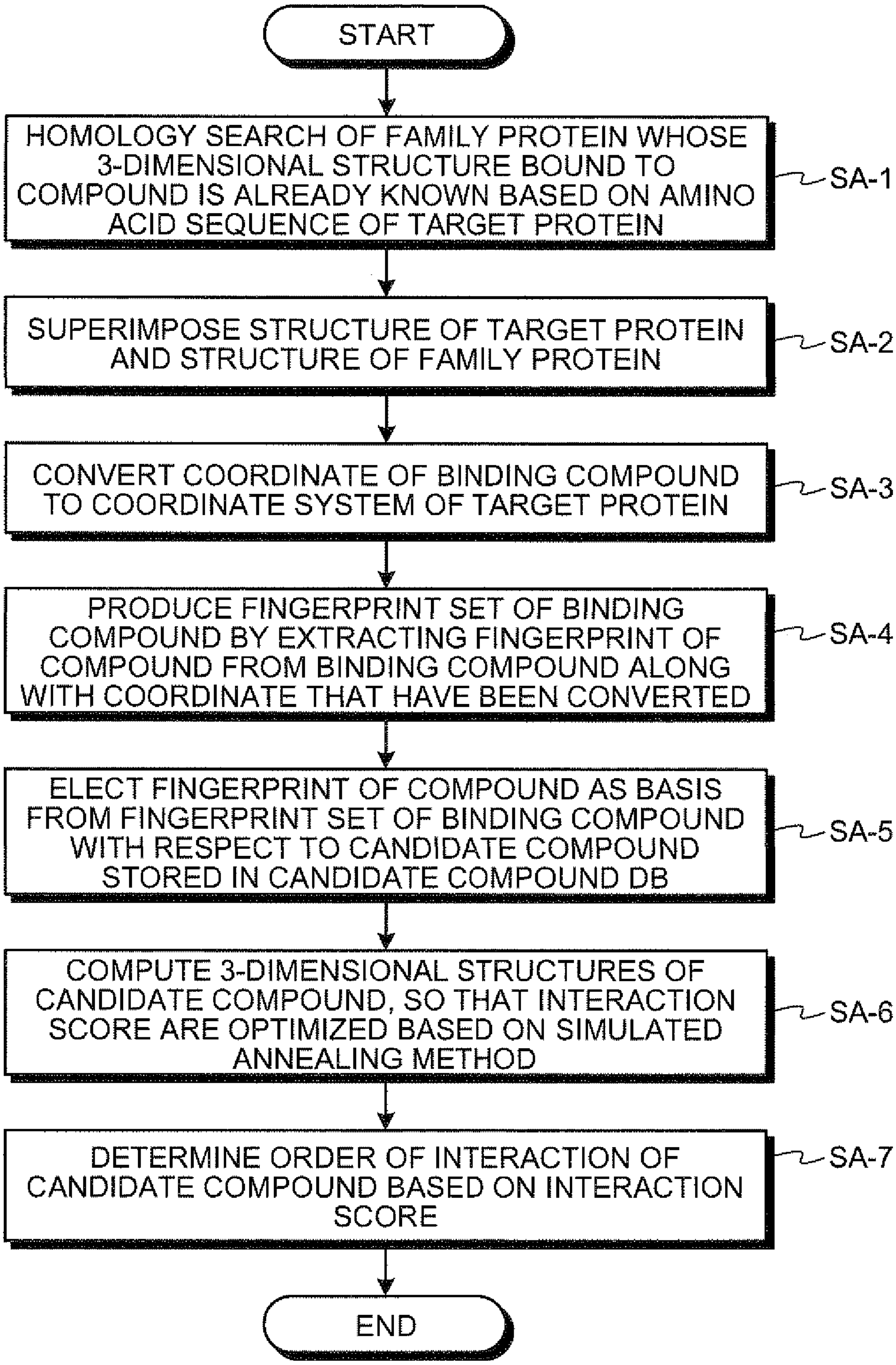
START
HOMOLOGY SEARCH OF FAMILY PROTEIN WHOSE 3-DIMENSIONAL STRUCTURE BOUND TO COMPOUND IS ALREADY KNOWN BASED ON AMINO ACID SEQUENCE OF TARGET PROTEIN
SA-1
SUPERIMPOSE STRUCTURE OF TARGET PROTEIN AND STRUCTURE OF FAMILY PROTEIN
SA-2
CONVERT COORDINATE OF BINDING COMPOUND TO COORDINATE SYSTEM OF TARGET PROTEIN
SA-3
PRODUCE FINGERPRINT SET OF BINDING COMPOUND BY EXTRACTING FINGERPRINT OF COMPOUND FROM BINDING COMPOUND ALONG WITH COORDINATE THAT HAVE BEEN CONVERTED
SA-4
ELECT FINGERPRINT OF COMPOUND AS BASIS FROM FINGERPRINT SET OF BINDING COMPOUND WITH RESPECT TO CANDIDATE COMPOUND STORED IN CANDIDATE COMPOUND DB
SA-5
COMPUTE 3-DIMENSIONAL STRUCTURES OF CANDIDATE COMPOUND, SO THAT INTERACTION SCORE ARE OPTIMIZED BASED ON SIMULATED ANNEALING METHOD
SA-6
DETERMINE ORDER OF INTERACTION OF CANDIDATE COMPOUND BASED ON INTERACTION SCORE
SA-7
END

FIG.3

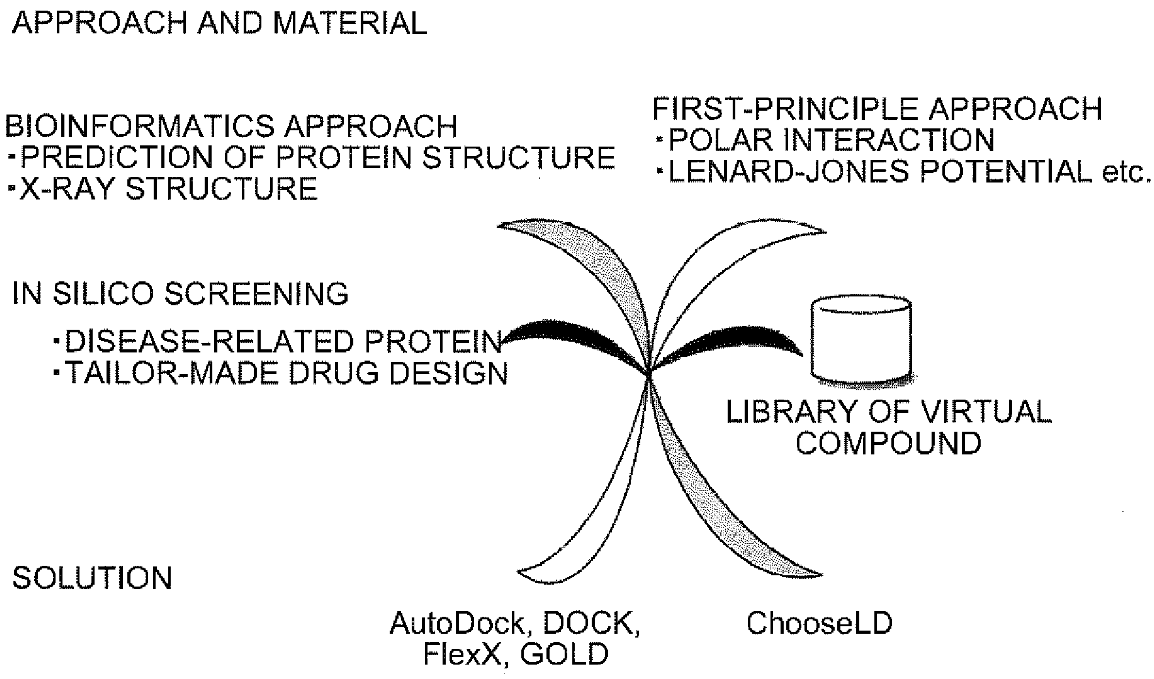
APPROACH AND MATERIAL
BIOINFORMATICS APPROACH
·PREDICTION OF PROTEIN STRUCTURE
·X-RAY STRUCTURE
FIRST-PRINCIPLE APPROACH
·POLAR INTERACTION
·LENARD-JONES POTENTIAL etc.
IN SILICO SCREENING
·DISEASE-RELATED PROTEIN
·TAILOR-MADE DRUG DESIGN
LIBRARY OF VIRTUAL COMPOUND
SOLUTION
AutoDock, DOCK, FlexX, GOLD
ChooseLD

FIG.4

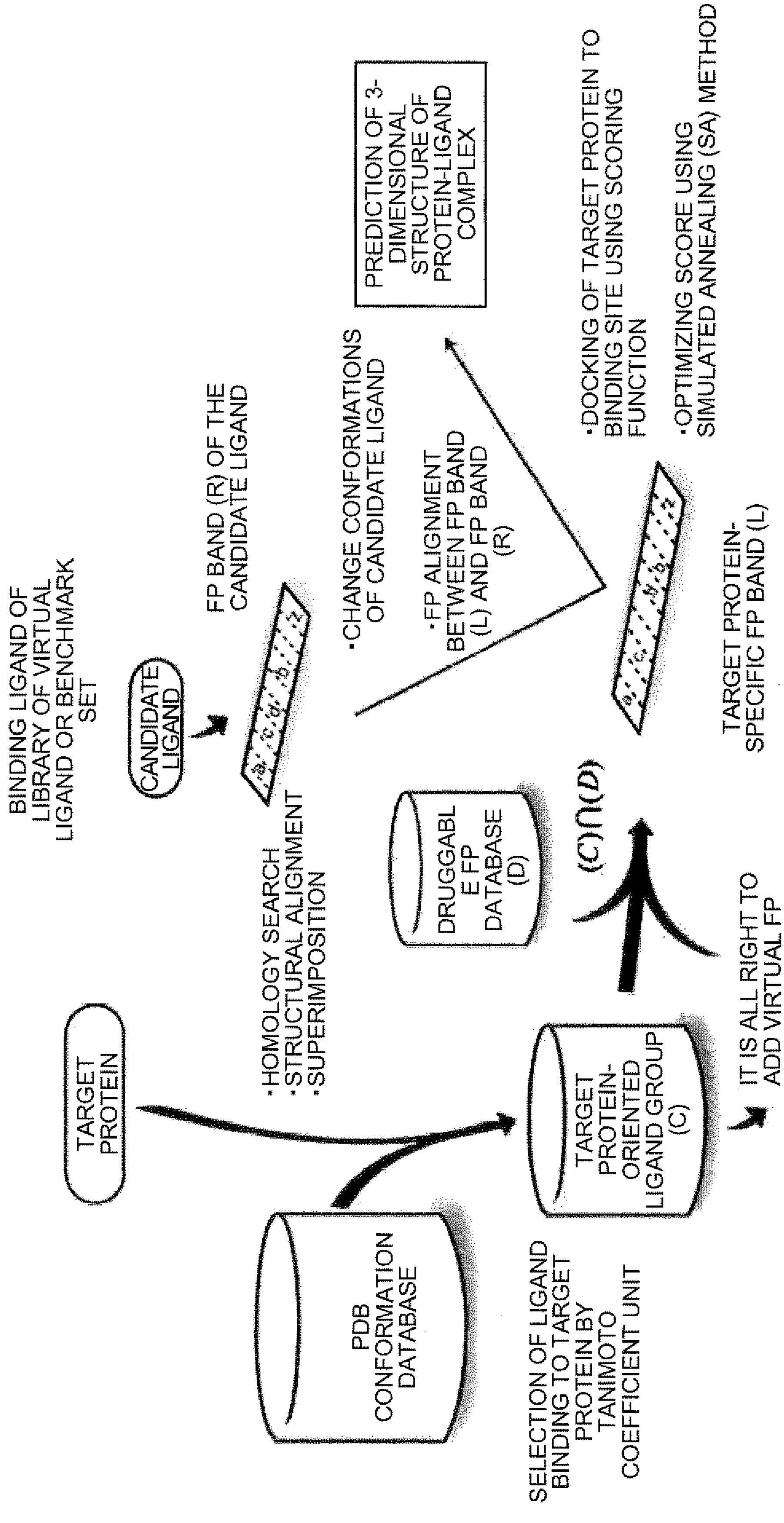
BINDING LIGAND OF LIBRARY OF VIRTUAL LIGAND OR BENCHMARK SET
TARGET PROTEIN
CANDIDATE LIGAND
FP BAND (R) OF THE CANDIDATE LIGAND
·HOMOLOGY SEARCH
·STRUCTURAL ALIGNMENT
·SUPERIMPOSITION
PDB CONFORMATION DATABASE
·CHANGE CONFORMATIONS OF CANDIDATE LIGAND
·FP ALIGNMENT BETWEEN FP BAND (L) AND FP BAND (R)
PREDICTION OF 3-DIMENSIONAL STRUCTURE OF PROTEIN-LIGAND COMPLEX
DRUGGABLE FP DATABASE (D)
(C)∩(D)
TARGET PROTEIN-ORIENTED LIGAND GROUP (C)
SELECTION OF LIGAND BINDING TO TARGET PROTEIN BY TANIMOTO COEFFICIENT UNIT
IT IS ALL RIGHT TO ADD VIRTUAL FP
TARGET PROTEIN-SPECIFIC FP BAND (L)
·DOCKING OF TARGET PROTEIN TO BINDING SITE USING SCORING FUNCTION
·OPTIMIZING SCORE USING SIMULATED ANNEALING (SA) METHOD

FIG.5

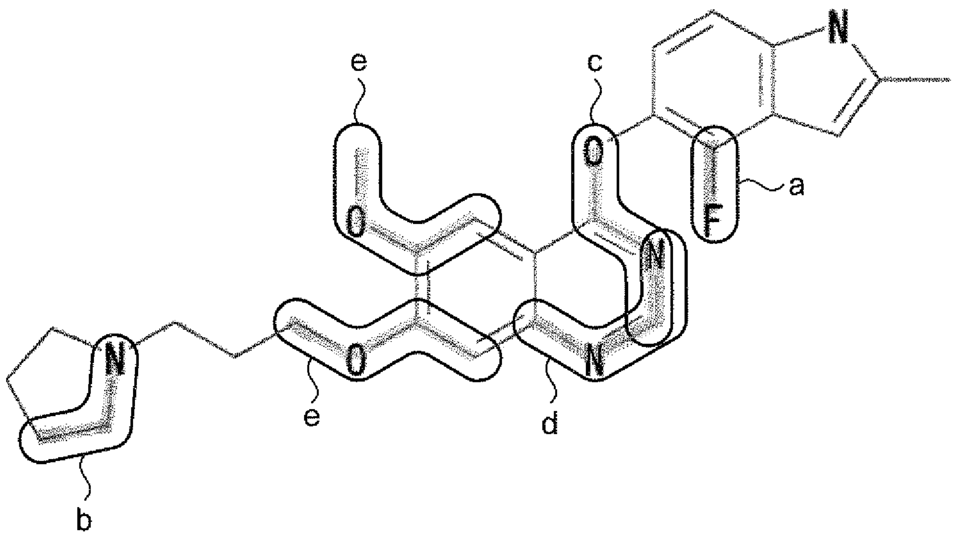

FIG.6

| CHARACTER STRING | NAME | CHARACTER STRING | NAME |
|---|---|---|---|
| H | hydrogen | Si | silane |
| LP | lone pair | P.3 | phosphorous sp3 |
| Li | lithium | S.3 | sulfur sp3 |
| B | boron | S.2 | sulfur sp2 |
| C.3 | carbon sp3 | S.o | sulfoxide sulfur |
| C.2 | carbon sp2 | S.o2 | sulfone sulfur |
| C.1 | carbon sp1 | Cl | chlorine |
| C.ar | carbon aromatic | K | po tassium |
| C.cat | carbocation | Ca | calcium |
| C.co2 | carbon sp2 carbonyl | Mn | manganese |
| N.3 | nitrogen sp3 | Fe | iron |
| N.2 | nitrogen sp2 | Co | cobalt |
| N.1 | nitrogen sp | Ni | nickel |
| N.ar | nitrogen aromatic | Zn | zinc |
| N.am | nitrogen amide | Ge | germanium |
| N.pl3 | nitrogen trigonal pl a | Se | selenium |
| N.4 | nitrogen sp3 positive | Br | bromine |
| O.3 | oxygen sp3 | Sr | strontium |
| O.2 | oxygen sp2 | Sn | tin |
| O.co2 | oxygen in carboxylate | Te | tellurium |
| O.hoh | oxygen water | I | iodine |
| F | fluorine | Ba | barium |
| Na | sodium | Pb | lead (IV) |
| Mg | magnesium | | |

FIG.7

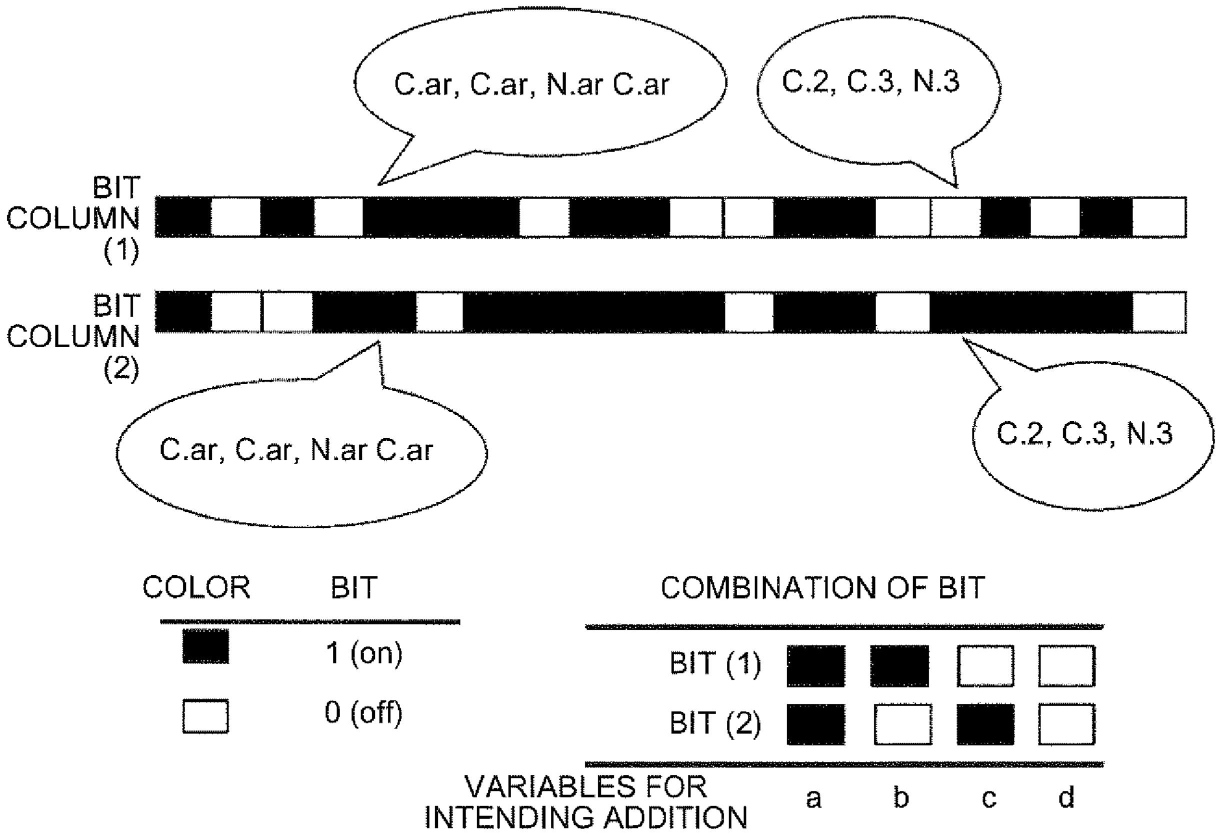
C.ar, C.ar, N.ar C.ar
C.2, C.3, N.3
BIT COLUMN (1)
BIT COLUMN (2)
C.ar, C.ar, N.ar C.ar
C.2, C.3, N.3
COLOR
BIT
1 (on)
0 (off)
COMBINATION OF BIT
BIT (1)
BIT (2)
VARIABLES FOR INTENDING ADDITION
a
b
c
d

FIG.8

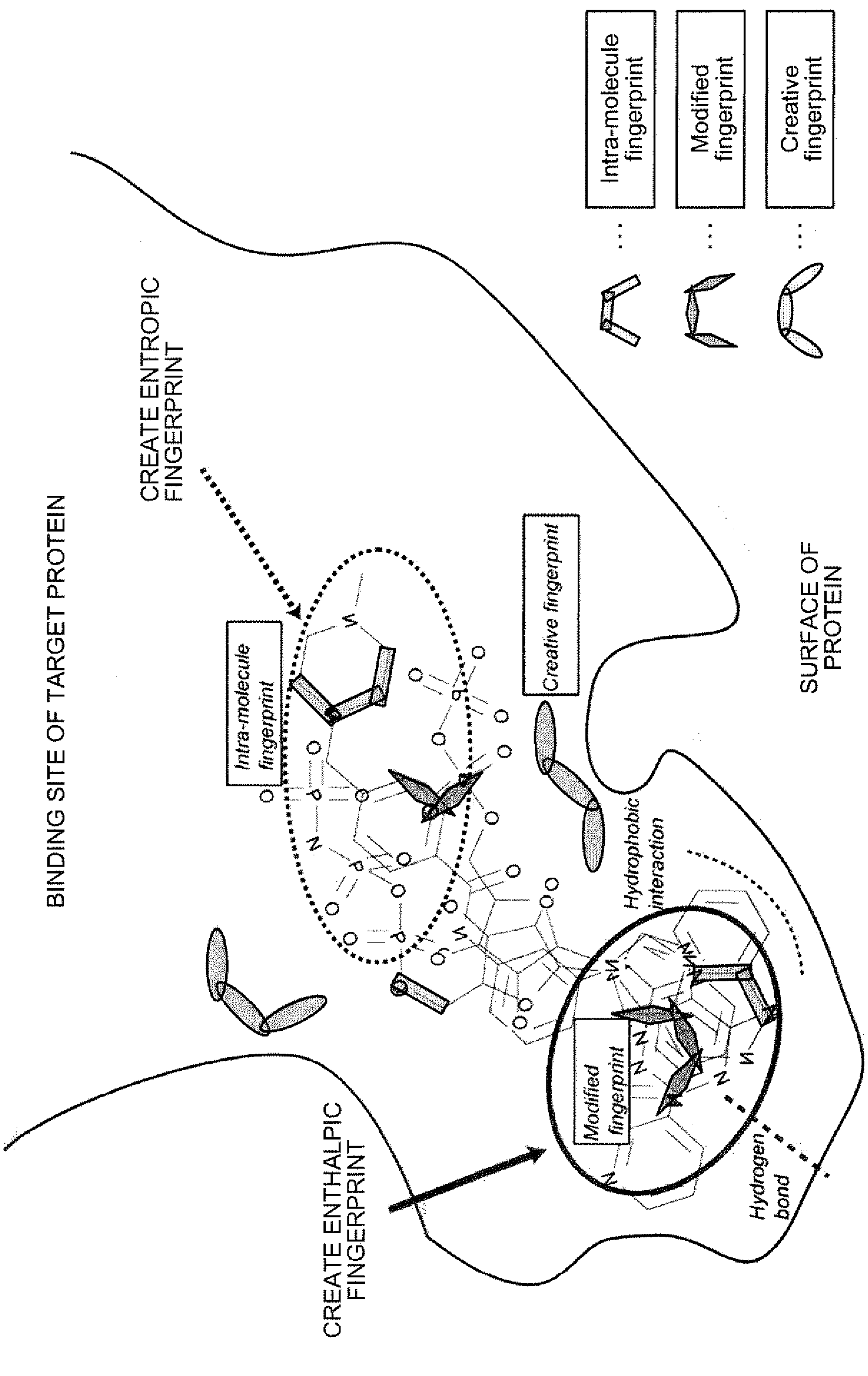
BINDING SITE OF TARGET PROTEIN
CREATE ENTROPIC FINGERPRINT
CREATE ENTHALPIC FINGERPRINT
Intra-molecule fingerprint
Creative fingerprint
Modified fingerprint
Hydrophobic interaction
Hydrogen bond
SURFACE OF PROTEIN
Intra-molecule fingerprint
Modified fingerprint
Creative fingerprint

FIG.9

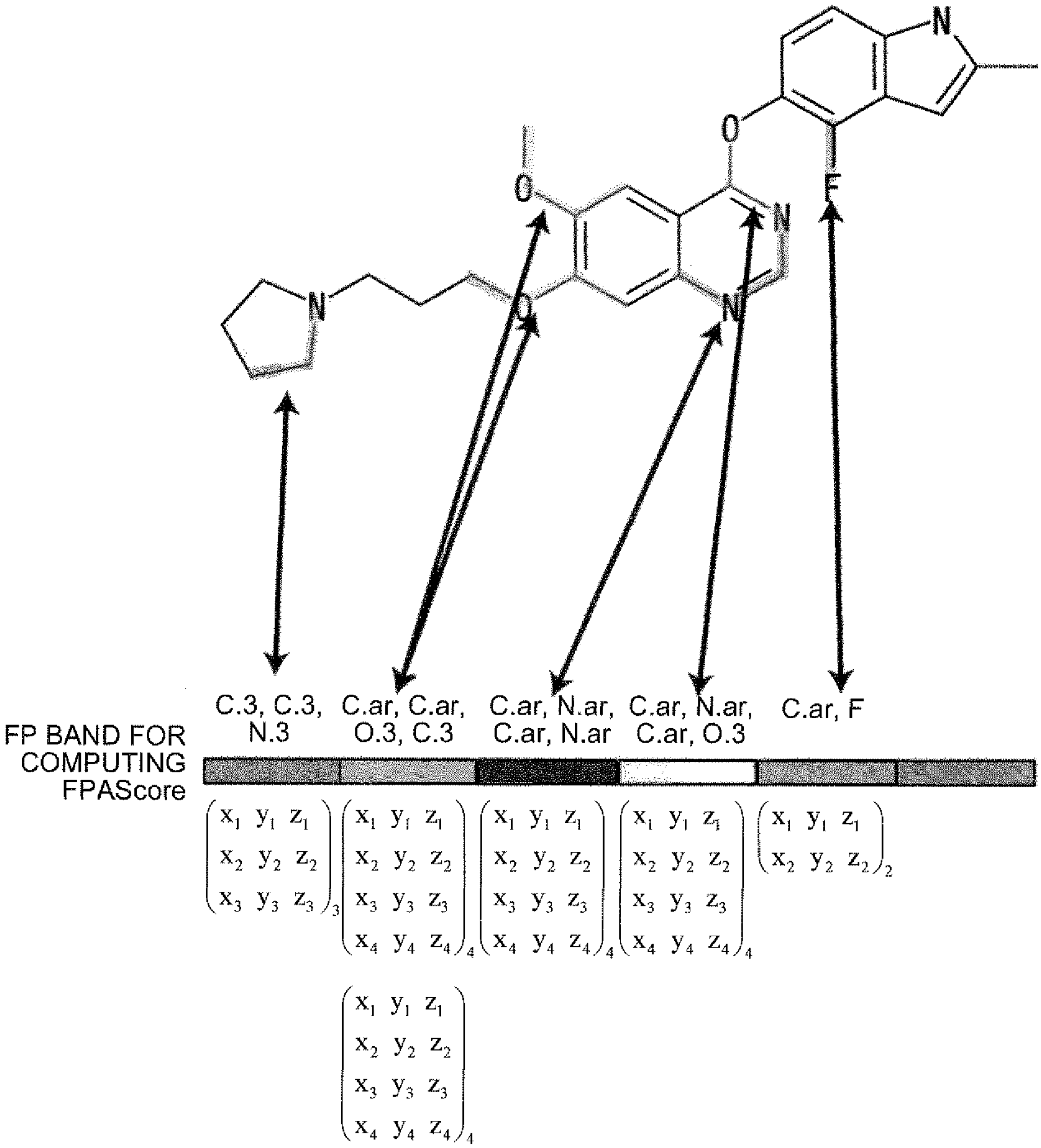
C.3, C.3, N.3
C.ar, C.ar, O.3, C.3
C.ar, N.ar, C.ar, N.ar
C.ar, N.ar, C.ar, O.3
C.ar, F
FP BAND FOR COMPUTING FPAScore

FIG.10

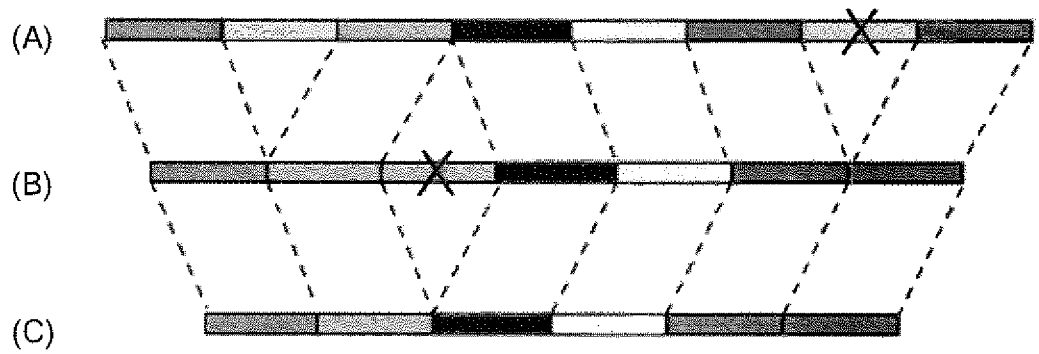

FIG.11

TWO VECTORS ARE REGISTERED IN ONE FP

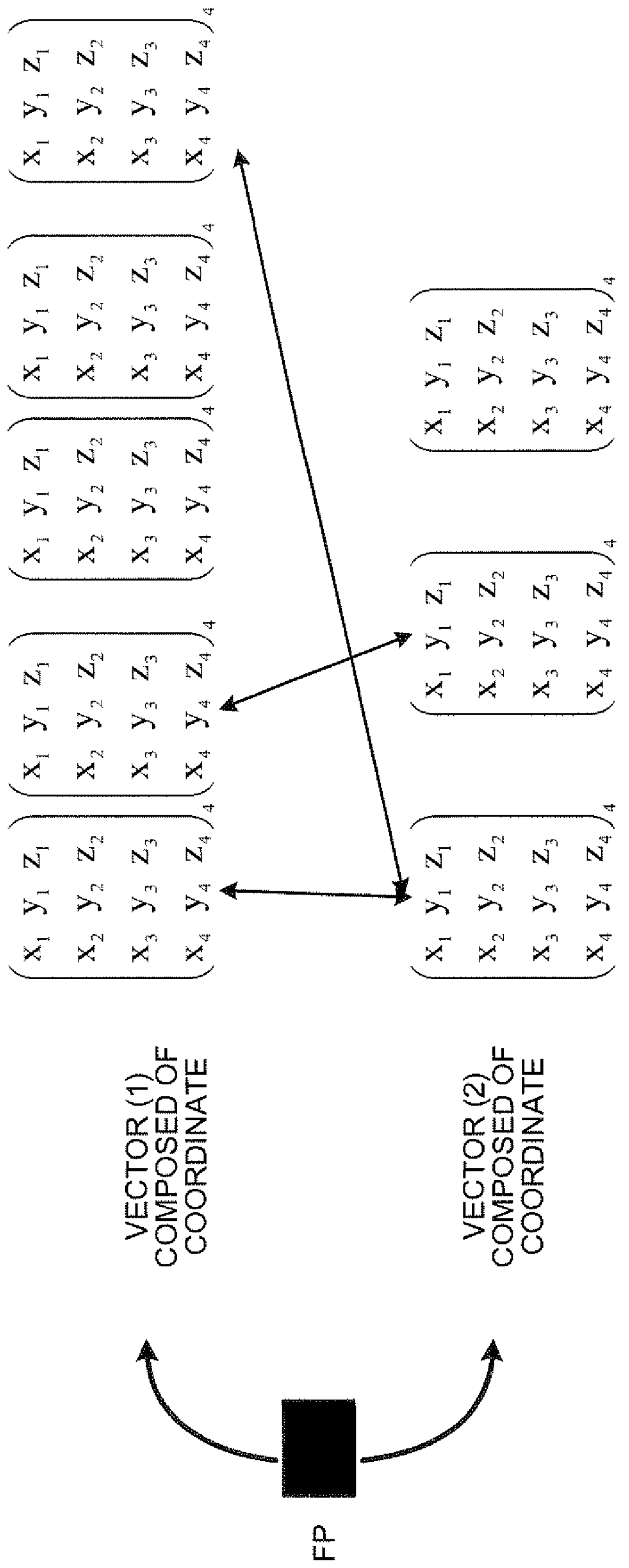

FIG.12

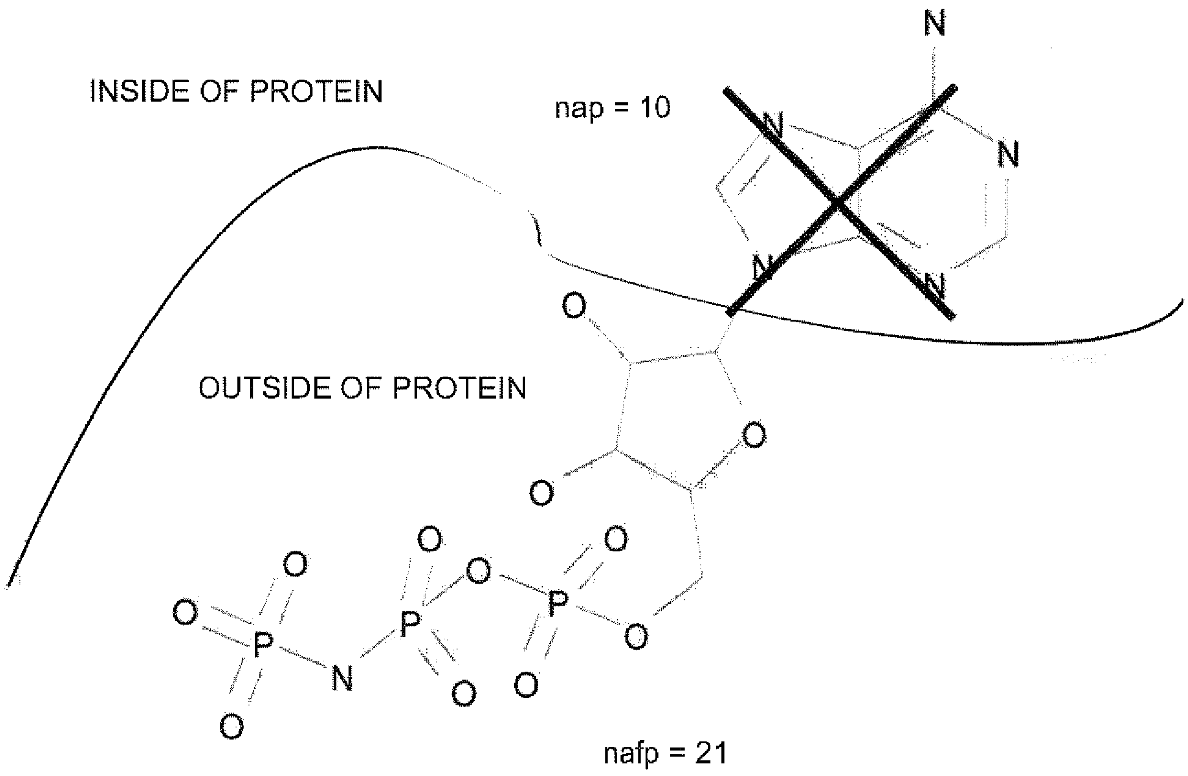

FIG.13

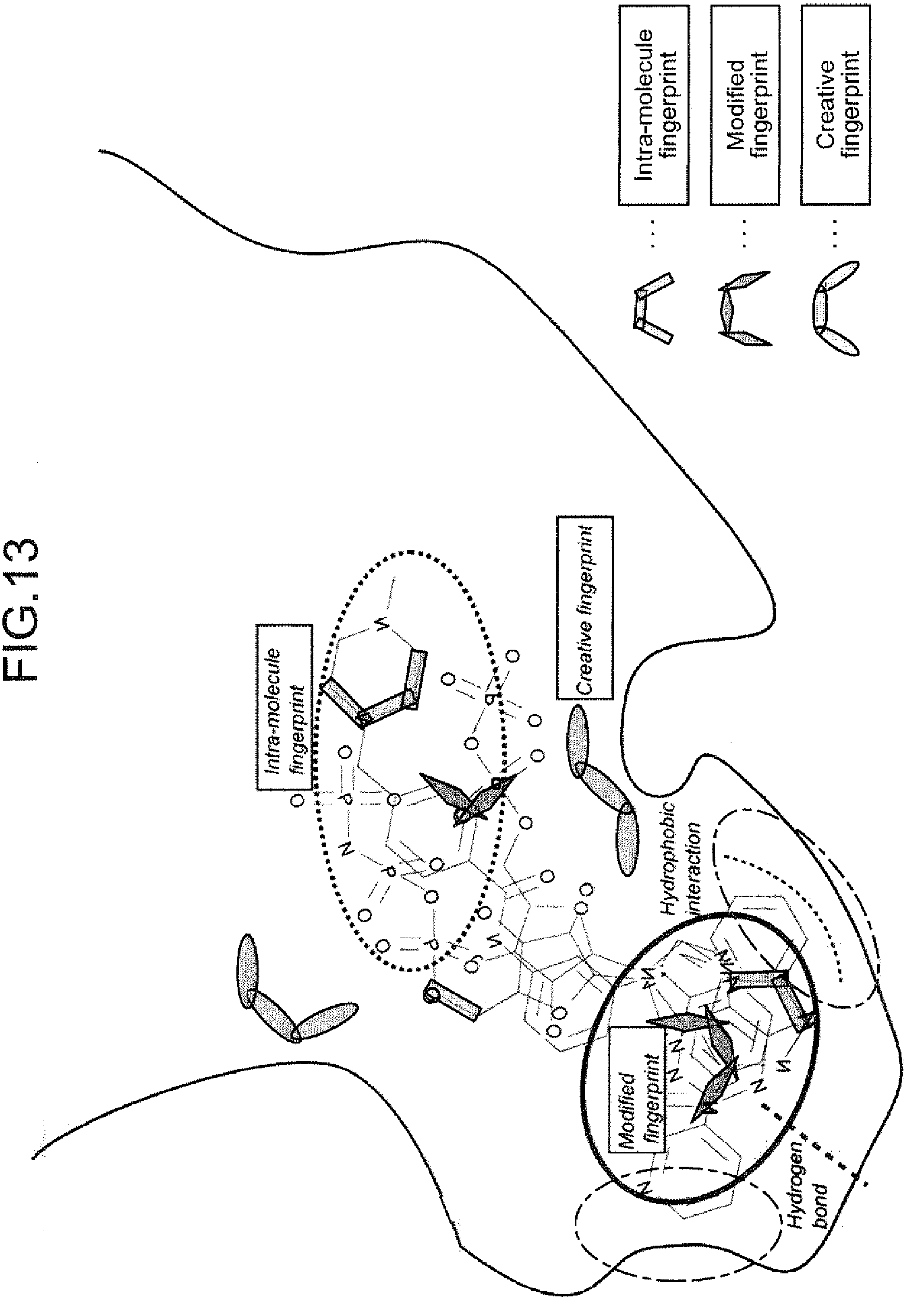
Intra-molecule fingerprint
Creative fingerprint
Hydrophobic interaction
Modified fingerprint
Hydrogen bond
Intra-molecule fingerprint
Modified fingerprint
Creative fingerprint

FIG.14

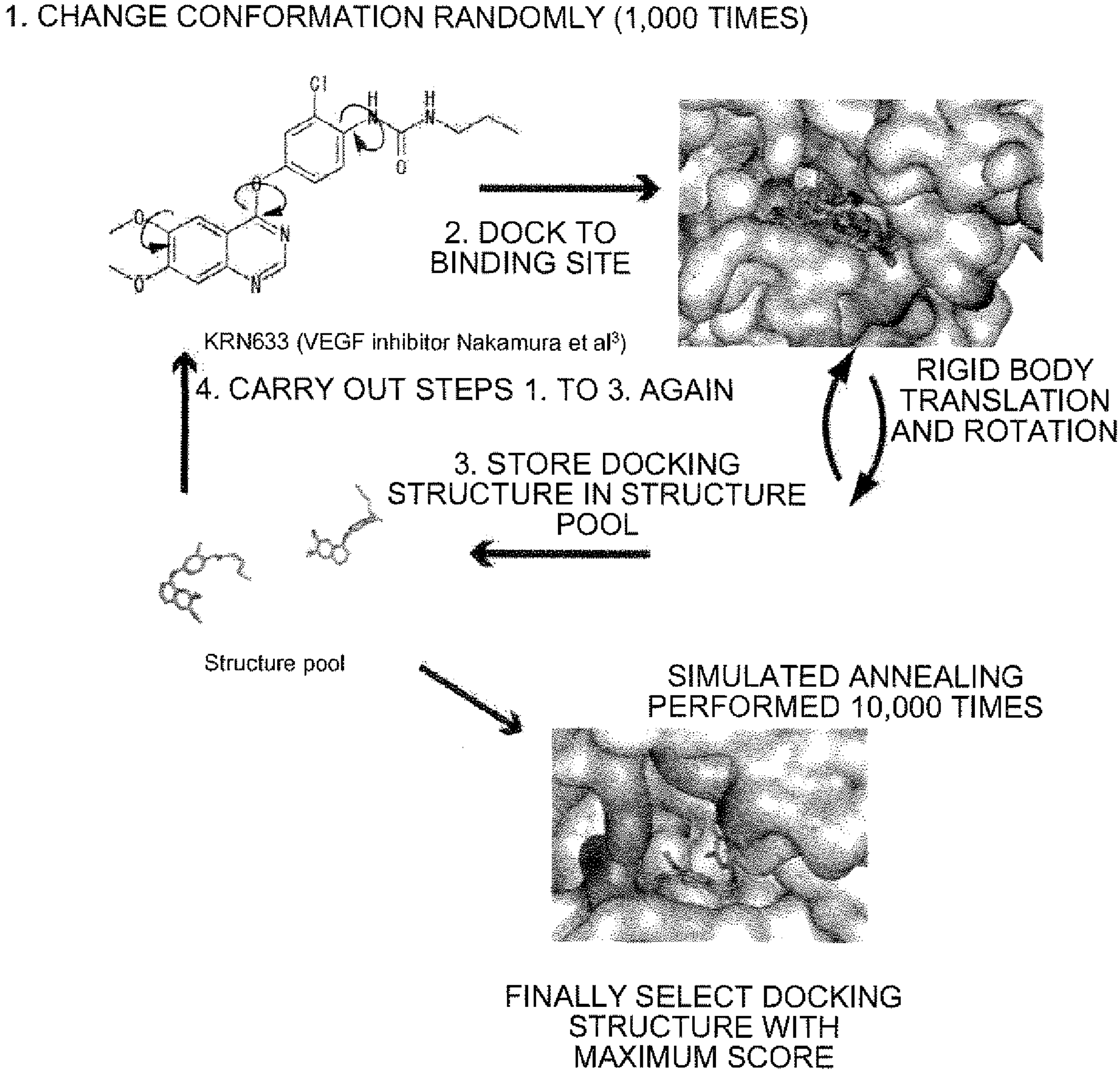
1. CHANGE CONFORMATION RANDOMLY (1,000 TIMES)
2. DOCK TO BINDING SITE
KRN633 (VEGF inhibitor Nakamura et al[3])
4. CARRY OUT STEPS 1. TO 3. AGAIN
RIGID BODY TRANSLATION AND ROTATION
3. STORE DOCKING STRUCTURE IN STRUCTURE POOL
Structure pool
SIMULATED ANNEALING PERFORMED 10,000 TIMES
FINALLY SELECT DOCKING STRUCTURE WITH MAXIMUM SCORE

FIG.15

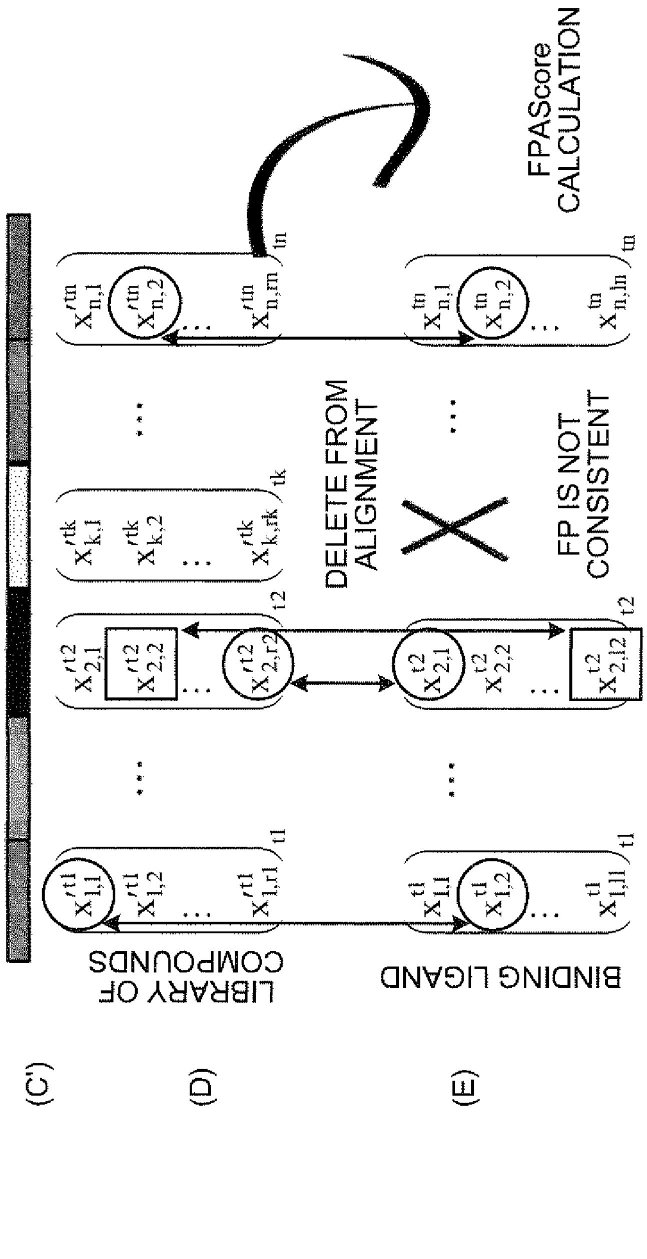


$$x^{t}_{a,b} = \begin{pmatrix} x^{1}_{a,b} & y^{1}_{a,b} & z^{1}_{a,b} \\ & \vdots & \\ x^{m}_{a,b} & y^{m}_{a,b} & z^{m}_{a,b} \\ & \vdots & \\ x^{t}_{a,b} & y^{t}_{a,b} & z^{t}_{a,b} \end{pmatrix}$$

FIG.16

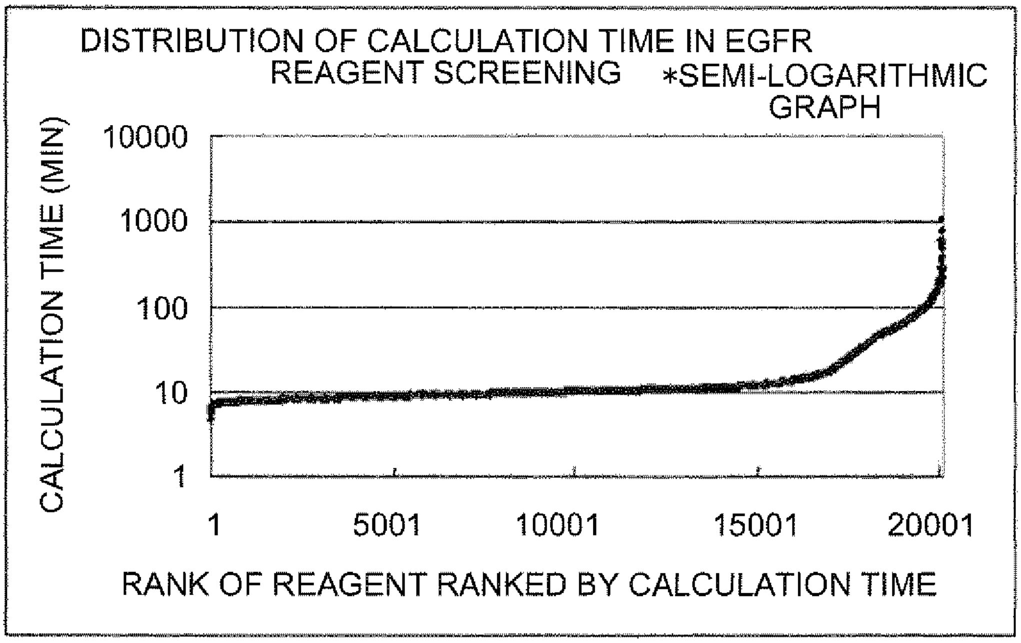


FIG.17

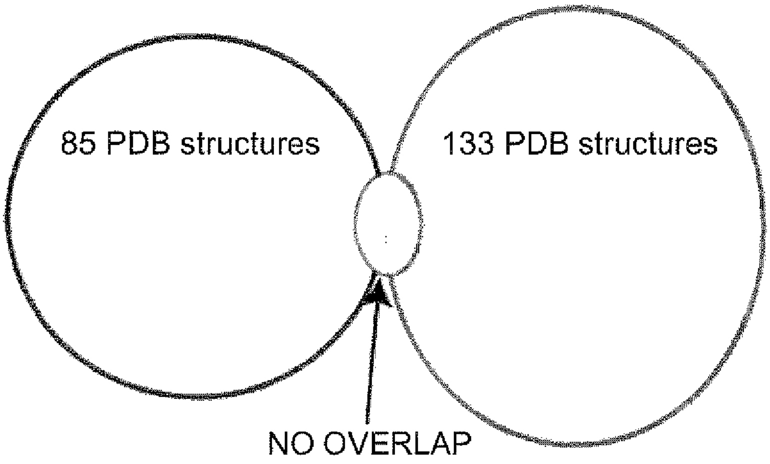


Total: 218 proteins.

FIG.18

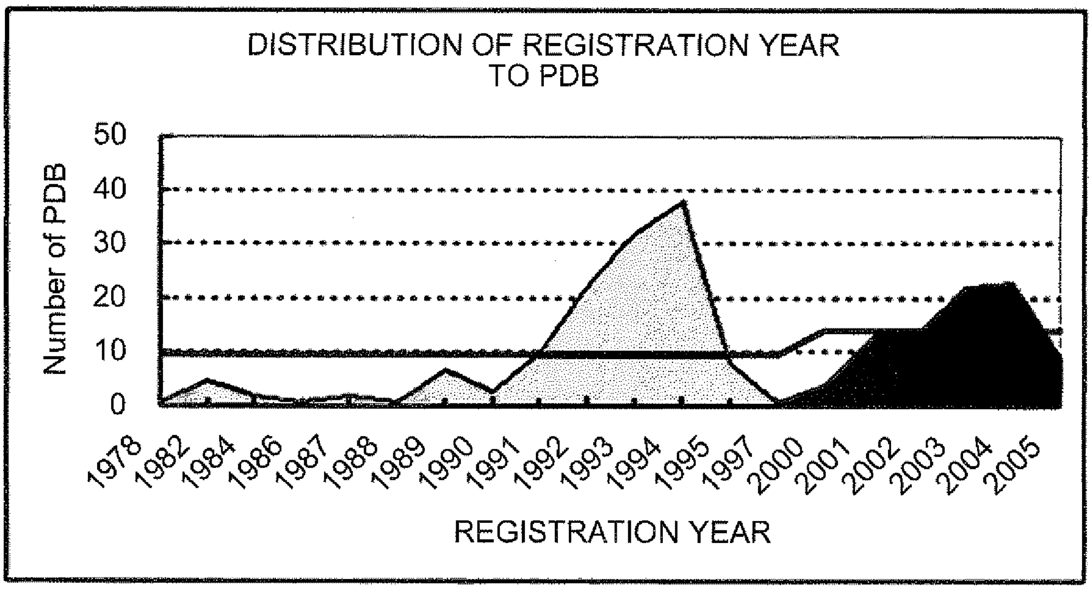


FIG.19

| r.m.s. | Total | Good | Close | Errors | Wrong |
|---|---|---|---|---|---|
| ≤0.5 | 8 | 8 | 0 | 0 | 0 |
| >0.5, <1.0 | 27 | 24 | 3 | 0 | 0 |
| >1.0, <1.5 | 20 | 7 | 13 | 0 | 0 |
| >1.5, <2.0 | 11 | 2 | 9 | 0 | 0 |
| >2.0, <2.5 | 2 | 0 | 2 | 0 | 0 |
| >2.5, <3.0 | 3 | 0 | 2 | 1 | 0 |
| >3.0 | 28 | 0 | 1 | 8 | 19 |

FIG.20

| Tc Range | | k1 1.0 | 2.0 | 3.0 | 4.0 | 5.0 | 6.0 |
|---|---|---|---|---|---|---|---|
| | 0.56-0.08 | 46.0 | 54.6 | 57.6 | 58.9 | 56.3 | 58.6 |
| | 0.76-0.08 | 50.0 | 61.0 | 60.7 | 62.1 | 59.4 | 62.1 |
| | 0.96-0.08 | 55.6 | 62.1 | 64.4 | 65.2 | 65.8 | 64.8 |
| | average (%) | 50.5 | 59.2 | 60.9 | 62.1 | 60.5 | 61.8 |

FIG.21

| | Tc Range | | |
|---|---|---|---|
| | 0.56-0.08 | 0.76-0.08 | 0.96-0.08 |
| 1 | 58.9 | 62.1 | 65.2 |
| 2 | 66.0 | 68.7 | 70.6 |
| 3 | 69.0 | 72.0 | 73.9 |
| 4 | 71.4 | 73.9 | 76.2 |
| 5 | 73.4 | 75.7 | 78.2 |
| 6 | 74.3 | 76.2 | 79.8 |
| 7 | 75.1 | 78.1 | 80.4 |
| 8 | 75.9 | 78.8 | 81.3 |
| 9 | 76.5 | 79.4 | 82.4 |
| 10 | 76.9 | 80.4 | 82.9 |

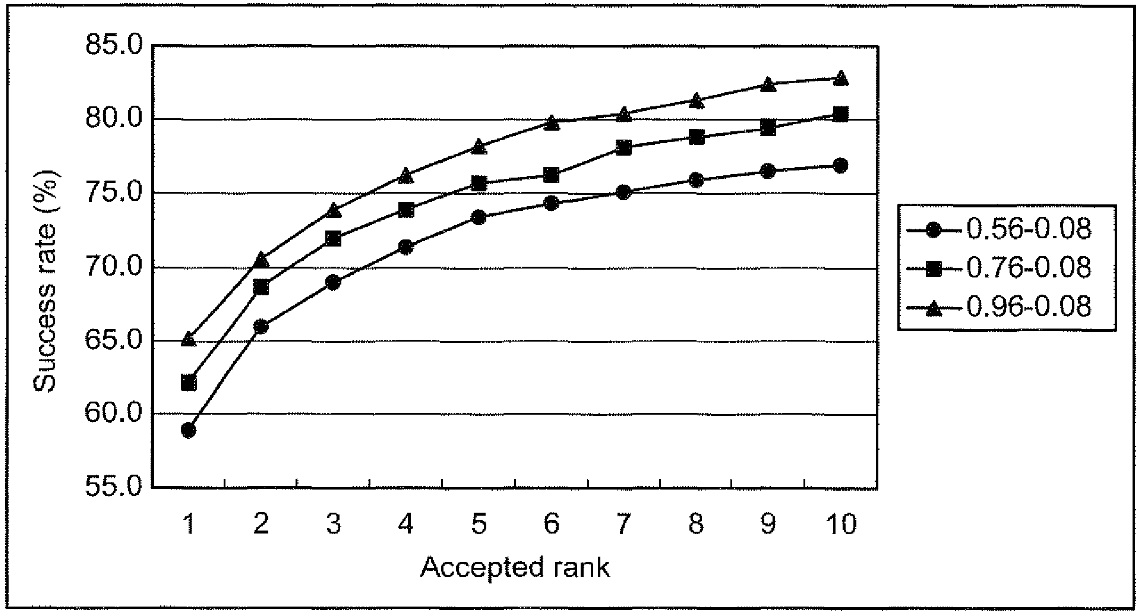

FIG.22

| | Tc Range | | |
|---|---|---|---|
| | 0.56-0.08 | 0.76-0.08 | 0.96-0.08 |
| 1 | 68.4 | 72.1 | 72.4 |
| 2 | 74.8 | 77.4 | 78.1 |
| 3 | 77.2 | 79.8 | 81.0 |
| 4 | 79.0 | 80.8 | 83.1 |
| 5 | 80.4 | 82.0 | 84.6 |
| 6 | 80.8 | 82.6 | 86.1 |
| 7 | 81.6 | 83.9 | 86.7 |
| 8 | 82.2 | 84.8 | 86.7 |
| 9 | 83.0 | 85.1 | 87.4 |
| 10 | 83.4 | 85.5 | 87.6 |

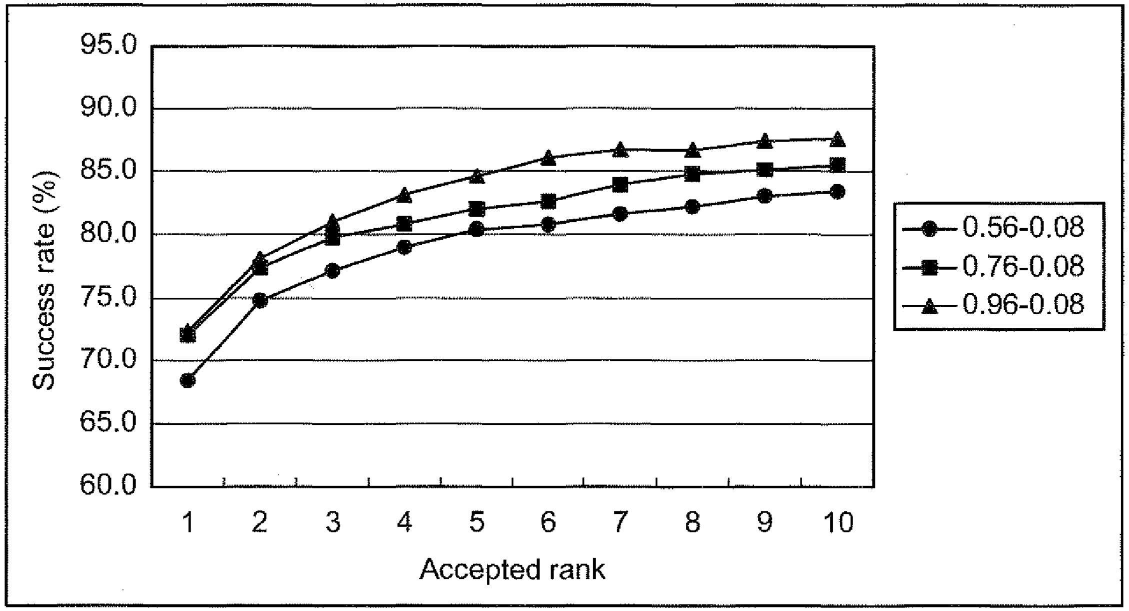

FIG.23

| | Tc Range | | |
|---|---|---|---|
| | 0.56-0.08 | 0.76-0.08 | 0.96-0.08 |
| 1.5 | 37.8 | 43.9 | 48.5 |
| 1.6 | 42.1 | 48.7 | 53.9 |
| 1.7 | 46.5 | 51.9 | 57.9 |
| 1.8 | 51.5 | 55.6 | 61.4 |
| 1.9 | 55.5 | 59.5 | 63.8 |
| 2 | 58.9 | 62.1 | 65.2 |
| 2.1 | 60.6 | 64.8 | 67.4 |
| 2.2 | 63.5 | 66.8 | 69.2 |
| 2.3 | 65.7 | 68.6 | 70.2 |
| 2.4 | 67.0 | 70.6 | 71.8 |
| 2.5 | 68.6 | 72.1 | 72.4 |
| 2.6 | 70.1 | 73.3 | 74.6 |
| 2.7 | 71.5 | 74.8 | 76.2 |
| 2.8 | 72.9 | 75.2 | 77.4 |
| 2.9 | 73.9 | 76.0 | 78.1 |
| 3 | 74.0 | 76.9 | 78.7 |
| 3.1 | 74.6 | 77.1 | 79.4 |
| 3.2 | 74.9 | 78.3 | 79.9 |
| 3.3 | 75.5 | 78.9 | 80.1 |
| 3.4 | 76.3 | 80.2 | 81.3 |
| 3.5 | 76.9 | 81.3 | 82.3 |

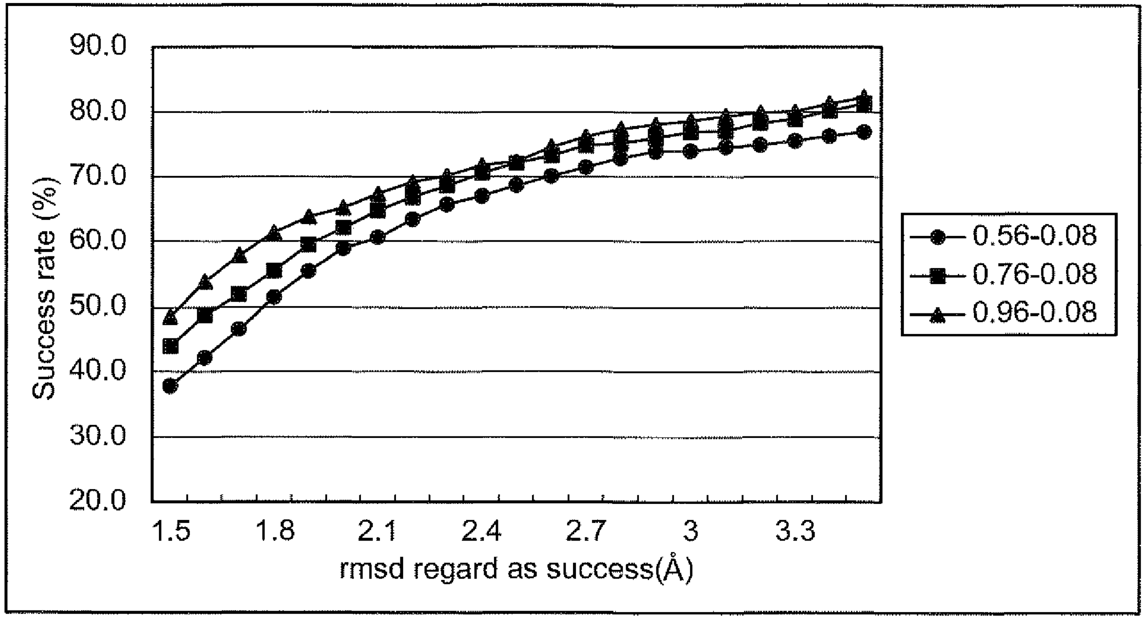

# FIG.24

| Docking soft | Corina | MINI | success rate (%) | SD(Corina, MINI) |
|---|---|---|---|---|
| AutoDock | 26.2 | 27 | 26.6 | 0.57 |
| DOCK | 21.6 | 20.6 | 21.1 | 0.71 |
| GOLD ChemScoreSTD | 45.5 | 45.3 | 45.4 | 0.14 |
| GOLD GOLDScoreLib | 44.1 | 44.9 | 44.5 | 0.57 |
| GOLD GOLDScoreSTD | 45.2 | 46.7 | 46.0 | 1.06 |
| ChooseLD Tc 0.56-0.08 | n/a | n/a | 40.1 | n/a |
| ChooseLD Tc 0.76-0.08 | n/a | n/a | 44.8 | n/a |
| ChooseLD Tc 0.96-0.08 | n/a | n/a | 46.4 | n/a |

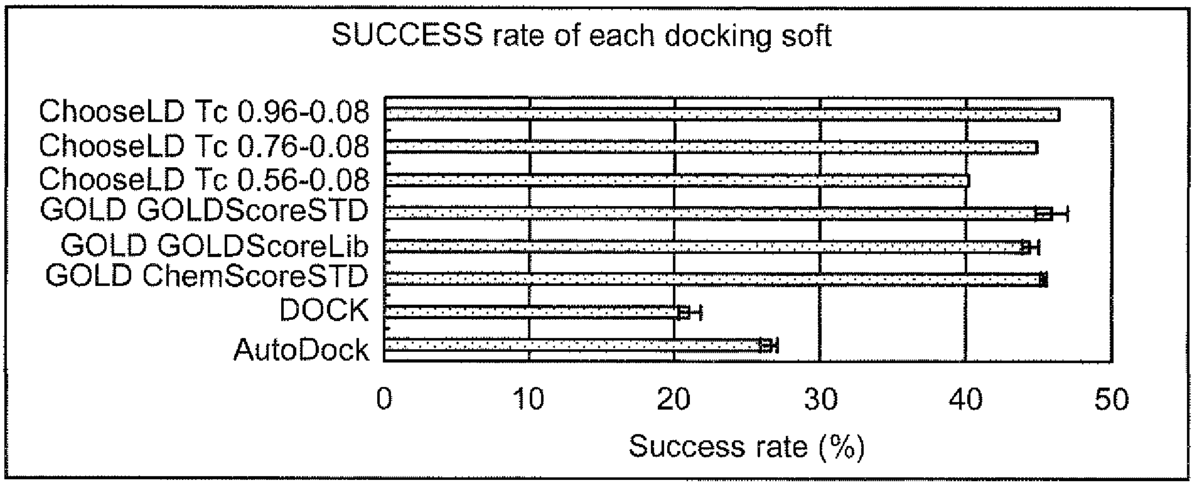

FIG.25

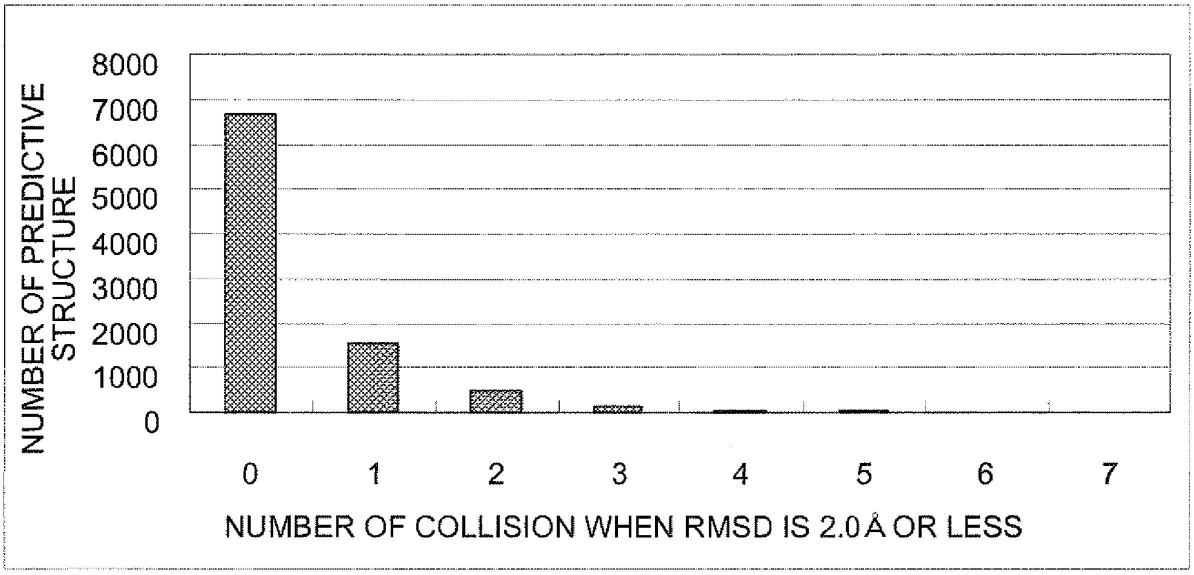


FIG.26

| Tc RANGE | NUMBER OF PREDICTIVE SUCCESS IN 10 TIMES IN 85 SETS | | | | | | | | | | | *1 |
|---|---|---|---|---|---|---|---|---|---|---|---|---|
| | 0 | 1 | 2 | 3 | 4 | 5 | 6 | 7 | 8 | 9 | 10 | SUM | |
| 0.56 - 0.08 | 19 | 3 | 3 | 3 | 3 | 6 | 3 | 3 | 1 | 8 | 31 | 83 | 62.7% |
| 0.76 - 0.08 | 17 | 3 | 4 | 4 | 2 | 2 | 3 | 5 | 3 | 6 | 35 | 84 | 64.3% |
| 0.96 - 008 | 13 | 5 | 3 | 5 | 3 | 1 | 2 | 5 | 4 | 4 | 39 | 84 | 65.5% |

FIG.27

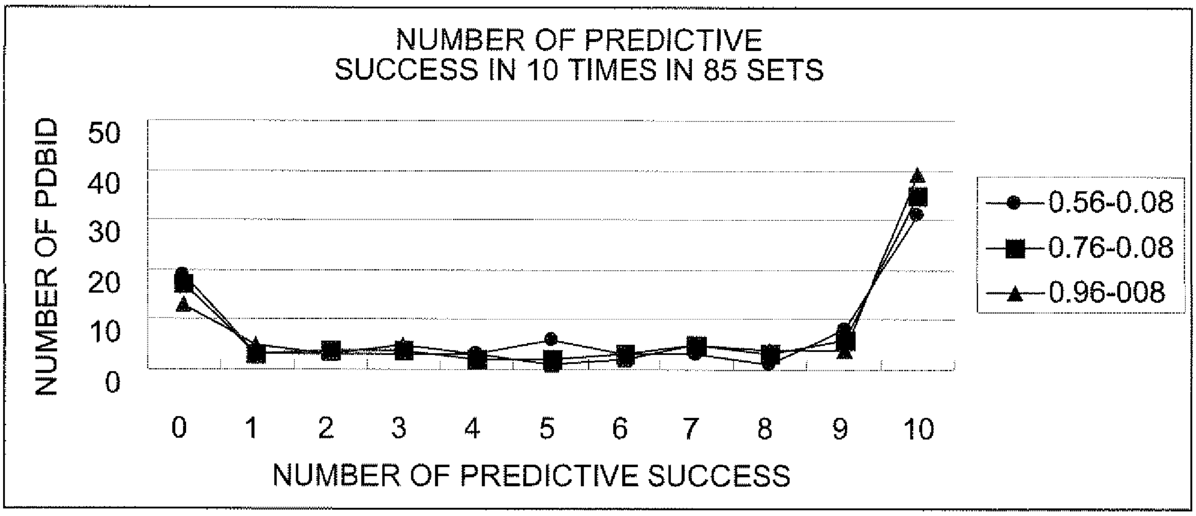
NUMBER OF PREDICTIVE
SUCCESS IN 10 TIMES IN 85 SETS
NUMBER OF PDBID
50
40
30
20
10
0
0 1 2 3 4 5 6 7 8 9 10
NUMBER OF PREDICTIVE SUCCESS
0.56-0.08
0.76-0.08
0.96-008

FIG.28

| DOCKING SOFTWARE PROGRAM | RATIO OF PREDICTIVE SUCCESS (%) | | | DOCKING SOFTWARE PROGRAM | RATIO OF PREDICTIVE SUCCESS (%) |
|---|---|---|---|---|---|
| | Corina | MINI | AVERAGE | | |
| DOCK | 21.6 | 20.6 | 21.1 | ChooseLD Tc 0.56 - 0.08 | 40.1 |
| AutoDock | 26.2 | 27.0 | 26.6 | ChooseLD Tc 0.76 - 0.08 | 44.8 |
| GOLD ChemScoreSTD | 45.5 | 45.3 | 45.4 | ChooseLD Tc 0.96 - 0.08 | 46.4 |
| GOLD GOLDScoreLib | 44.1 | 44.9 | 44.5 | | |
| GOLD GOLDScoreSTD | 45.2 | 46.7 | 46.0 | | |

FIG.29

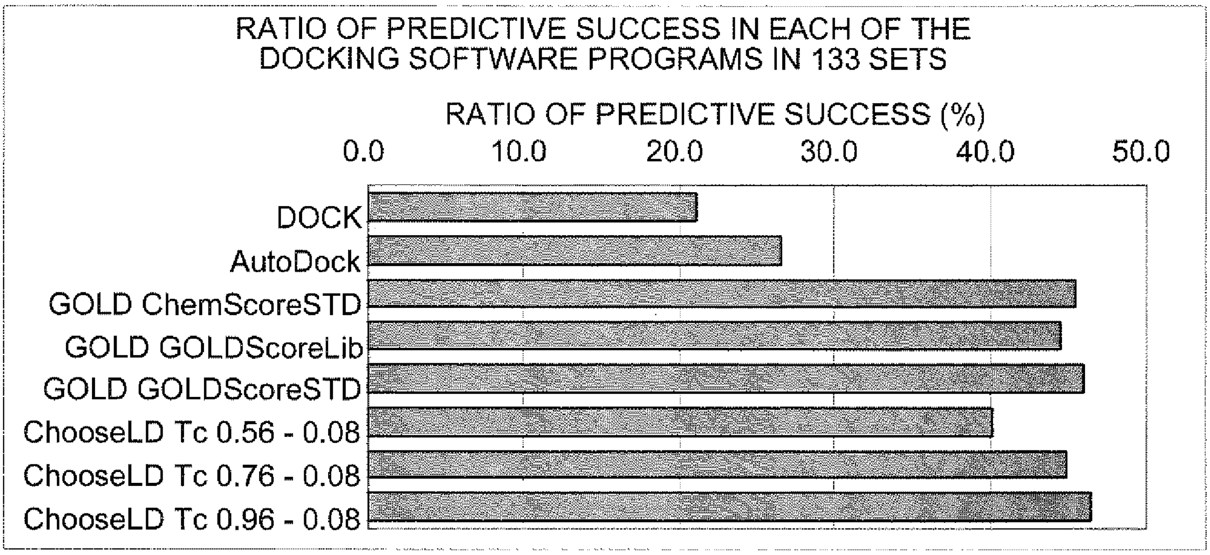


FIG.30

| Tc RANGE | NUMBER OF PREDICTIVE SUCCESS IN 10 TIMES IN 133 SETS | | | | | | | | | | | | *1 |
|---|---|---|---|---|---|---|---|---|---|---|---|---|---|
| | 0 | 1 | 2 | 3 | 4 | 5 | 6 | 7 | 8 | 9 | 10 | SUM | |
| 0.56-0.08 | 46 | 5 | 6 | 9 | 3 | 8 | 1 | 3 | 2 | 4 | 29 | 116 | 40.5% |
| 0.76-0.08 | 45 | 6 | 6 | 5 | 0 | 5 | 3 | 1 | 6 | 1 | 38 | 116 | 46.6% |
| 0.96-0.08 | 41 | 7 | 3 | 9 | 3 | 2 | 4 | 1 | 5 | 5 | 36 | 116 | 45.7% |

FIG.31

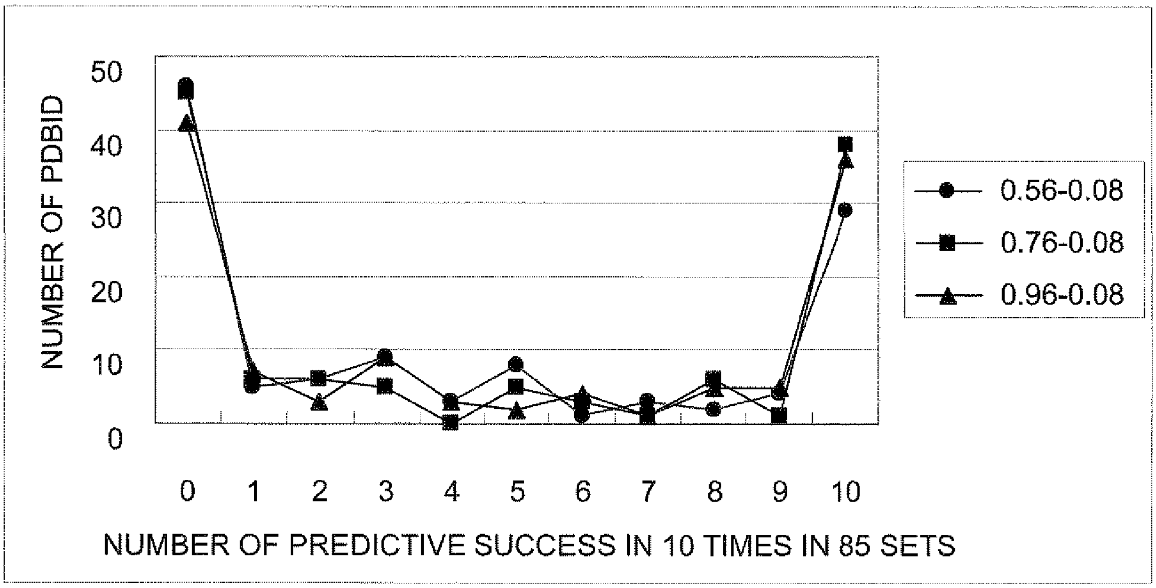


FIG.32

| Tc RANGE | ADOPTED FPAScore RANK | | | | | | | | | |
|---|---|---|---|---|---|---|---|---|---|---|
| | 1 | 2 | 3 | 4 | 5 | 6 | 7 | 8 | 9 | 10 |
| 0.56 - 0.08 | 40.1 | 46.9 | 49.7 | 52.4 | 54.2 | 55.9 | 56.5 | 57.2 | 57.8 | 58.0 |
| 0.76 - 0.08 | 44.8 | 49.7 | 52.9 | 55.6 | 57.2 | 58.5 | 59.5 | 60.1 | 60.3 | 61.0 |
| 0.96 - 0.08 | 46.4 | 52.7 | 56.6 | 58.7 | 60.3 | 61.6 | 62.3 | 63.3 | 63.7 | 64.7 |

FIG.33

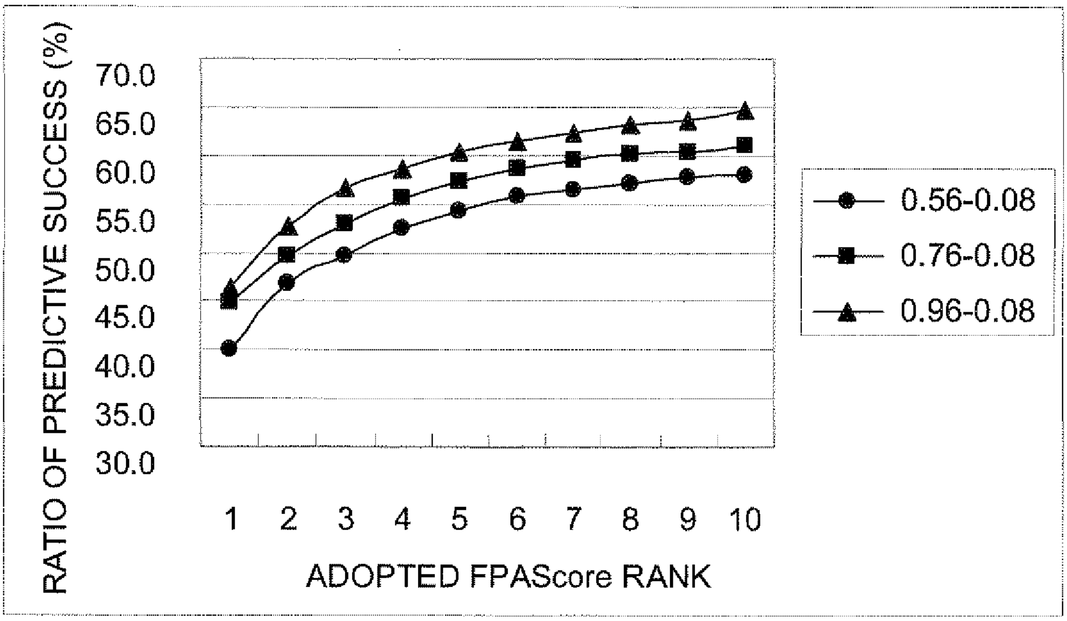
RATIO OF PREDICTIVE SUCCESS (%)
70.0
65.0
60.0
55.0
50.0
45.0
40.0
35.0
30.0
1
2
3
4
5
6
7
8
9
10
ADOPTED FPAScore RANK
0.56-0.08
0.76-0.08
0.96-0.08

FIG.34

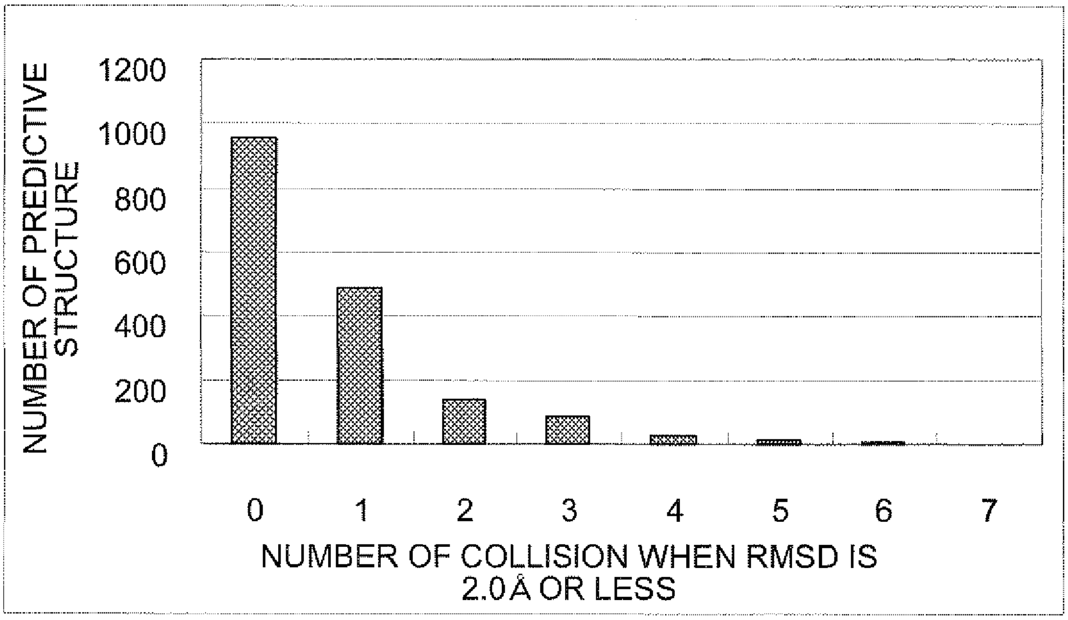
NUMBER OF PREDICTIVE STRUCTURE
1200
1000
800
600
400
200
0
0
1
2
3
4
5
6
7
NUMBER OF COLLISION WHEN RMSD IS 2.0Å OR LESS

FIG.35

| Tc RANGE | RATIO OF PREDICTIVE SUCCESS (%) | Tc RANGE | RATIO OF PREDICTIVE SUCCESS (%) |
|---|---|---|---|
| 0.16 - 0.08 | 12.6 | 0.56 - 0.08 | 40.1 |
| 0.24 - 0.08 | 20.8 | 0.76 - 0.08 | 44.8 |
| 0.36 - 0.08 | 29.2 | 0.96 - 0.08 | 46.4 |

# FIG.36

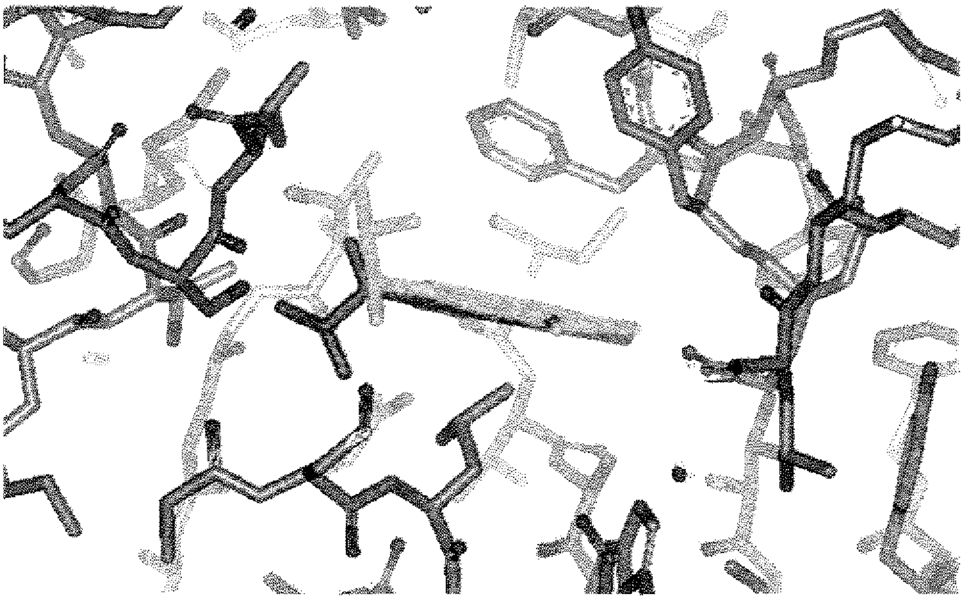

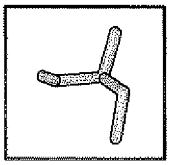

: CORRECT STRUCTURE OF LIGAND
(CENTRAL CYAN PART)

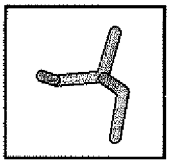

: PREDICTIVE STRUCTURE OF LIGAND
(CENTRAL GREEN PART)

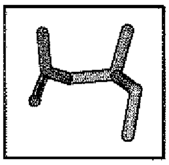

: BINDING SITE OF PROTEIN
(PERIPHERAL PART)

FIG.37

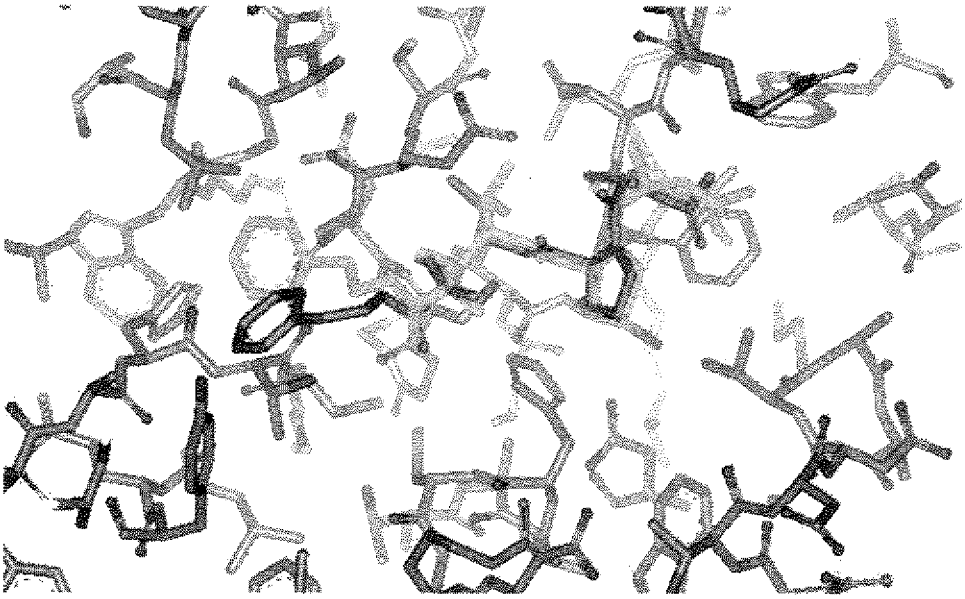

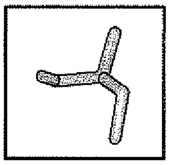

: CORRECT STRUCTURE OF LIGAND (CENTRAL CYAN PART)

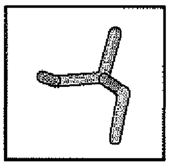

: PREDICTIVE STRUCTURE OF LIGAND (CENTRAL GREEN PART)

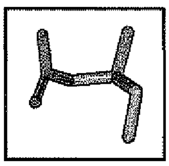

: BINDING SITE OF PROTEIN (PERIPHERAL PART)

FIG.38

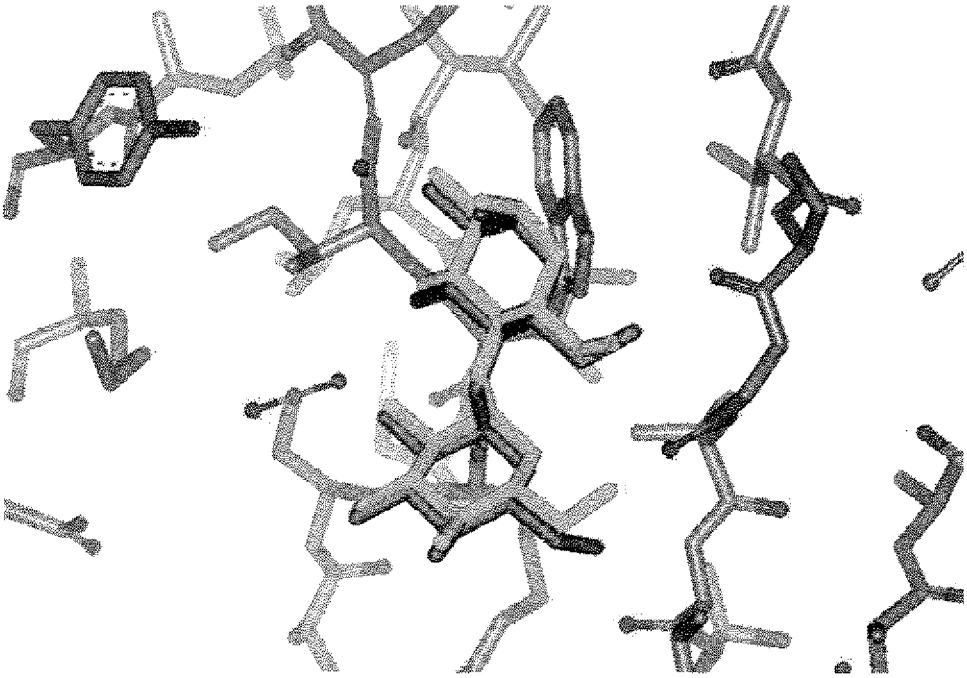

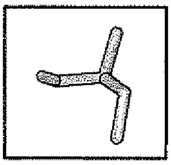
: CORRECT STRUCTURE OF LIGAND (CENTRAL CYAN PART)

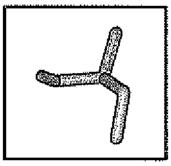
: PREDICTIVE STRUCTURE OF LIGAND (CENTRAL GREEN PART)

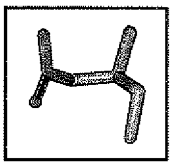
: BINDING SITE OF PROTEIN (PERIPHERAL PART)

FIG.39

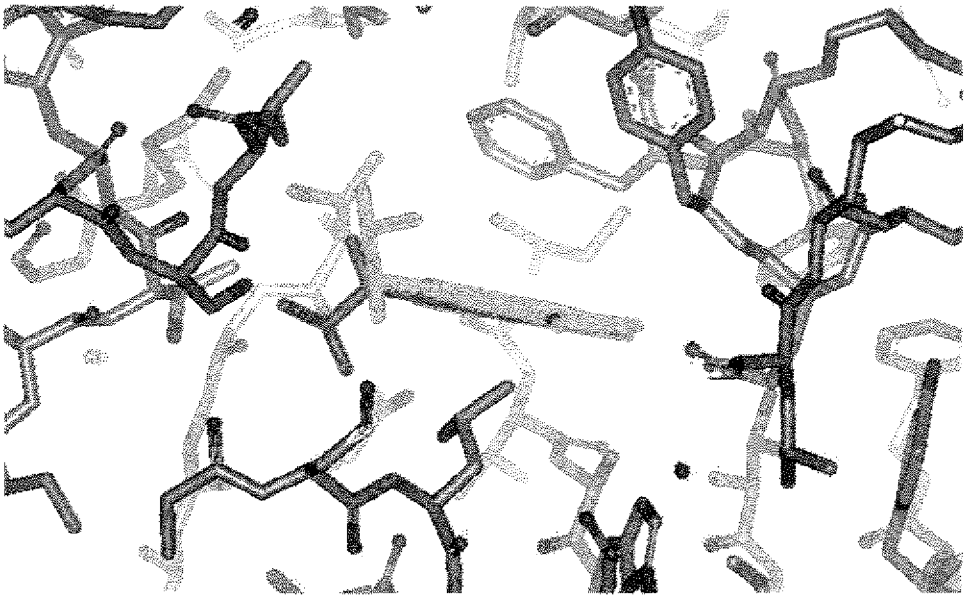

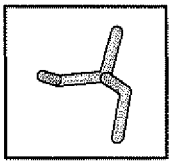

: CORRECT STRUCTURE OF LIGAND
(CENTRAL CYAN PART)

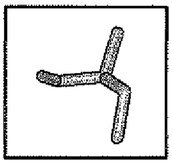

: PREDICTIVE STRUCTURE OF LIGAND
(CENTRAL GREEN PART)

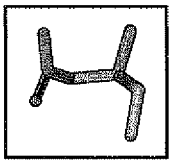

: BINDING SITE OF PROTEIN
(PERIPHERAL PART)

FIG.40

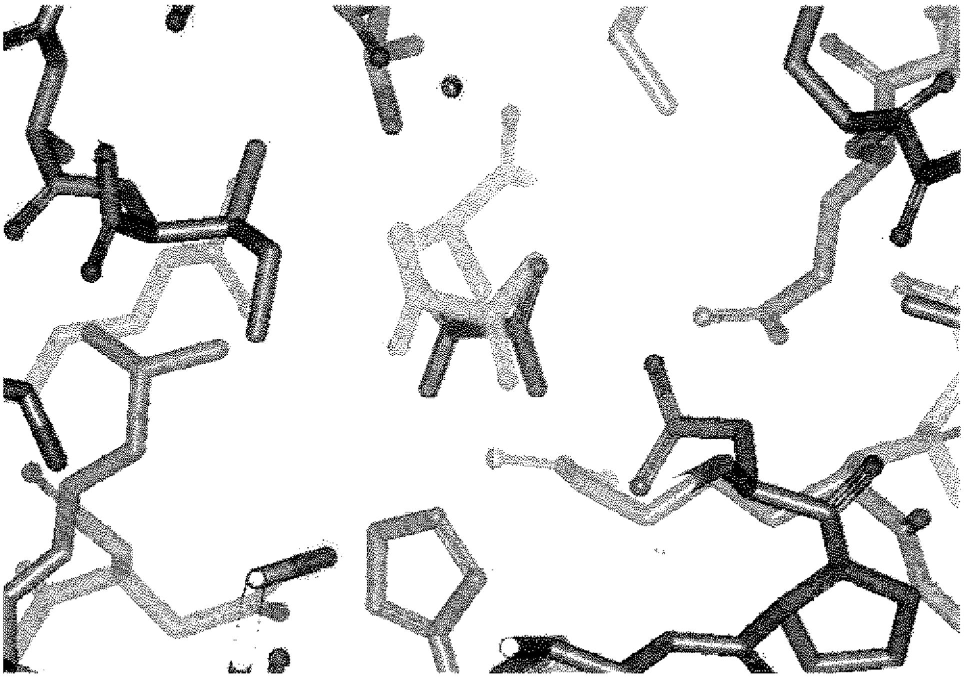

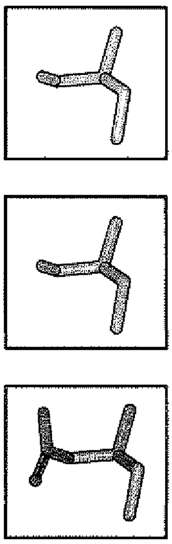

: CORRECT STRUCTURE OF LIGAND
(CENTRAL CYAN PART)

: PREDICTIVE STRUCTURE OF LIGAND
(CENTRAL GREEN PART)

: BINDING SITE OF PROTEIN
(PERIPHERAL PART)

FIG.41

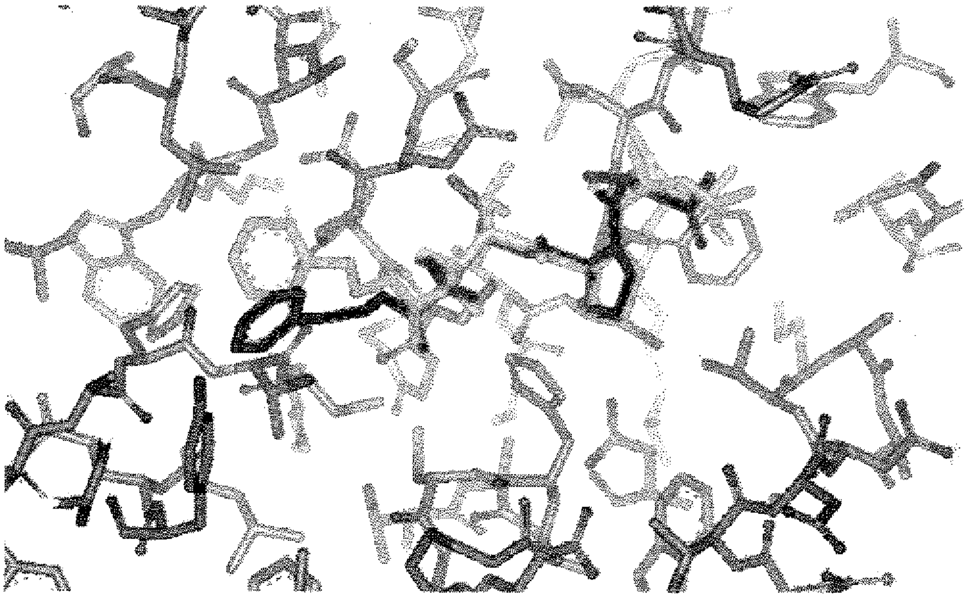

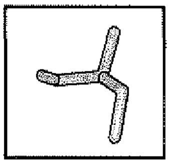

: CORRECT STRUCTURE OF LIGAND (CENTRAL CYAN PART)

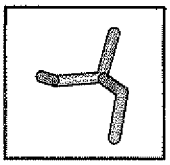

: PREDICTIVE STRUCTURE OF LIGAND (CENTRAL GREEN PART)

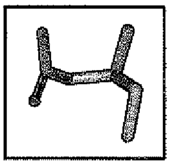

: BINDING SITE OF PROTEIN (PERIPHERAL PART)

# FIG.42

| C | RATIO OF PREDICTIVE SUCCESS (%) |
|---|---|
| ChooseLD 0.96 - 0.08 | 62.2 |
| ChooseLD 0.76 - 0.08 | 61.1 |
| ChooseLD 0.56 - 0.08 | 60.0 |
| Glide | 72.2 |
| GOLD | 66.7 |

# FIG.43

| | Glide | GOLD | FlexX | ChooseLD |
|---|---|---|---|---|
| Glide | 1.00 | 0.65 | 0.61 | 0.55 |
| Gold | 0.65 | 1.00 | 0.66 | 0.50 |
| FlexX | 0. 61 | 0.66 | 1.00 | 0.47 |
| ChooseLD | 0.55 | 0.50 | 0.47 | 1.00 |

FIG.44

| | Glide SUCCESS | Glide FAILURE |
|---|---|---|
| ChooseLD SUCCESS | 42 | 12 |
| ChooseLD FAILURE | 23 | 13 |

| | GOLD SUCCESS | GOLD FAILURE |
|---|---|---|
| ChooseLD SUCCESS | 38 | 16 |
| ChooseLD FAILURE | 23 | 13 |

FIG.45

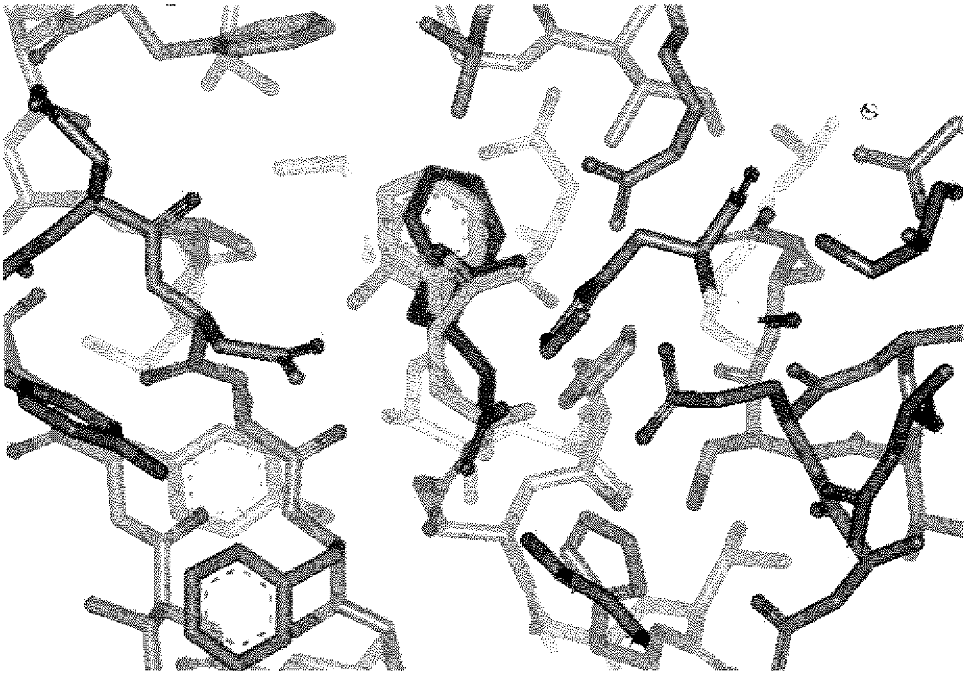

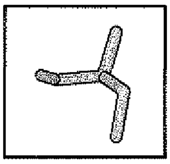 : CORRECT STRUCTURE OF LIGAND (CENTRAL CYAN PART)

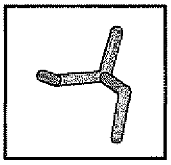 : PREDICTIVE STRUCTURE OF LIGAND (CENTRAL GREEN PART)

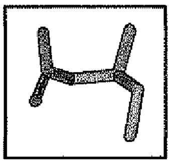 : BINDING SITE OF PROTEIN (PERIPHERAL PART)

FIG.46

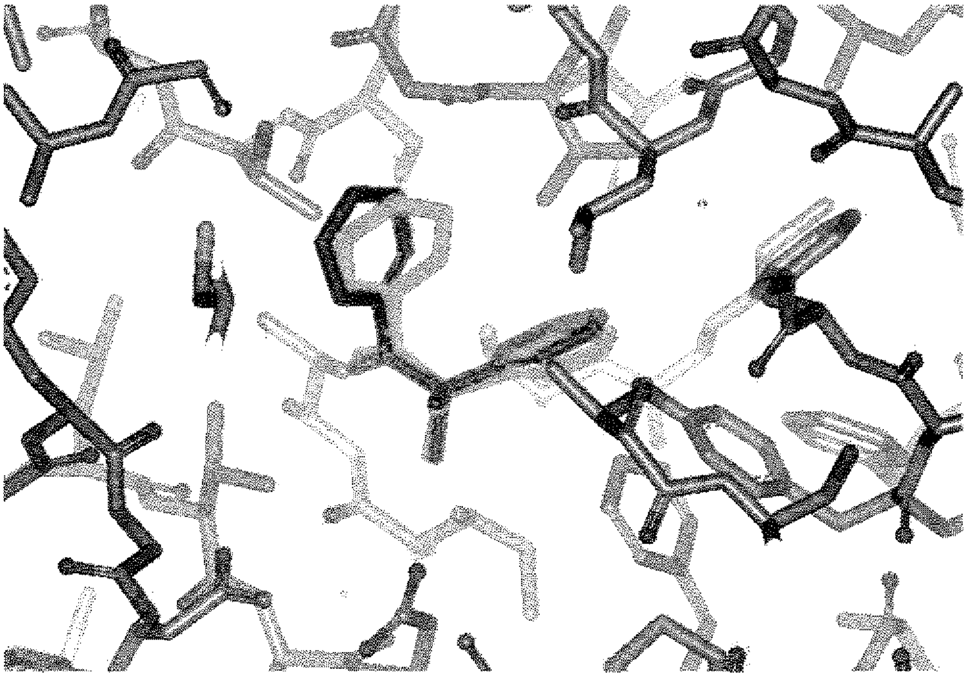

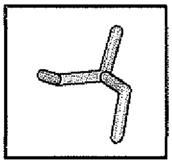
: CORRECT STRUCTURE OF LIGAND (CENTRAL CYAN PART)

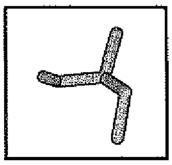
: PREDICTIVE STRUCTURE OF LIGAND (CENTRAL GREEN PART)

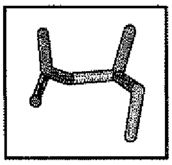
: BINDING SITE OF PROTEIN (PERIPHERAL PART)

FIG.47

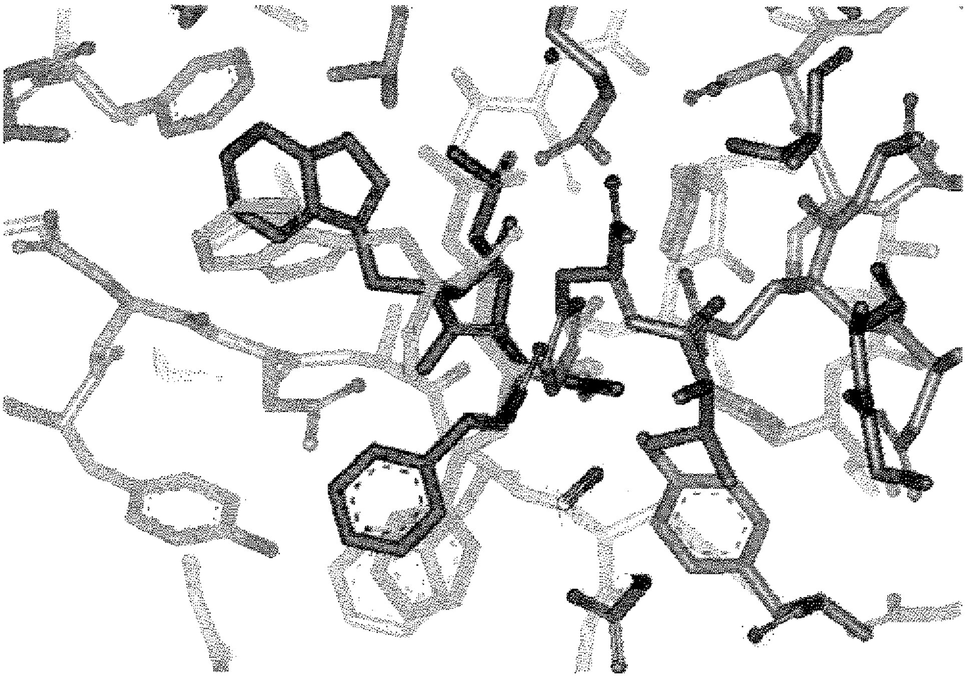

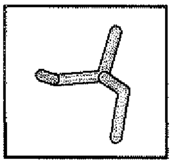

: CORRECT STRUCTURE OF LIGAND
(CENTRAL CYAN PART)

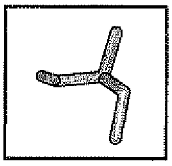

: PREDICTIVE STRUCTURE OF LIGAND
(CENTRAL GREEN PART)

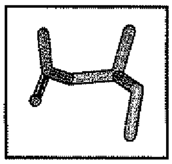

: BINDING SITE OF PROTEIN
(PERIPHERAL PART)

# FIG.48

| | Tc Range | | |
|---|---|---|---|
| | 0.56-0.08 | 0.76-0.08 | 0.96-0.08 |
| 1 | 40.1 | 44.8 | 46.4 |
| 2 | 46.9 | 49.7 | 52.7 |
| 3 | 49.7 | 52.9 | 56.6 |
| 4 | 52.4 | 55.6 | 58.7 |
| 5 | 54.2 | 57.2 | 60.3 |
| 6 | 55.9 | 58.5 | 61.6 |
| 7 | 56.5 | 59.5 | 62.3 |
| 8 | 57.2 | 60.1 | 63.3 |
| 9 | 57.8 | 60.3 | 63.7 |
| 10 | 58.0 | 61.0 | 64.7 |

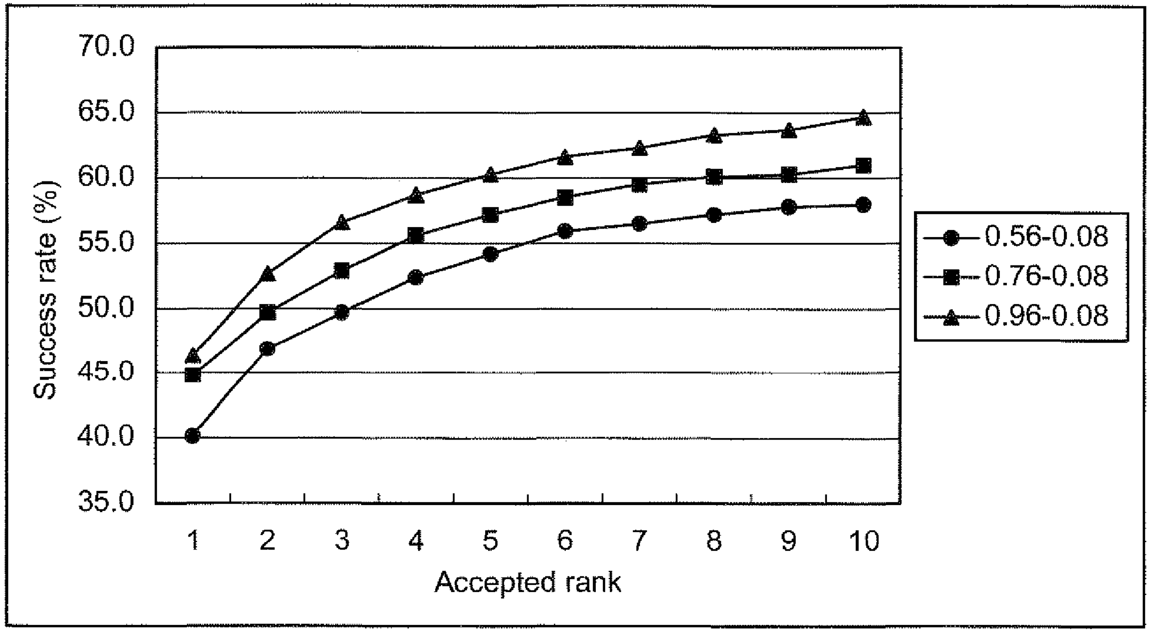

FIG.49

| | Tc Range | | |
|---|---|---|---|
| | 0.56-0.08 | 0.76-0.08 | 0.96-0.08 |
| 1 | 53.1 | 57.8 | 59.3 |
| 2 | 59.2 | 62.8 | 65.1 |
| 3 | 62.2 | 64.8 | 68.1 |
| 4 | 64.7 | 67.1 | 69.4 |
| 5 | 66.4 | 68.2 | 70.5 |
| 6 | 67.5 | 69.3 | 71.5 |
| 7 | 68.2 | 70.2 | 72.4 |
| 8 | 69.7 | 70.9 | 73.7 |
| 9 | 70.6 | 71.2 | 74.7 |
| 10 | 71.0 | 72.1 | 75.8 |

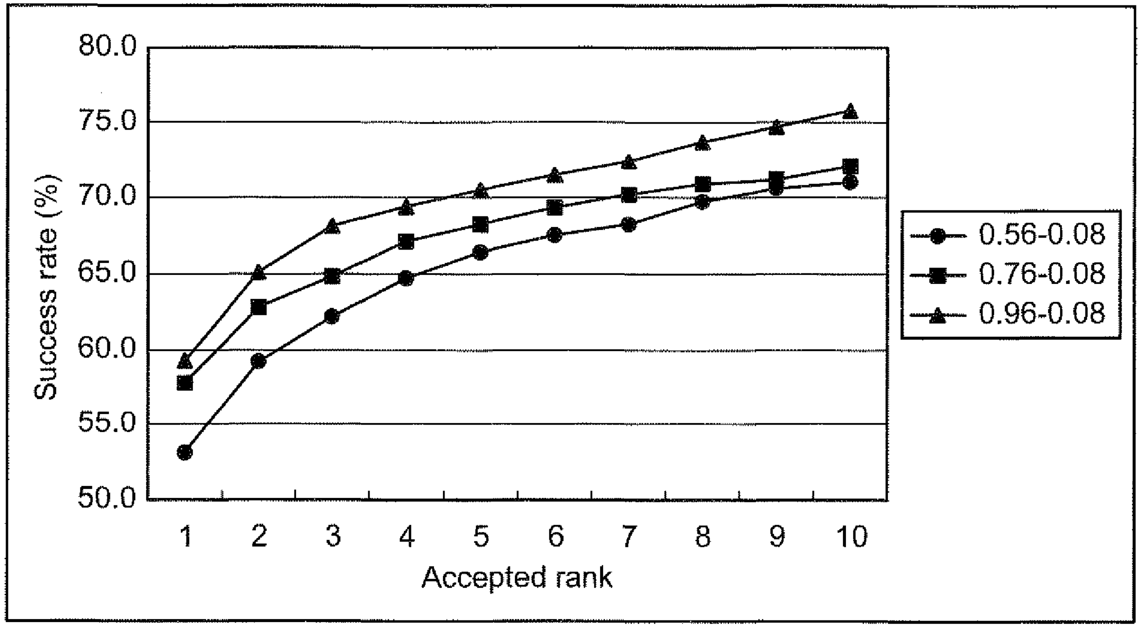

# FIG.50

| | Tc Range | | |
|---|---|---|---|
| | 0.56-0.08 | 0.76-0.08 | 0.96-0.08 |
| 1.5 | 26.4 | 29.6 | 29.9 |
| 1.6 | 27.9 | 31.5 | 32.1 |
| 1.7 | 30.7 | 33.6 | 34.1 |
| 1.8 | 33.1 | 36.4 | 38.2 |
| 1.9 | 36.8 | 41.4 | 42.0 |
| 2.0 | 40.1 | 44.8 | 46.4 |
| 2.1 | 43.3 | 47.8 | 50.0 |
| 2.2 | 46.0 | 51.0 | 53.1 |
| 2.3 | 48.8 | 53.6 | 55.0 |
| 2.4 | 50.9 | 55.9 | 57.6 |
| 2.5 | 53.2 | 57.8 | 59.3 |
| 2.6 | 55.2 | 59.4 | 61.4 |
| 2.7 | 57.6 | 61.1 | 63.5 |
| 2.8 | 59.8 | 62.4 | 65.4 |
| 2.9 | 61.8 | 64.1 | 67.1 |
| 3.0 | 63.3 | 65.7 | 68.6 |
| 3.1 | 64.9 | 66.6 | 69.5 |
| 3.2 | 65.5 | 67.5 | 70.9 |
| 3.3 | 67.3 | 67.9 | 71.5 |
| 3.4 | 67.9 | 68.5 | 72.1 |
| 3.5 | 68.9 | 69.1 | 73.1 |

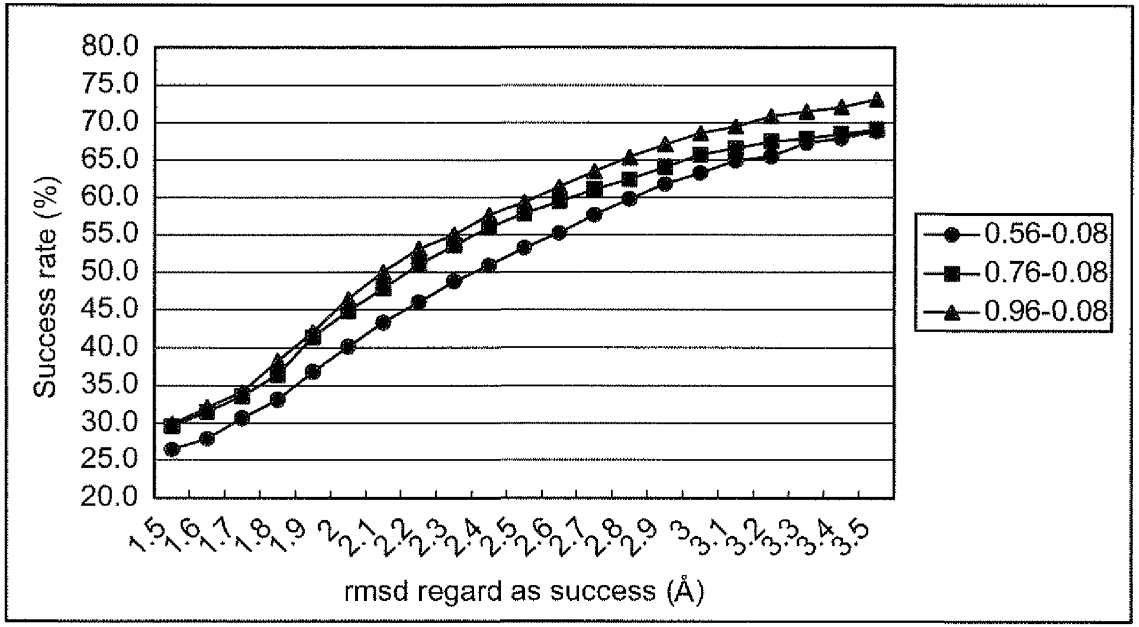

# FIG.51

| | | rmsd 2.0(Good) | rmsd 2.5(Close) |
|---|---|---|---|
| Druglike-ligand vs Druggable-protein (85 set) | Tc Range 0.56-0.08 | 58.9 | 68.6 |
| | Tc Range 0.76-0.08 | 62.1 | 72.1 |
| | Tc Range 0.56-0.08 | 65.2 | 72.4 |
| Various-ligand vs Druggable-protein (133 set) | Tc Range 0.56-0.08 | 40.1 | 53.2 |
| | Tc Range 0.76-0.08 | 44.8 | 57.8 |
| | Tc Range 0.56-0.08 | 46.4 | 59.3 |

# FIG.52

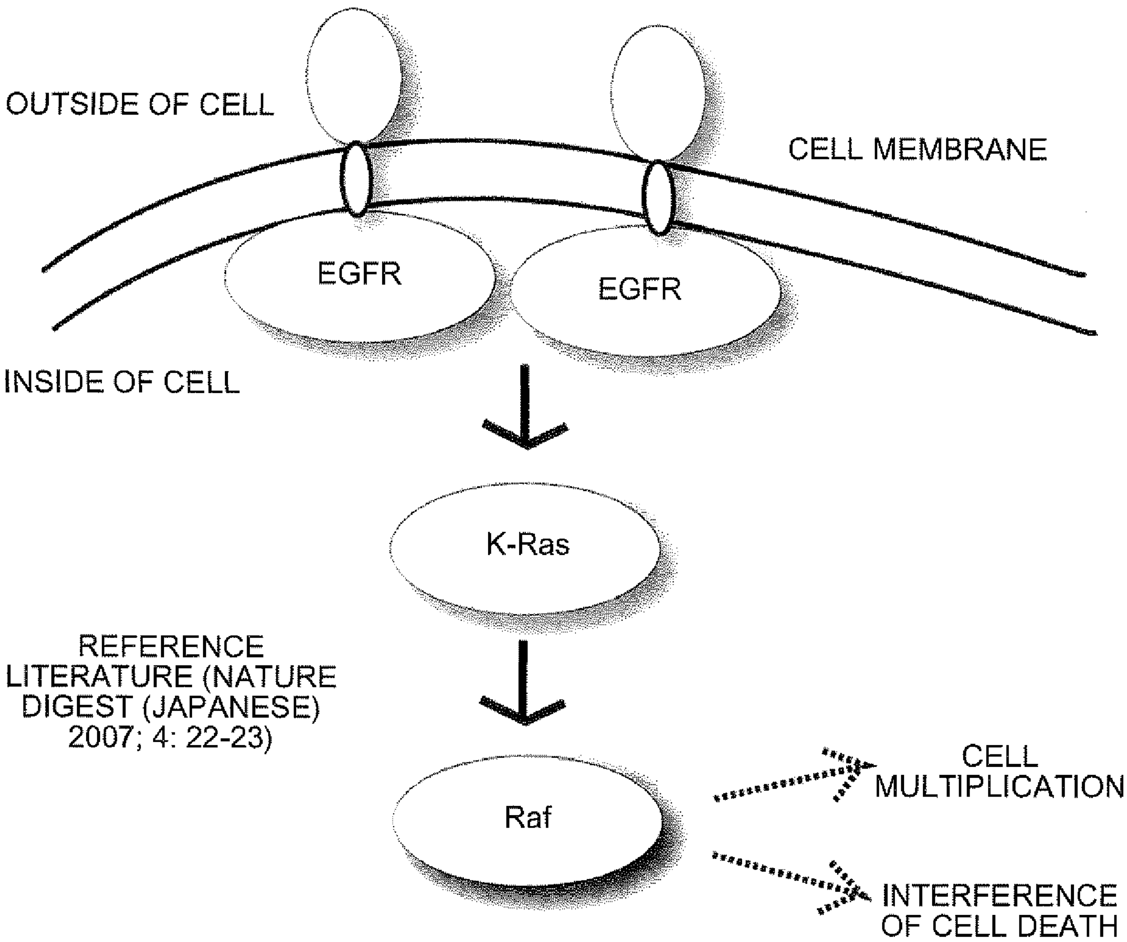

# FIG.53

MULTIPLE MATCHING Version in2mlmt

| | | | | | | |
|---|---|---|---|---|---|---|
| >EGFR_HUMAN696 | | HOMOLOGY | MATCH | MISMATCH | INSERTION | DELETION |
| >1M17_A | 312 | 98.7% | 308 | 4 | 0 | 12 |

```
GEAPNQALLRILKETEFKKIKVLGSGAFGTVYKGLWIPEGEKVKIPVAIKELREATSPKANKEILDEAYVMASVDNPHVC
GEAPNQALLRILKETEFKKIKVLGSGAFGTVYKGLWIPEGEKVKIPVAIKELREATSPKANKEILDEAYVMASVDNPHVC
********************************************************************************
cccc111111ccccc2222222ccccccc2222222ccccccc22222222ccccc1111111111111ccccccc22
ccccccccccc4444222222222c4444222222222c4444ccc2222222cccccc11111111111111cccc44cc

RLLGICLTSTVQLITQLMPFGCLLDYVREHKDNIGSQYLLNWCVQIAKGMNYLEDRRLVHRDLAARNVLVKTPQHVKITD
RLLGICLTSTVQLITQLMPFGCLLDYVREHKDNIGSQYLLNWCVQIAKGMNYLEDRRLVHRDLAARNVLVKTPQHVKITD
********************************************************************************
222222cccc222222cccccc111111cccccc111111111111111111ccc111111122ccccc22222c
c2222224442222222c44422111111c111cc1111111111111111111c224444c11122222442222cc

FGLAKLLGAEEKEYHAEGGKVPIKWMALESILHRIYTHQSDVWSYGVTVWELMTFGSKPYDGIPASEISSILEKGERLPQ
FGLAKLLGAEEKEYHAEGGKVPIKWMALESILHRIYTHQSDVWSYGVTVWELMTFGSKPYDGIPASEISSILEKGERLPQ
********************************************************************************
cc111cccccccc22cccccccc11cc111111ccccc11111111111111ccccccccccc11111111ccccccc
444422c4444cc22cccccc44444c111111c22c1111111111111111144444444444411111111cccccc

PPICTIDVYMIMVKCWMIDADSRPKFRELIIEFSKMARDPQRYLVIQGDERMHLPSPTDSNFYRALMDEEDMDDVVDADE
PPICTIDVYMIMVKCWMIDADSRPKFRELIIEFSKMARDPQRYLVIQGDERMHLMDEE------------DMDDVVDADE
******************************************************++++++++++++++++**********
ccccc11111111111cc111ccc111111111111c111111ccccccccccccccccccccccc11111ccccccc
444cc111111111c444111ccc111111111111c111ccc444444ccccccc............c4444cc111

YLIP
YLIP
****
cccc
cccc

//
```

| |
|---|
| * CONCORDANT AMINO ACID RESIDUE |
| + DISCORDANT AMINO ACID RESIDUE |

FIG.54

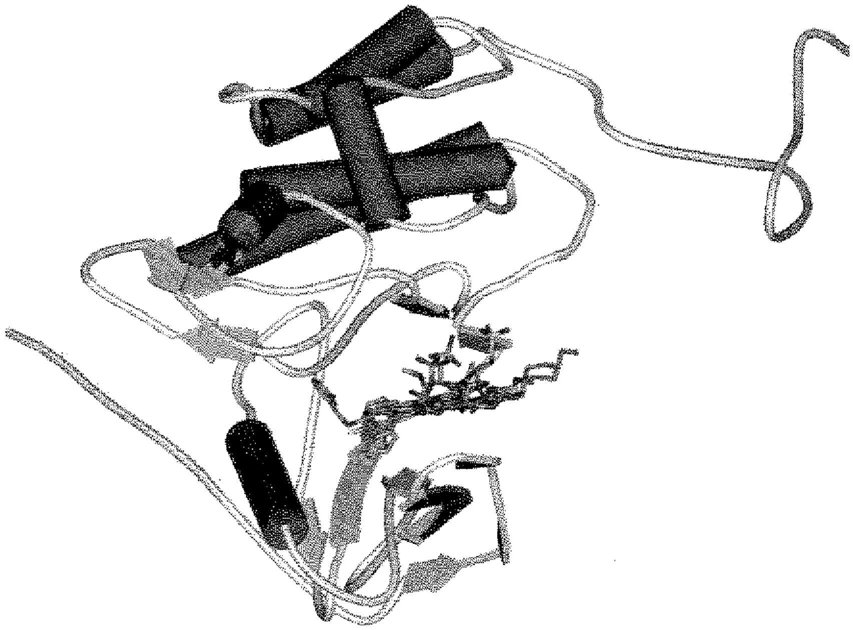

MODEL OF EGFR; USING 1M17 AS TEMPLATE

FIG.55

LIST OF COMPOUND WITH KNOWN IC50 VALUE (11 EGFR INHIBITORS)

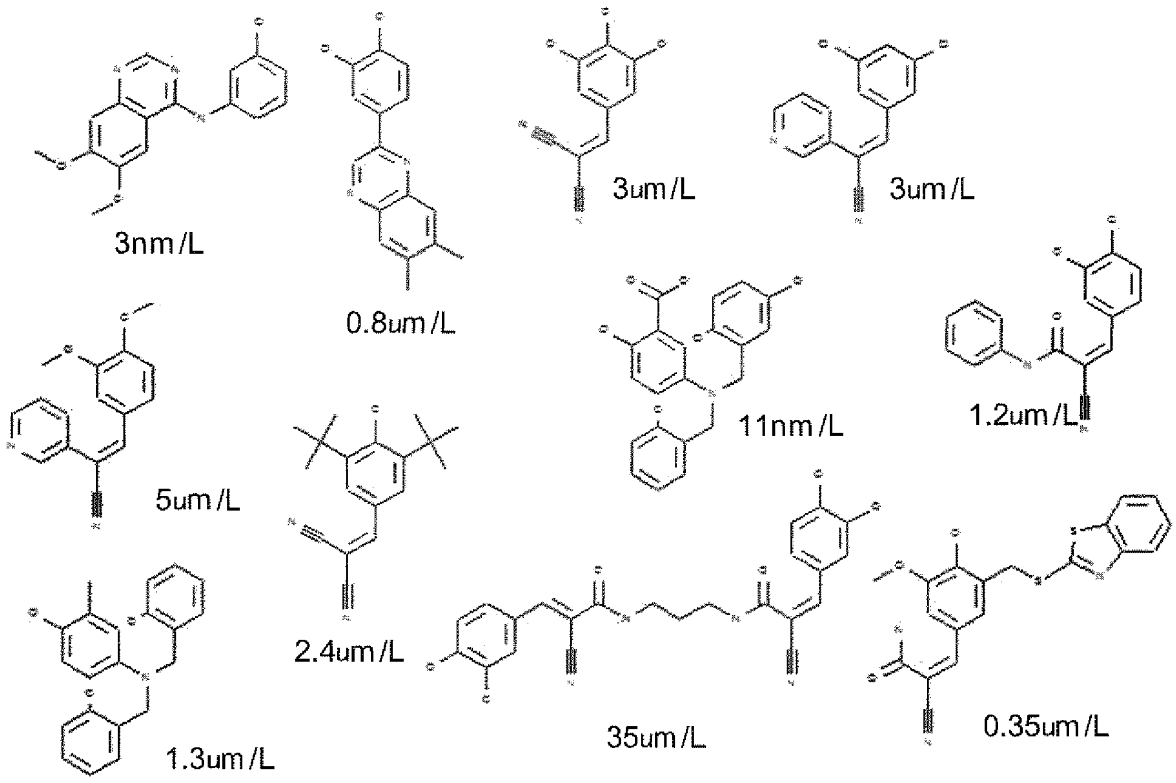

3nm/L

0.8um/L

3um/L

3um/L

11nm/L

1.2um/L

5um/L

2.4um/L

35um/L

0.35um/L

1.3um/L

# FIG.56

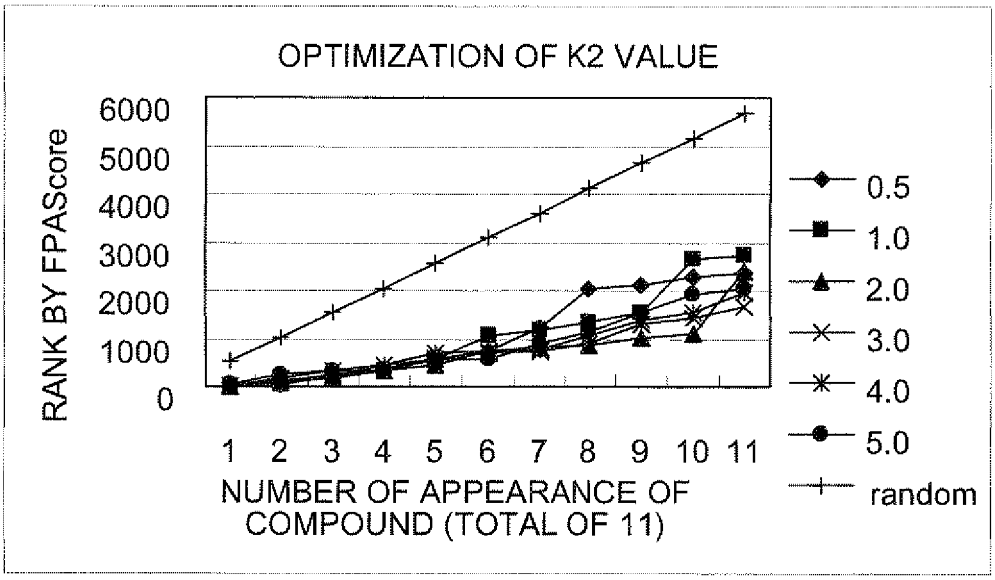
OPTIMIZATION OF K2 VALUE
RANK BY FPAScore
6000
5000
4000
3000
2000
1000
0
1 2 3 4 5 6 7 8 9 10 11
NUMBER OF APPEARANCE OF COMPOUND (TOTAL OF 11)
0.5
1.0
2.0
3.0
4.0
5.0
random

# FIG.57

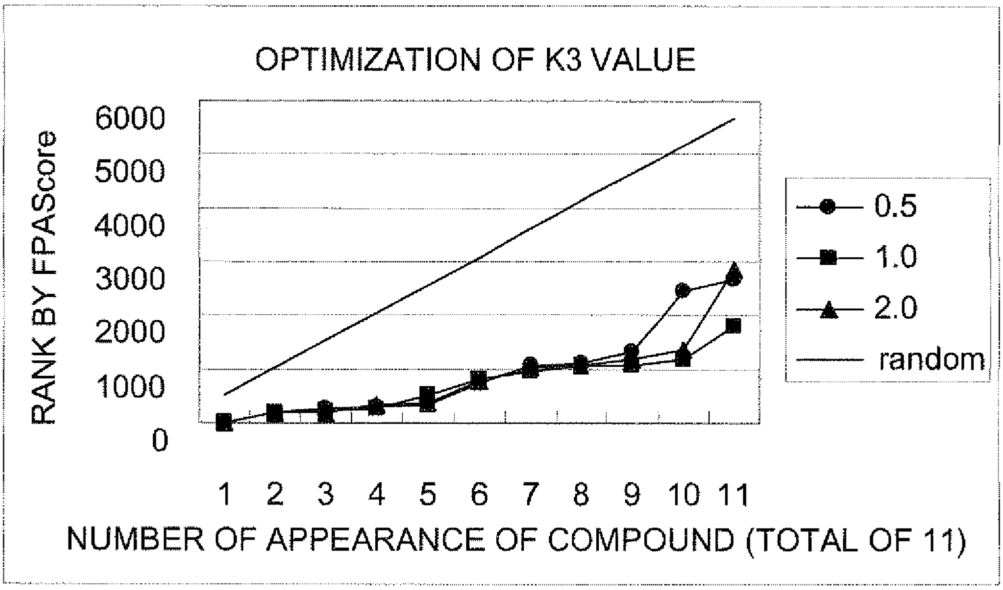
OPTIMIZATION OF K3 VALUE
RANK BY FPAScore
6000
5000
4000
3000
2000
1000
0
1 2 3 4 5 6 7 8 9 10 11
NUMBER OF APPEARANCE OF COMPOUND (TOTAL OF 11)
0.5
1.0
2.0
random

FIG.58

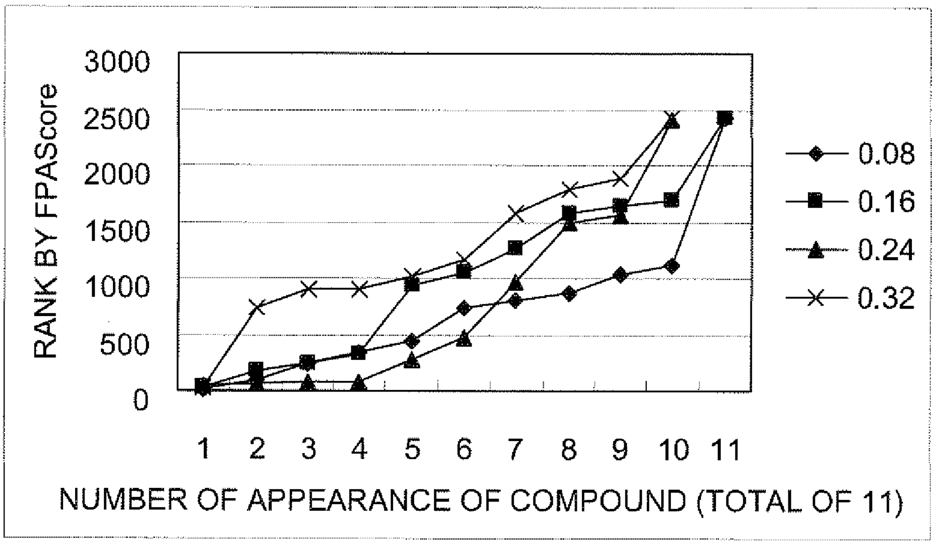


FIG.59

| RANK | FPAScore | PDBID AND LIGAND ID |
|---|---|---|
| 1 | 2834.83 | 2ITQ; STU |
| 2 | 2430.896 | 2J5F; DJK |
| 3 | 2414.404 | 2ITO; IRE |
| 4 | 2178.805 | 2GS7; ANP; |
| 5 | 2133.221 | 1M17; AQ4 |
| 8 | 1986.738 | 1XKK; FMM |
| 81 | 1407.896 | 2ITP; AEE |

K1=4.0, k2=2.0, k3=1.0 Tc UPPER LIMIT VALUE 1.0 Tc LOWER LIMIT VALUE 0.08

| RANK | FPAScore | PDBID AND LIGAND ID |
|---|---|---|
| 6 | 3733.392 | 2ITQ; STU |
| 17 | 2846.782 | 2GS7; ANP |
| 23 | 2671.382 | 1XKK; FMM |
| 29 | 2484.615 | 2J5F; DJK |
| 42 | 2363.591 | 2ITO; IRE |
| 48 | 2308.081 | 2ITP; AEE |
| 105 | 2075.384 | 1M17; AQ4 |

K1=4.0, k2=2.0, k3=1.0 Tc UPPER LIMIT VALUE 1.0 Tc LOWER LIMIT VALUE 0.24

# FIG.60

| LIGAND ID | CORRESPONDING COMPOUND NAME |
|---|---|
| AEE | AEE7 88 |
| ANP | PHOSPHOAMINOPHOSPHONIC ACID-ADENYLATE ESTER |
| AQ4 | ERLOTINIB |
| DJK | N-[4-(3-BROMO-PHENYLAMINO)-QUINAZOLIN-6-YL]-ACRYLAMIDE |
| FMM | Lapatinib |
| IRE | IRESSA |
| STU | STAUROSPORINE |

FIG.61

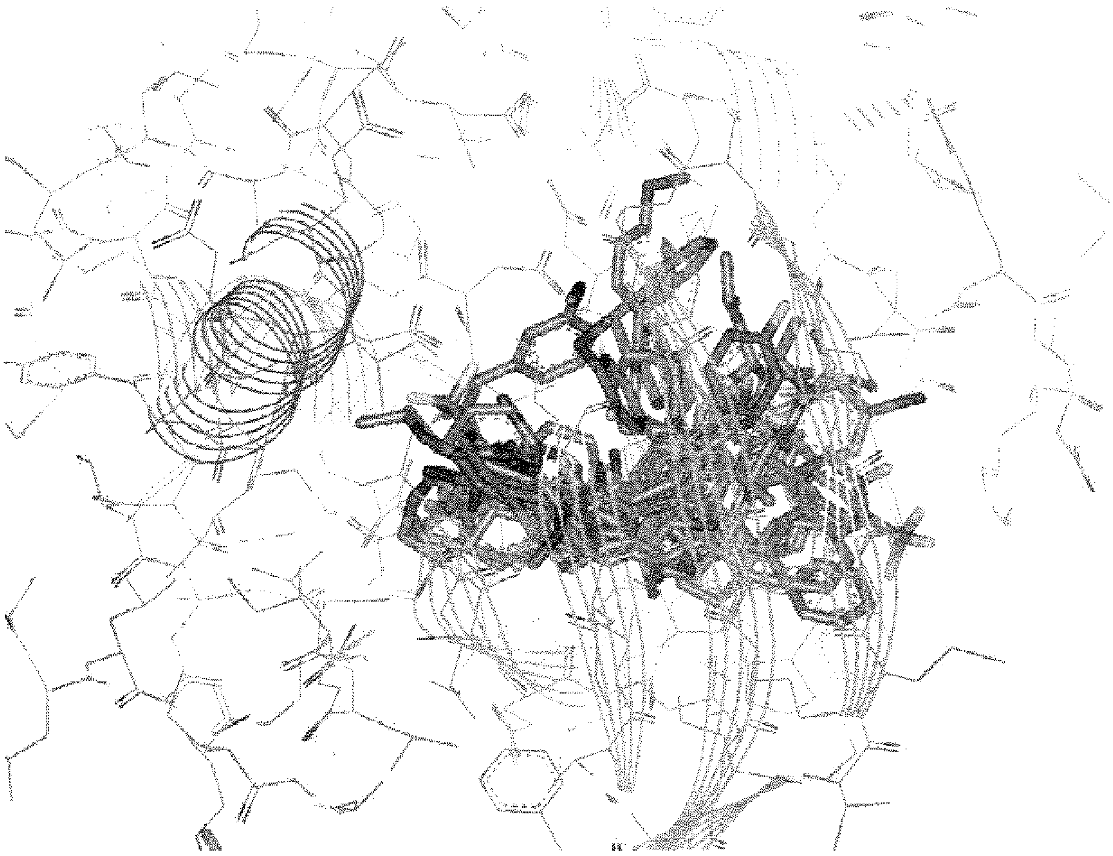

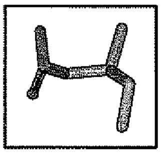 : LIGAND (CENTRAL PART)

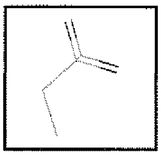 : BINDING SITE OF PROTEIN (PERIPHERAL PART)

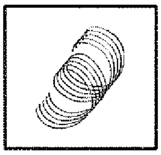 : $\alpha$ -HELIX (RED)

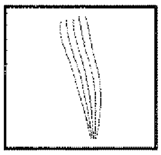 : $\beta$ -SHEET (CYAN)

FIG.62

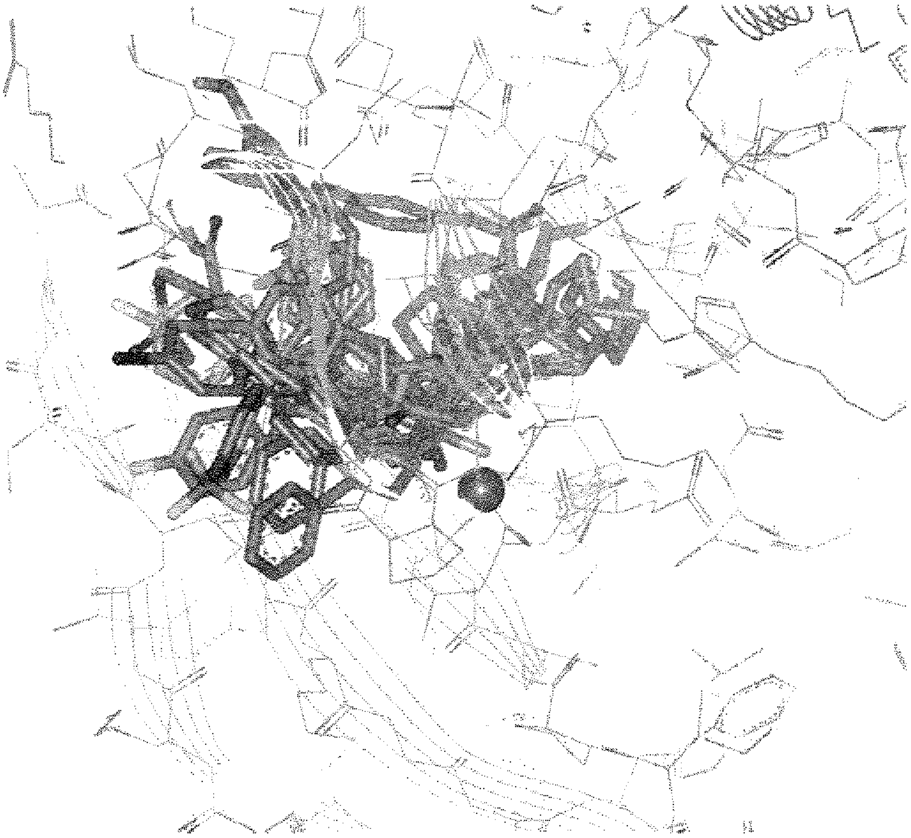

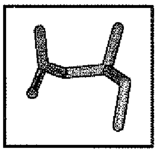 : LIGAND (CENTRAL PART)

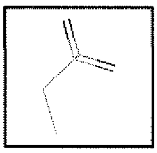 : BINDING SITE OF PROTEIN (PERIPHERAL PART)

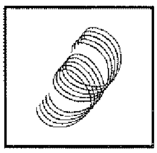 : $\alpha$ -HELIX (RED)

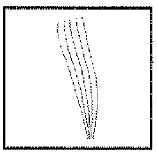 : $\beta$ -SHEET (CYAN)

FIG.63

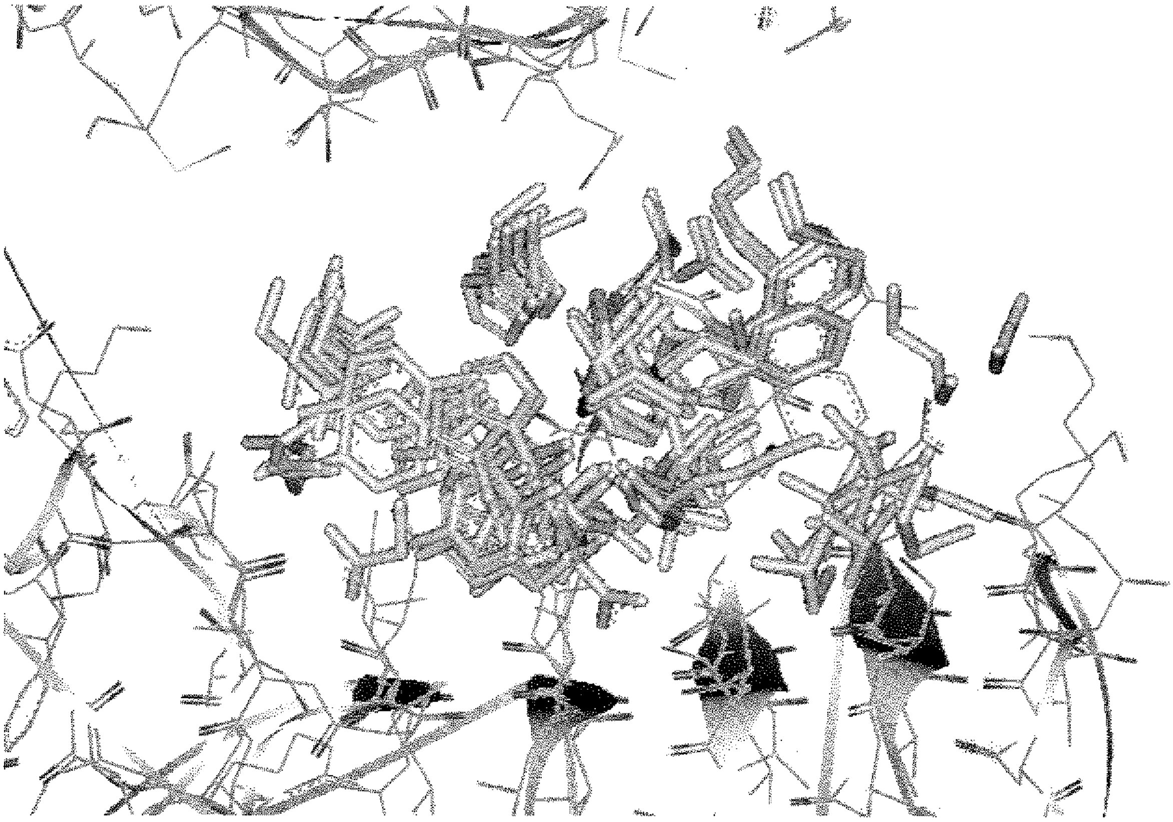

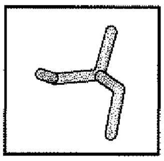
: SIDE CHAIN OF TGF- $\alpha$ ANALOG (CENTRAL YELLOW PART)

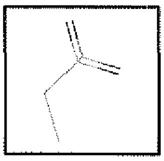
: EGFR BINDING SITE (PERIPHERAL PART)

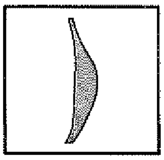
: $\beta$ -SHEET (CYAN)

FIG.64

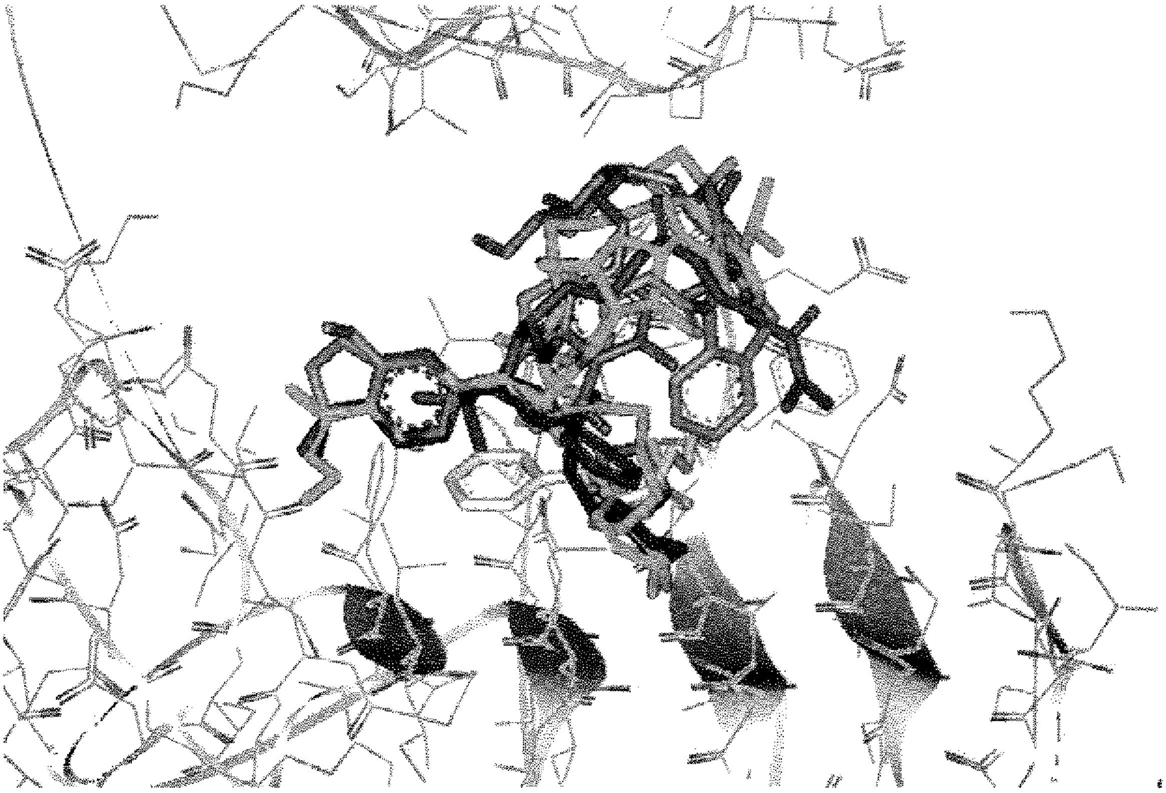

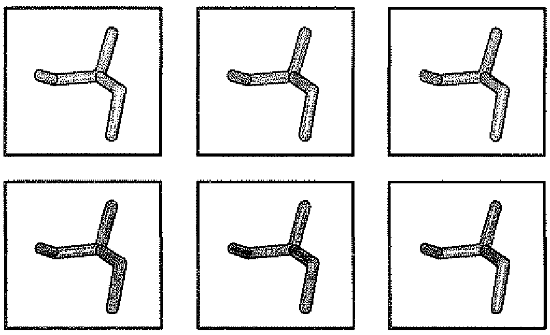

: LIGAND (CENTRAL PART)

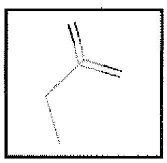

: BINDING SITE OF PROTEIN (PERIPHERAL PART)

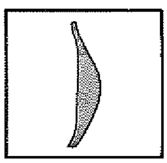

: $\beta$ -SHEET (CYAN)

FIG.65

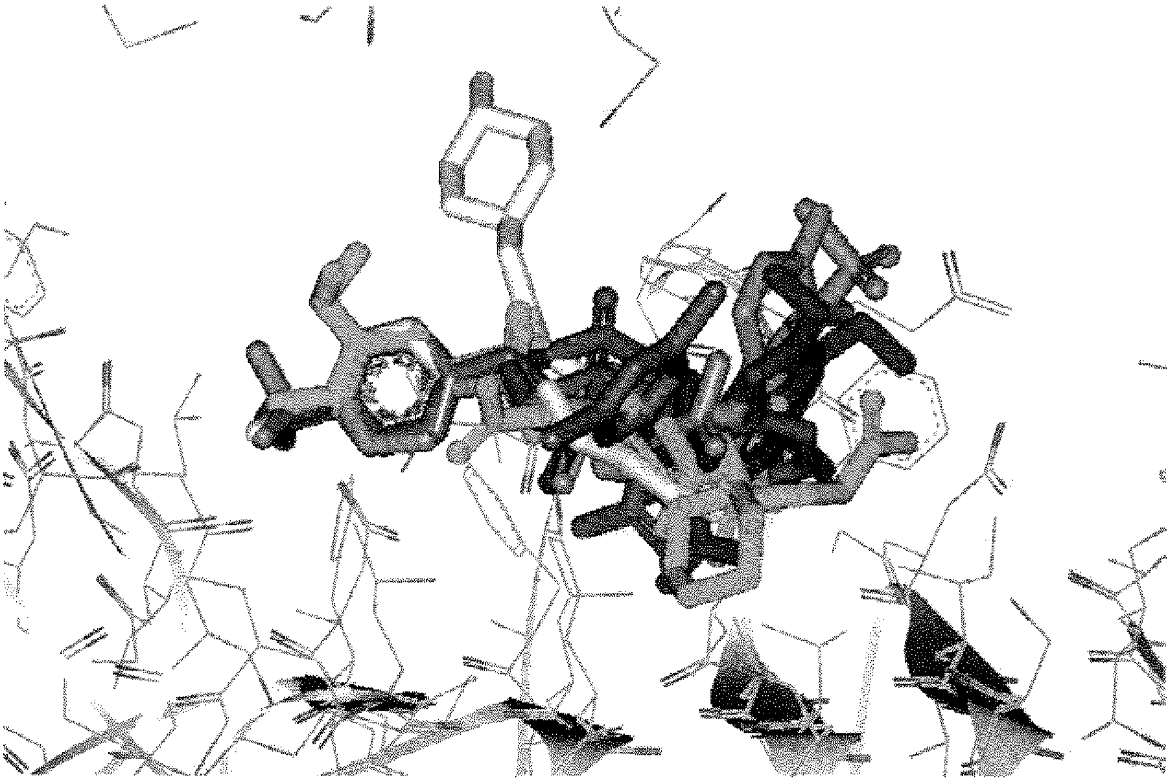

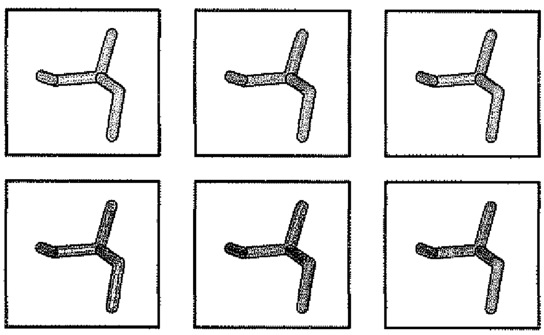

: LIGAND (CENTRAL PART)

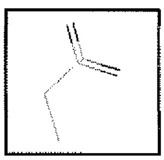

: BINDING SITE OF PROTEIN
(PERIPHERAL PART)

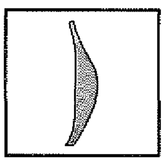

: $\beta$ -SHEET (CYAN)

FIG.66

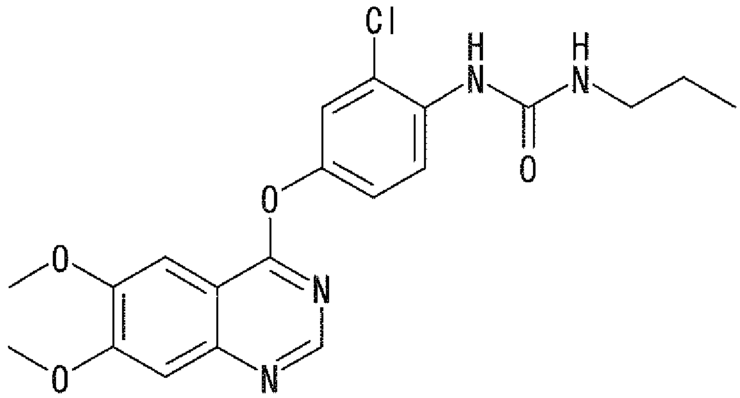

FIG.67

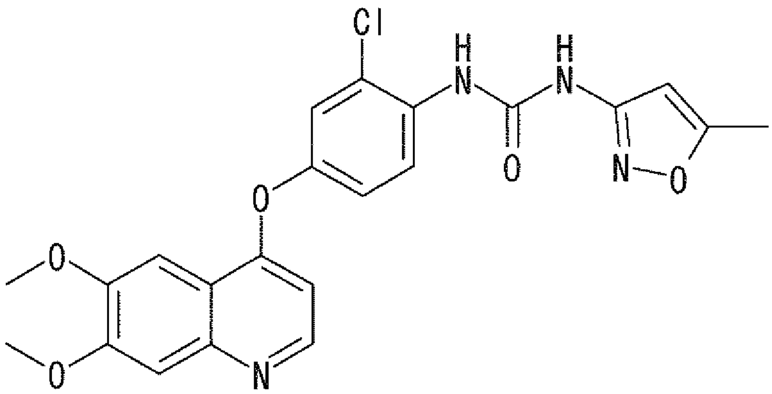

FIG.68

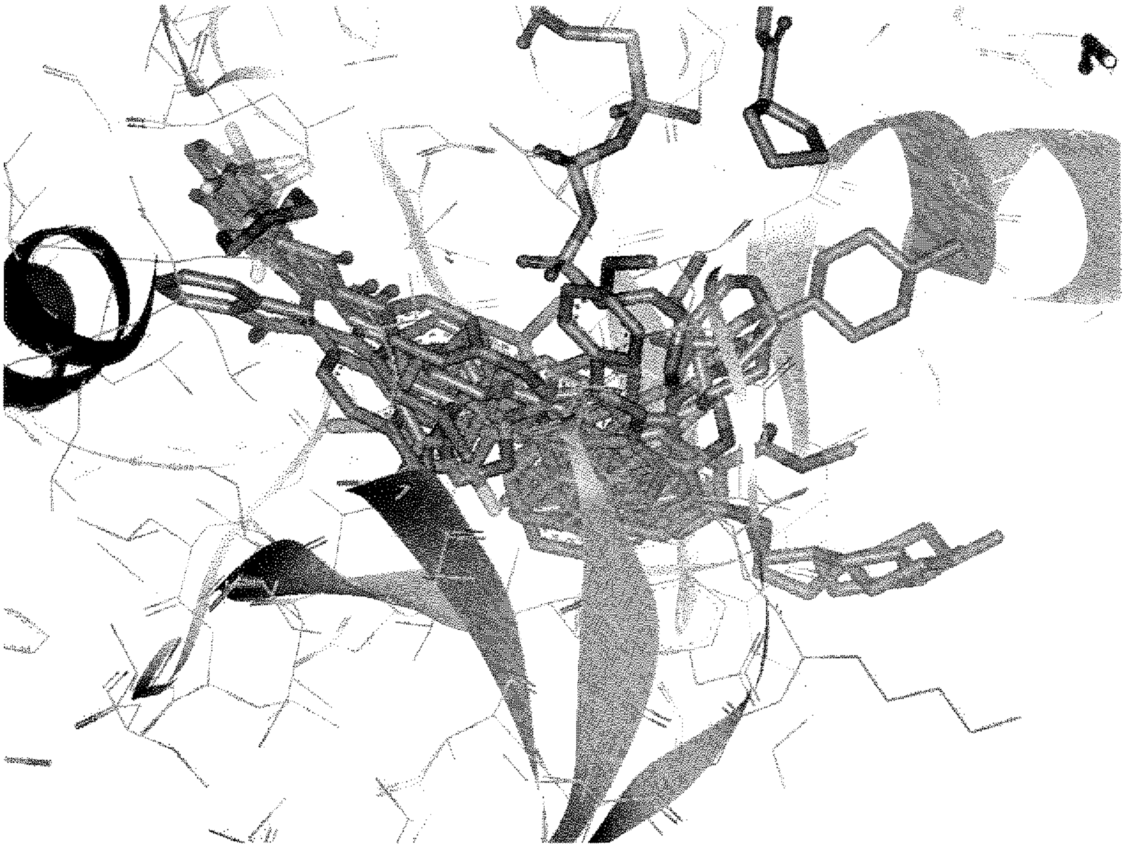

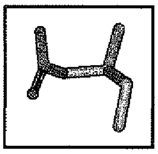 : LIGAND (CENTRAL PART)

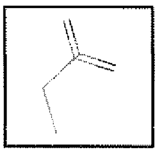 : BINDING SITE OF PROTEIN (PERIPHERAL PART)

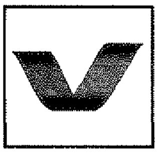 : α-HELIX (RED)

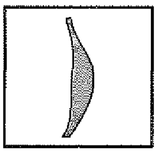 : β-SHEET (CYAN)

FIG.69

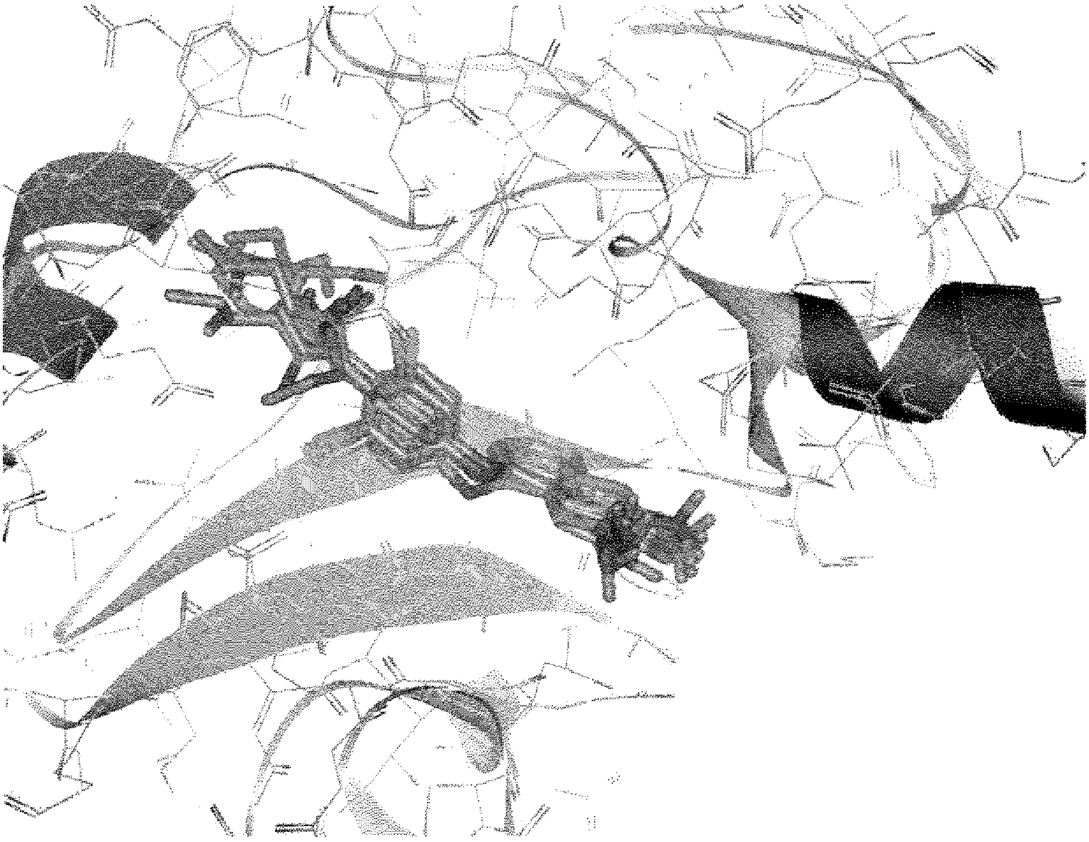

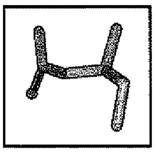 : LIGAND (CENTRAL PART)

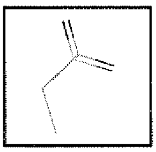 : BINDING SITE OF PROTEIN (PERIPHERAL PART)

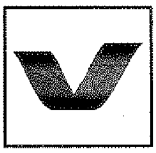 : $\alpha$ -HELIX (RED)

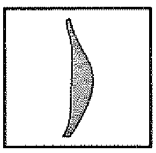 : $\beta$ -SHEET (CYAN)

FIG.70

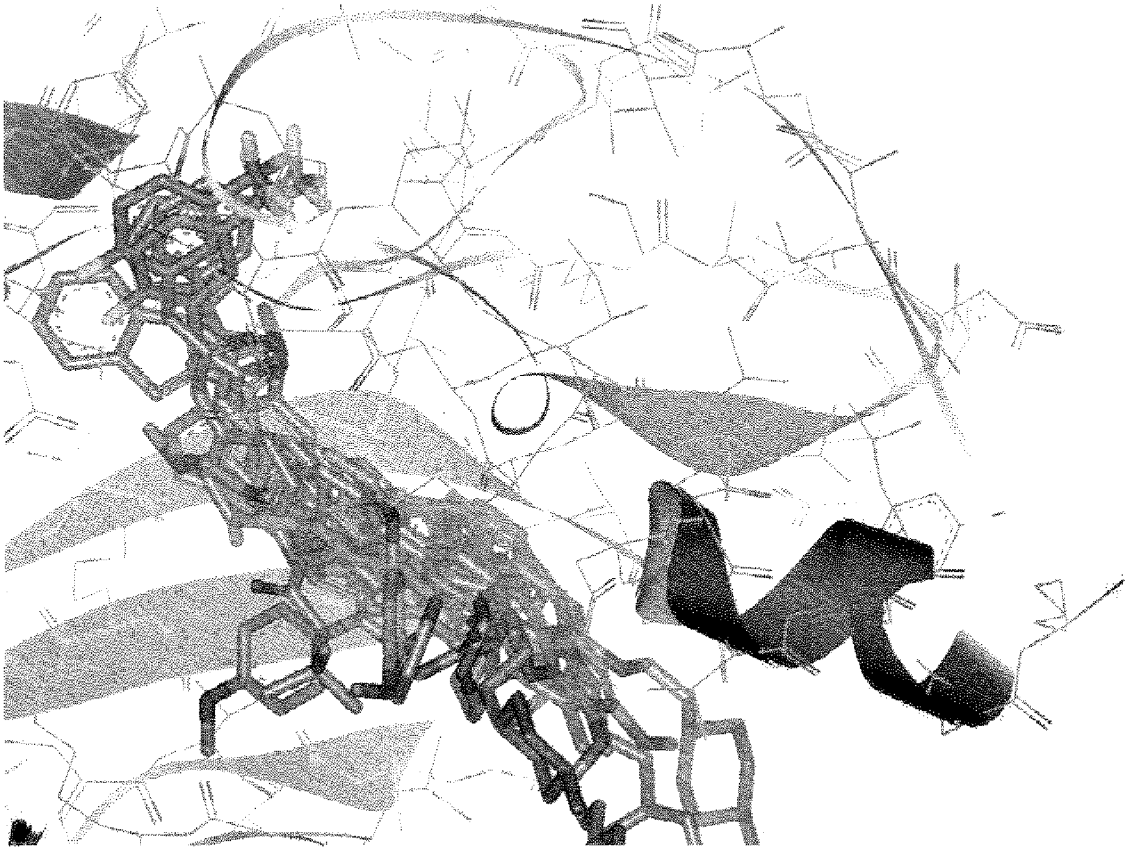

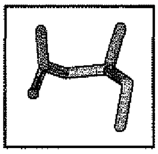 : LIGAND (CENTRAL PART)

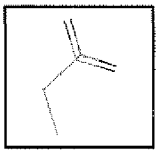 : BINDING SITE OF PROTEIN (PERIPHERAL PART)

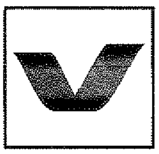 : $\alpha$-HELIX (RED)

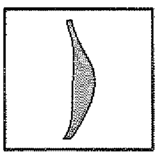 : $\beta$-SHEET (CYAN)

# FIG.71

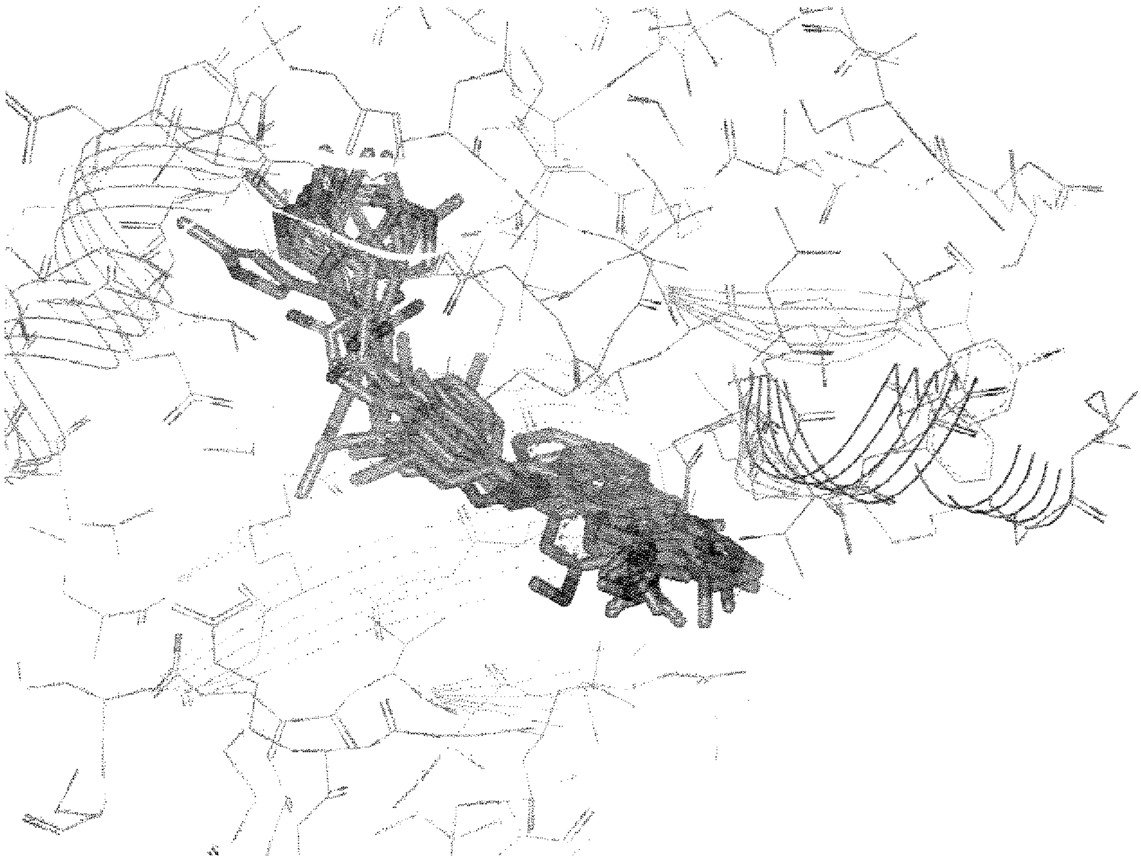

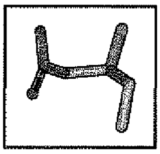

: LIGAND (CENTRAL PART)

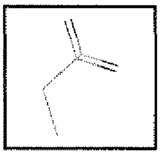

: BINDING SITE OF PROTEIN
(PERIPHERAL PART)

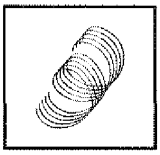

: $\alpha$-HELIX (RED)

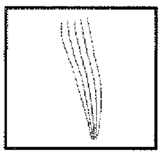

: $\beta$-SHEET (CYAN)

FIG.72

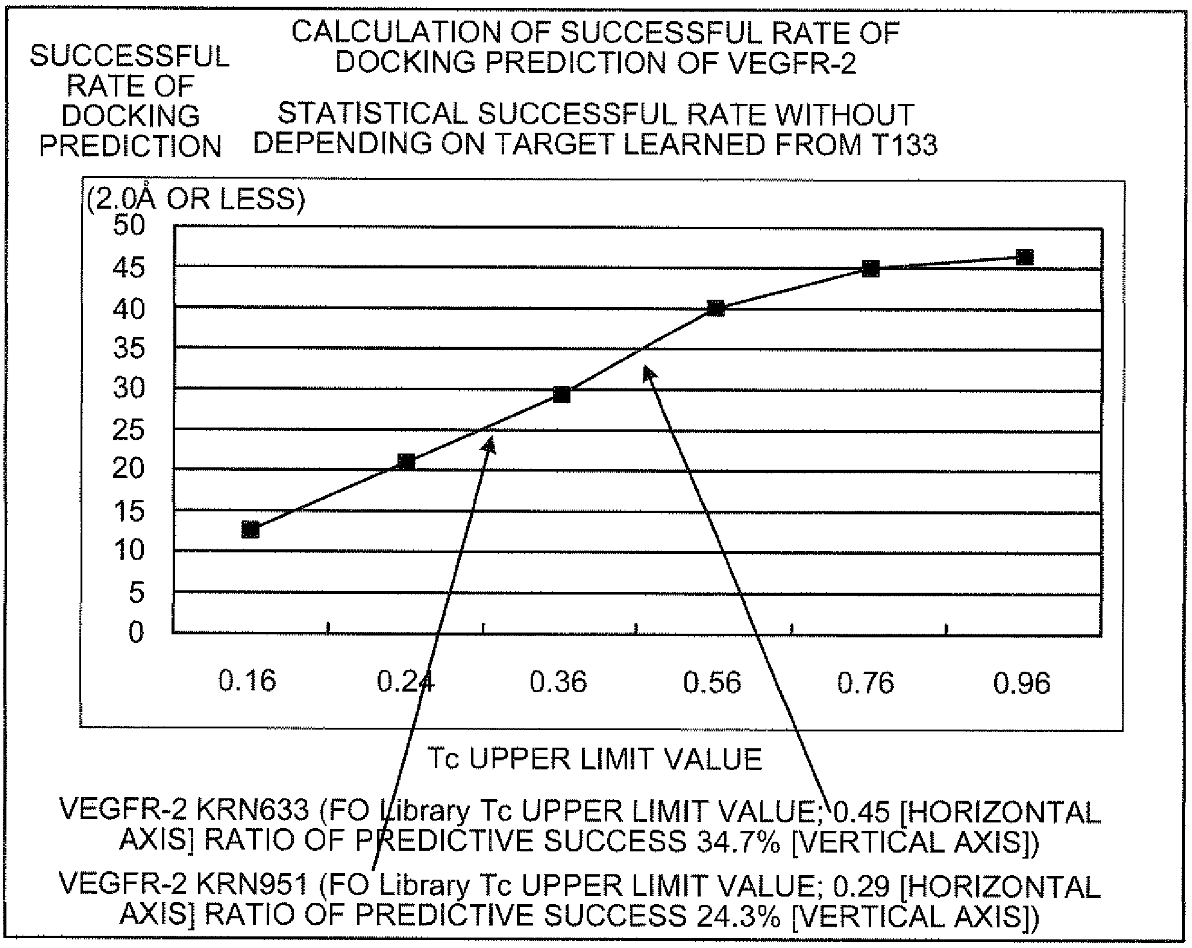
CALCULATION OF SUCCESSFUL RATE OF DOCKING PREDICTION OF VEGFR-2
STATISTICAL SUCCESSFUL RATE WITHOUT DEPENDING ON TARGET LEARNED FROM T133
SUCCESSFUL RATE OF DOCKING PREDICTION
(2.0Å OR LESS)
50
45
40
35
30
25
20
15
10
5
0
0.16
0.24
0.36
0.56
0.76
0.96
Tc UPPER LIMIT VALUE
VEGFR-2 KRN633 (FO Library Tc UPPER LIMIT VALUE; 0.45 [HORIZONTAL AXIS] RATIO OF PREDICTIVE SUCCESS 34.7% [VERTICAL AXIS])
VEGFR-2 KRN951 (FO Library Tc UPPER LIMIT VALUE; 0.29 [HORIZONTAL AXIS] RATIO OF PREDICTIVE SUCCESS 24.3% [VERTICAL AXIS])

FIG.73

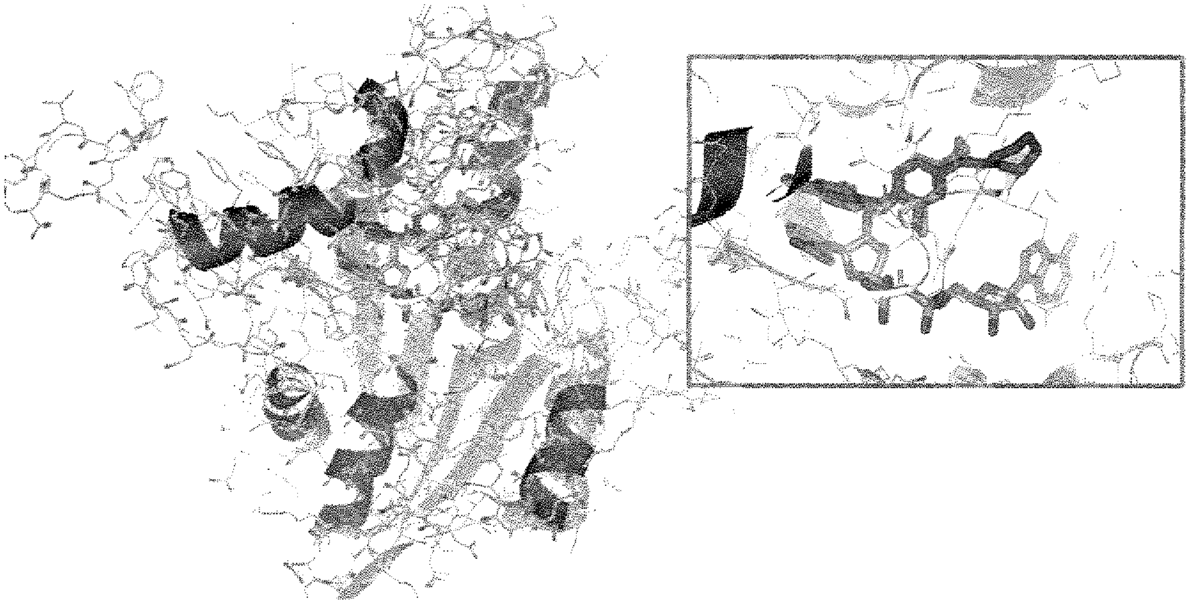

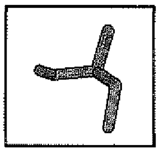
: ANTAGONIST (UPPER SIDE OF CENTER)

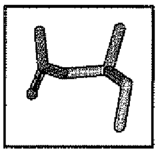
: NAD (LOWER SIDE OF CENTER)

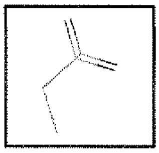
: BINDING SITE OF PROTEIN (PERIPHERAL PART)

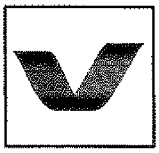
: $\alpha$ -HELIX (RED)

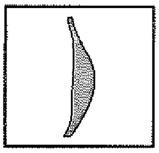
: $\beta$ -SHEET (CYAN)

# FIG.74

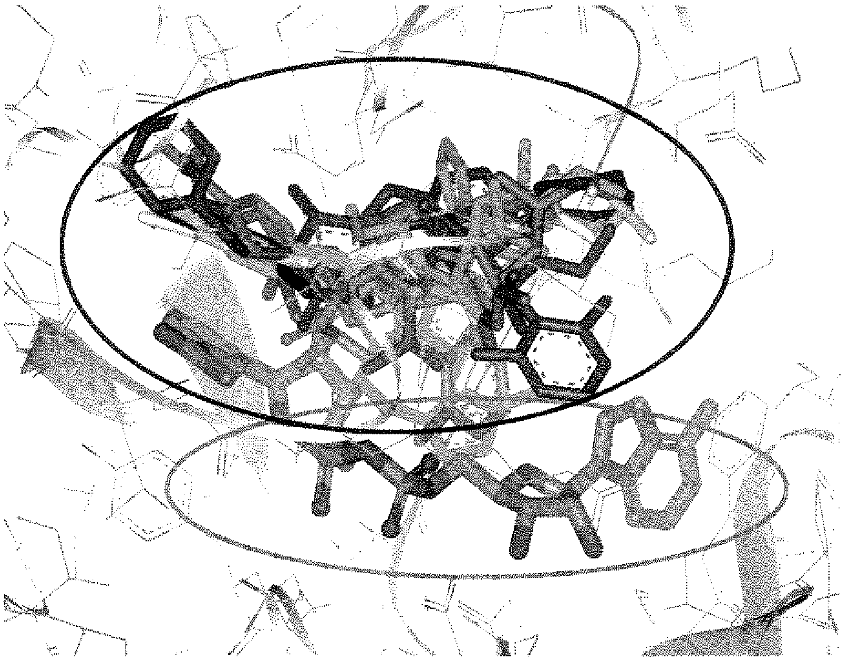

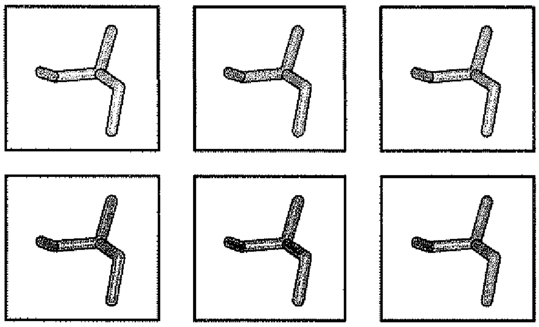

: LIGAND (UPPER SIDE OF CENTER)

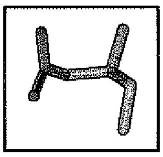

: NAD (LOWER SIDE OF CENTER)

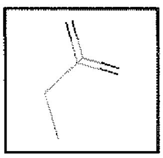

: BINDING SITE OF PROTEIN
(PERIPHERAL PART)

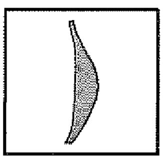

: $\beta$ -SHEET (CYAN)

# FIG.75

| >AMPKhomoGAMMA1 | 304 | homology | match | mismatch | insertion | deletion |
|---|---|---|---|---|---|---|
| >2V9J_E | 304 | 98.7% | 300 | 4 | 0 | 0 |

SNNSVYTSFMKSHRCYDLIPTSSKLVVFDTSLQVKKAFFALVTNGVRAAPLWDSKKQSFVGMLTITDFINILHRYYKSAL
SNSSVYTTFMKSHRCYDLIPTSSKLVVFDTSLQVKKAFFALVTNGVRAAPLWDSKKQSFVGMLTITDFINILHRYYKSAL
** **** ************************************************************************

VQIYELEEHKIETWREVYLQDSFKPLVCISPNASLFDAVSSLIRNKIHRLPVIDPESGNTLYILTHKRILKFLKLFITEF
VQIYELEEHKIETWREVYLQDSFKPLVCISPNASLFDAVSSLIRNKIHRLPVIDPESGNTLYILTHKRILKFLKLFITEF
********************************************************************************

PKPEFMSKSLEELQIGTYANIAMVRTTTPVYVALGIFVQHRVSALPVVDEKGRVVDIYSKFDVINLAAEKTYNNLDVSVT
PKPEFMSKSLEELQIGTYANIAMVRTTTPVYVALGIFVQHRVSALPVVDEKGRVVDIYSKFDVINLAAEKTYNNLDVSVT
********************************************************************************

KALQHRSHYFEGVLKCYLHETLETIINRLVEAEVHRLVVVDENDVVKGIVSLSDILQALVLTGG
KALQHRSHYFEGVLKCYLHETLEAIINRLVEAEVHRLVVVDEHDVVKGIVSLSDILQALVLTGG
*********************** ****************** *********************

# FIG.76

## CMC SCREENING RESULT OF AMPKhomoGAMMA1

| Rank | chk | Docked ligand | original ligand | FPAS core | colli | name | png | formula | Rings | M.W. | logP | Acc | Dnr | tky L.J. |
|---|---|---|---|---|---|---|---|---|---|---|---|---|---|---|
| 1(14) | ☐ | CMC2006v1_00004868(1) | -1 | 901.267 | 1,2,4 | EPIROPRIM | png | $C_{19}H_{23}N_5O_2$ | 3 | 330.24 | 3.98 | 5 | 4 | -29.31 |
| 2(137) | ☐ | CMC2006v1_00005124(1) | -1 | 724.98 | 1,2,2 | ROSIGLITAZONE | png | $C_{18}H_{19}N_3O_3$ | 3 | 338.28 | 2.82 | 4 | 4 | -7.75 |
| 3(199) | ☐ | CMC2006v1_00007786(1) | -1 | 693.542 | 0,1,2 | BUTANIXIN | png | $C_{16}H_{17}N_2O_2$ | 2 | 252.19 | 2.6 | 1 | 3 | -2.7 |
| 4(247) | ☐ | CMC2006v1_00006998(1) | -1 | 675.763 | 0,1,5 | PROPIOMAZINE | png | $C_{20}H_{25}N_2$ | 3 | 316.3 | 3.48 | 3 | 1 | -48.82 |
| 5(260) | ☐ | CMC2006v1_00006302(1) | -1 | 672.265 | 0,1,2 | BUTANSERIN | png | $C_{24}H_{26}FN_3O_3$ | 4 | 397.28 | 3.14 | 3 | 4 | -36.29 |
| 6(389) | ☐ | CMC2006v1_00005803(1) | -1 | 632.201 | 0,0,1 | ANISOPIROL | png | $C_{21}H_{28}FN_2O_2$ | 3 | 331.24 | 3.69 | 3 | 3 | -31.39 |
| 7(414) | ☐ | CMC2006v1_00005171(1) | -1 | 624.97 | 0,2,5 | AZAPERONE | png | $C_{19}H_{23}FN_3$ | 3 | 305.23 | 3.22 | 2 | 3 | -36.02 |
| 8(451) | ☐ | CMC2006v1_00002706(1) | -1 | 618.414 | 0,2,4 | PIPRATECOL | png | $C_{19}H_{25}N_2O_4$ | 3 | 320.22 | 2.18 | 3 | 4 | -36.03 |
| 9(469) | ☐ | CMC2006v1_00005967(1) | -1 | 615.724 | 0,2,2 | BUTROPIPAZONE | png | $C_{20}H_{24}FN_2$ | 3 | 303.23 | 3.83 | 2 | 2 | -21.13 |
| 10(671) | ☐ | CMC2006v1_00005328(1) | -1 | 582.259 | 0,2,5 | CARPHENAZINE | png | $C_{24}H_{33}N_3O_2$ | 4 | 394.35 | 4.25 | 4 | 2 | -15.16 |
| 11(756) | ☐ | CMC2006v1_00006323(1) | -1 | 567.542 | 0,1,2 | MEFECLORAZINE | png | $C_{20}H_{26}ClN_2O_2$ | 3 | 335.69 | 3.94 | 4 | 3 | -35.22 |
| 12(836) | ☐ | CMC2006v1_00006505(1) | -1 | 557.475 | 1,1,1 | MCMC00010966 | png | $C_{24}H_{23}N_3O_2$ | 5 | 362.28 | 4.11 | 4 | 2 | -38.47 |
| 13(894) | ☐ | CMC2006v1_00006361(1) | -1 | 551.318 | 1,2,3 | MCMC00011316 | png | $C_{28}H_{30}N_5O_3$ | 5 | 454.34 | 3.8 | 5 | 5 | -64.06 |
| 14(904) | ☐ | CMC2006v1_00004094(1) | -1 | 550.754 | 1,3,5 | LESOPITRON | png | $C_{15}H_{22}ClN_6$ | 3 | 299.66 | 2.15 | 4 | 3 | -34.77 |
| 15(974) | ☐ | CMC2006v1_00004397(1) | -1 | 543.014 | 1,2,3 | TRAZODONE | png | $C_{19}H_{23}ClN_5$ | 4 | 349.7 | 2.58 | 5 | 2 | -19.05 |
| 16(1258) | ☐ | CMC2006v1_00008285(1) | -1 | 512.867 | 0,2,5 | LOFEPRAMINE | png | $C_{26}H_{28}ClN_2$ | 4 | 391.75 | 4.43 | 2 | 2 | -30.95 |
| 17(1426) | ☐ | CMC2006v1_00004887(1) | -1 | 499.07 | 0,0,0 | KETANSERIN | png | $C_{22}H_{22}FN_3O_3$ | 4 | 373.26 | 2.36 | 3 | 4 | -11.87 |
| 18(1519) | ☐ | CMC2006v1_00004722(1) | -1 | 491.994 | 0,0,1 | SPIPERONE | png | $C_{23}H_{27}FN_3O_2$ | 4 | 369.27 | 3.76 | 3 | 3 | -16.87 |
| 19(2047) | ☐ | CMC2006v1_00006678(1) | -1 | 453.939 | 1,2,3 | AMIPERONE | png | $C_{24}H_{29}ClFN_2O_2$ | 3 | 402.73 | 4.72 | 2 | 4 | -16.26 |
| 20(2209) | ☐ | CMC2006v1_00002983(1) | -1 | 444.529 | 1,2,4 | ECABAPIDE | png | $C_{20}H_{25}N_3O_4$ | 2 | 346.24 | 2.69 | 5 | 4 | -21.95 |
| 21(2710) | ☐ | CMC2006v1_00006571(1) | -1 | 417.289 | 1,3,5 | PROSTAGLANDIN | png | $C_{20}H_{35}O_5$ | 1 | 322.23 | 1.93 | 3 | 5 | -29.73 |
| 22(3012) | ☐ | CMC2006v1_00004756(1) | -1 | 402.001 | 1,1,2 | 2-AMINO-4H-3,1-BENZOXAZINONES | png | $C_{14}H_8ClIN_2O_2$ | 3 | 390.52 | 4.26 | 3 | 3 | -29.57 |
| 23(3069) | ☐ | CMC2006v1_00005798(1) | -1 | 398.616 | 0,3,5 | PROGESTERONE | png | $C_{21}H_{30}O_2$ | 4 | 287.25 | 4.72 | 0 | 2 | -5.42 |
| 24(3577) | ☐ | CMC2006v1_00006639(1) | -1 | 368.772 | 0,1,4 | PROTIZINIC ACID | png | $C_{17}H_{16}NO_3$ | 3 | 298.26 | 2.85 | 3 | 3 | -10.23 |
| 25(3787) | ☐ | CMC2006v1_00005686(1) | -1 | 356.823 | 0,1,2 | TRIMEGESTONE | png | $C_{22}H_{30}O_3$ | 4 | 315.26 | 4.15 | 0 | 3 | -10.59 |
| 26(3915) | ☐ | CMC2006v1_00004045(1) | -1 | 350.149 | 0,2,5 | DEPRODONE PROPION | png | $C_{24}H_{32}O_5$ | 4 | 371.28 | 3.55 | 1 | 5 | 268.09 |
| 27(4885) | ☐ | CMC2006v1_00003905(1) | -1 | 277.556 | 1,1,2 | QUINAZOSIN | png | $C_{17}H_{24}N_5O_2$ | 3 | 306.22 | 2.34 | 5 | 4 | -40.89 |

# FIG.77

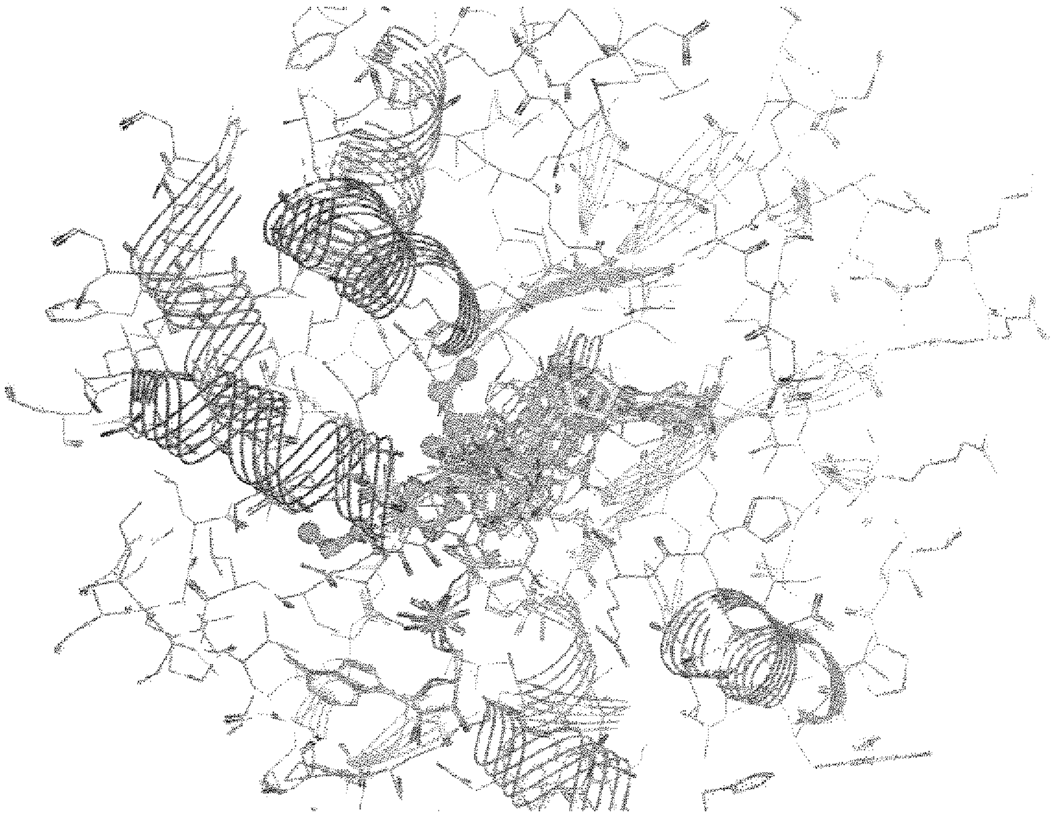

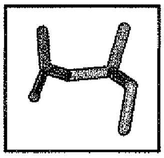 : LIGAND (CENTRAL PART)

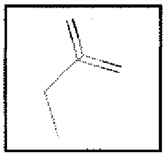 : BINDING SITE OF PROTEIN (PERIPHERAL PART)

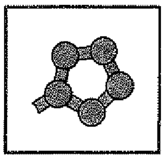 : HIS RESIDUE (GREEN)

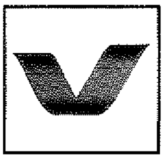 : $\alpha$-HELIX (RED)

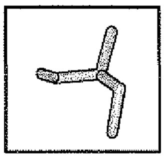 : ADENOSINE etc. (YELLOW)

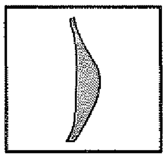 : $\beta$-SHEET (CYAN)

**INTERNATIONAL SEARCH REPORT**

International application No.
PCT/JP2008/070973

A. CLASSIFICATION OF SUBJECT MATTER
*C40B30/02*(2006.01)i, *G06F19/00*(2006.01)i

According to International Patent Classification (IPC) or to both national classification and IPC

B. FIELDS SEARCHED

Minimum documentation searched (classification system followed by classification symbols)
C40B30/02, G06F19/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
PubMed, JSTPlus(JDreamII), JMEDPlus(JDreamII)

C. DOCUMENTS CONSIDERED TO BE RELEVANT

| Category* | Citation of document, with indication, where appropriate, of the relevant passages | Relevant to claim No. |
|---|---|---|
| A | WO 2002/041184 A1 (Kyorin Pharmaceutical Co., Ltd.), 23 May, 2002 (23.05.02), Full text; all drawings & US 2004/0038429 A1 & AU 2403402 A | 1-12 |
| A | JP 2002-530727 A (Glaxo Group Ltd.), 17 September, 2002 (17.09.02), Full text; all drawings & US 2002/0052694 A1 & EP 1153358 A & WO 2000/025106 A2 | 1-12 |
| P,X | TAKAYA, D., Bioinformatics based Ligand-Docking and in-silico screening, Chemical & pharmaceutical bulletin, 2008.05, Vol.56, No.5, p.742-4 | 1-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>15 January, 2009 (15.01.09) | Date of mailing of the international search report<br>27 January, 2009 (27.01.09) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2008/070973

C (Continuation). DOCUMENTS CONSIDERED TO BE RELEVANT

| Category* | Citation of document, with indication, where appropriate, of the relevant passages | Relevant to claim No. |
|---|---|---|
| A | MPAMHANGA, C.P., Knowledge-based interaction fingerprint scoring: a simple method for improving the effectiveness of fast scoring functions, Journal of chemical information and modeling, 2006, Vol.46, No.2, p.686-98 | 1-12 |
| A | SCIABOLA, S., Pharmacophoric fingerprint method (TOPP) for 3D-QSAR modeling: application to CYP2D6 metabolic stability, Journal of chemical information and modeling, 2007.02, Vol.47, No.1, p.76-84 | 1-12 |
| A | KELLY, M.D., Expanded interaction fingerprint method for analyzing ligand binding modes in docking and structure-based drug design, Journal of chemical information and computer sciences, 2004, Vol.44, No.6, p.1942-51 | 1-12 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Ewing et al.** *J. Comput. Aided Mol. Des.,* 2001, vol. 15 (5), 411-428
- **Goodsell et al.** *J. Mol. Recognit.,* 1996, vol. 9, 1-5
- **Jones et al.** *J. Mol. Biol.,* 1997, vol. 267, 724-748
- Complete Course of C Algorithm: From Basics to Graphics. Kindai Kagaku Sha Co., Ltd,
- **Terashi G ; Takeda-Shitaka M ; Kanou K ; Iwadate M ; Takaya D ; Hosoi A ; Ohta K ; Umeyama H.** *Proteins,* 2007, vol. 69 (8), 98-107
- **Westbrook et al.** *Nucleic Acids Res.,* 01 January 2003, vol. 31 (1), 489-91
- **Takeda-Shitaka, M. ; Terashi, G. ; Takaya, D ; Kanou, K. ; Iwadate, M. ; Umeyama, H.** Protein structure prediction in CASP6 using CHIMERA and FAMS. *Proteins,* 2005, vol. 61, 122-127
- **Takede-Shitaka, M. ; Takaya, D. ; Chiba, C. ; Tanaka, H. ; Umeyama, H.** *Curr. Med. Chem.,* 2004, vol. 11, 551-558
- **Edgar R. Wood et al.** *CANCER RESEARCH,* 2004, vol. 64, 6652-6659
- **Jennifer et al.** *J. Bio. Chem.,* 2002, vol. 277 (48), 46265-46272
- **Lopez, G ; Rojas, A ; Tress, M ; Valencia, A.** *Proteins,* 2007, vol. 69 (S8), 165-174
- **Goodsell et al.** *J. Mol. Recognit,* 1996, vol. 9, 1-5
- **Ewing et al.** *J Comput Aided Mol Des.,* 2001, vol. 15 (5), 411-428
- **Gareth et al.** *J. Mol. Biol.,* 1997, vol. 267, 727-748
- **Onodera et al.** *J. Chem. Inf. Model.,* 2007, vol. 47, 1609-1618
- **Michael et al.** *J. Med. Chem.,* 2007, vol. 50, 726-741
- **Budin et al.** *Biol Chem.,* 2001, vol. 382 (9), 1365-1372
- **Sukumaran et al.** *Eur. J. Med. Chem.,* 2007, vol. 42, 966-976
- **Zhan et al.** *J. Med. Chem.,* 2004, vol. 47, 337-344
- **Brooks, R. B ; Bruccoleri, E. R. ; Olafson, D. B. ; States, J. D. ; Swamlnathan, S. ; Karplus, M. CHARMM.** A program for macromolecular energy, minimization, and dynamics calculations. *J. Comp. Chem.,* 1983, vol. 4, 187-217
- **Case, A. D. ; Cheatham III, E. T. ; Darden, T. ; Gohike, H. ; Luo, R. ; Merz Jr., M. K. ; Onufriev, A. ; Simmerling, C. ; Wang, B. ; Woods, J. R.** The Amber Biomolecular Simulation Programs. *J. Comput. Chem.,* 2005, vol. 26, 1668-1688
- **Fedorov, G. D. ; Kitaura, K.** Extending the Power of Quantum Chemistry to Large Systems with the Fragment Molecular Orbital Method. *J. Phys. Chem.,* 2007, vol. 111, 6904-6914
- **Shindyalov et al.** *Protein Engineering,* 1998, vol. 11 (9), 739-747
- **Altschul et al.** *Nucleic Acids Res.,* 1997, vol. 27 (17), 3389-3402
- **Swamidass, S. J. ; Baldi, P.** Mathematical Correction for Fingerprint Similarity Measures to Improve Chemical Retrieval. *J. Chem. Inf. Model.,* 2007, vol. 47, 952-964
- *Cancer Res,* 15 May 2005, vol. 65 (10
- **Chiba et al.** *C algorithm ZENKA,* 1995, ISBN 4-7649-0239-7
- *J. Comput. Chem.,* 2005, vol. 26, 1668-1688
- *J. Chem. Inf. Comput. Sci.,* 2000, vol. 40, 163-166
- *Nucleic Acids Res.,* 1997, vol. 27, 3398-3402
- *Protein Engineering,* 1998, vol. 11, 739-747
- *J. Mol. Biol.,* 1993, vol. 233, 123-138
- *Protein Science,* 2004, vol. 13, 1865-1874
- **Terashi G ; Takeda-Shitaka M ; Kanou K ; Iwadate M ; Takaya D ; Hosoi A ; Ohta K ; Umeyama H.** *Proteins,* 2007, vol. 69 (S8), 98-107
- *J. Mol. Graphics Mod.,* 2000, vol. 18, 258-272305-306
- *Nucleic Acids Res.,* 2003, vol. 31, 489-491
- *J. Med. Chem.,* 2007, vol. 50, 726-741
- **Jones et al.** *J. Mol. Biol.,* 1997, vol. 267, 727-748
- **Onodera et al.** *J. Chem. In. Model.,* 2007, vol. 47, 1609-1618
- **Hartshom et al.** *J. Med. Chem.,* 2007, vol. 50, 726-741
- *J. Med. Chem.,* 2004, vol. 47, 1739-1749
- *J. Mol. Biol.,* 1996, vol. 261, 470-489
- *Eur. J. Med. Chem.,* 2007, vol. 42, 966-976
- *J. Med. Chem.,* 2004, vol. 47, 337-344
- **Case, A. D. ; Cheatham III, E. T. ; Darden, T. ; Gohlke, H. ; Luo, R. ; Merz Jr., M. K. ; Onufriev, A. ; Simmering, C. ; Wang, B. ; Woods, J. R.** The Amber Biomolecular Simulation Programs. *J. Comput. Chem.,* 2005, vol. 26, 1668-1688

- **Brooks, R. B ; Bruccoleri, E. R. ; Olafson, D. B. ; States, J. D. ; Swaminathan, S. ; Karplus, M.** CHARMM A program for macromolecular energy, minimization, and dynamics calculations. *J. Comp. Chem.,* 1983, vol. 4, 187-217
- **Kamiya K ; Sugawara Y ; Umeyama H.** *J. Comput. Chem.,* 2003, vol. 24, 826-841
- *J. Biol. Chem.,* 2002, vol. 277, 46265-46272
- *Cell,* 2006, vol. 125, 1137-1149
- **Wheeler, D. L. et al.** *Nucleic Acids Res.,* 27 November 2007
- *Proteins,* 2005, vol. 7, 122-127
- **Terashi, G. et al.** *Proteins,* 2007
- *Nat. Rev. Cancer.,* 2004, vol. 4, 361-370
- *Proteins,* 2005, vol. 61, 122-127
- *Mol. Garxoer. Ther.,* 2004, vol. 3, 1639-1649
- *Cancer Res.,* 2006, vol. 66, 9134-9142
- *J. Biol. Chem.,* 2002, vol. 277, 13106-13114
- Comprehensive Medicinal Chemistry. Elsevier, 2006, vol. 1
- Available Chemicals Directory. Elsevier, 2008, vol. 1